# EUROPÄISCHE PATENTSCHRIFT

(11) **EP 1 501 795 B1**
(45) Veröffentlichungstag und Bekanntmachung des Hinweises auf die Patenterteilung: **28.02.2007**
(21) Anmeldenummer: 03720461.7
(22) Anmeldetag: 14.04.2003
(51) Int. Cl.: C07D 207/40, C07D 401/12, C07D 405/12, C07D 409/12, A61K 31/4015, A61P 31/04

(54) **ZIMTSÄUREAMIDE**
CINNAMIC ACID AMIDES
AMIDES D'ACIDE CINNAMIQUE

(30) Priorität: 26.04.2002 DE 10218582
(43) Veröffentlichungstag der Anmeldung: 02.02.2005
(73) Patentinhaber: Bayer HealthCare AG, 51368 Leverkusen (DE)
(72) Erfinder: BRUNNER, Nina, 41547 Essen (DE); FREIBERG, Christoph, 42115 Wuppertal (DE); LAMPE, Thomas, 40223 Düsseldorf (DE); NEWTON, Ben, Amersham, Buckinghamshire HP6 5LP (GB); OTTENEDER, Michael, CH-4144 Arlesheim (CH); PERNERSTORFER, Josef, 40721 Hilden (DE); POHLMANN, Jens, 4058 Basel (CH); SCHIFFER, Guido, 42111 Wuppertal (DE); SHIMADA, Mitsuyuki, Higashigawa-cho, Nara 630-8323 (JP); SVENSTRUP, Niels, 42103 Wuppertal (DE); ENDERMANN, Rainer, 42113 Wuppertal (DE); NELL, Peter, 42115 Wuppertal (DE)
(86) Internationale Anmeldenummer: PCT/EP2003/003834
(87) Internationale Veröffentlichungsnummer: WO 2003/091212

(56) Entgegenhaltungen:
- EP-A- 0 250 115
- PATENT ABSTRACTS OF JAPAN vol. 014, no. 090 (C-0691), 20. Februar 1990 (1990-02-20) & JP 01 301657 A (SUNTORY LTD), 5. Dezember 1989 (1989-12-05) in der Anmeldung erwähnt

## Beschreibung

Die vorliegende Erfindung betrifft Verbindungen, die geeignet sind zur Behandlung und/oder Prophylaxe von Erkrankungen bei Menschen oder Tieren, insbesondere bakteriellen Infektionskrankheiten, Verfahren zur ihrer Herstellung sowie sie umfassende pharmazeutische Zusammensetzungen.

Die Naturstoffe Moiramid B (R^{a} = Wasserstoff, R^{b} = Methyl) und Andrimid (R^{a} = Wasserstoff, R^{b} = Propenyl) sind als antibakteriell wirksam beschrieben, während Moiramid C (R^{a} = Hydroxy, R^{b} = Propenyl) unwirksam ist. (A. Fredenhagen, S. Y. Tamura, P. T. M. Kenny, H. Komura, Y. Naya, K. Nakanishi, *J. Am. Chem. Soc.,* 1987, 109, 4409-4411; J. Needham, M. T. Kelly, M. Ishige, R. J. Andersen, *J. Org. Chem.,* 1994, 59, 2058-2063; M. P. Singh, M. J. Mroczenski-Wildey, D. A. Steinberg, R. J. Andersen, W. M. Maiese, M. Greenstein, *J. Antibiot.,* 1997, 50(3), 270-273). Die Isolierung und antibakterielle Wirksamkeit von Andrimid ist auch in EP-A-250 115 beschrieben. JP 01301657 beschreibt die Verwendung von Andrimid und einiger amidischer Derivate als agrochemische Antibiotika.

Die Synthese von Andrimid wird in A. V. Rama Rao, A. K. Singh, Ch. V. N. S. Varaprasad, *Tetrahedron Letters,* **1991**, 32, 4393-4396 beschrieben, diejenige von Moiramid B und Andrimid in S. G. Davies, D. J. Dixon, *J Chem. Soc., Perkin Trans.* 1, 1998, 2635-2643.

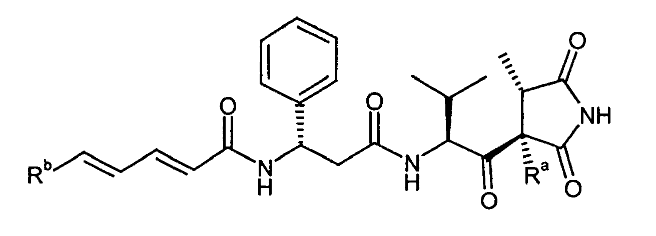

Die Naturstoffe entsprechen hinsichtlich ihrer Eigenschaften, wie z.B. ihrer Wirksamkeit, nicht den Anforderungen, die an antibakteriell wirkende Arzneimittel gestellt werden.

Auf dem Markt sind zwar strukturell andersartige antibakteriell wirkende Mittel vorhanden, es kann aber regelmäßig zu einer Resistenzentwicklung kommen. Neue Mittel für eine bessere und wirksame Therapie sind daher wünschenswert.

Eine Aufgabe der vorliegenden Erfindung ist es daher, neue und alternative Verbindungen mit gleicher oder verbesserter antibakterieller Wirkung zur Behandlung von bakteriellen Erkrankungen bei Menschen und Tieren zur Verfügung zu stellen.

Überraschenderweise wurde gefunden, dass Derivate dieser Verbindungsklasse, worin die beta-Phenylalanin-Amidgruppe durch ein vinyloges aromatisches oder heteroaromatisches Amid ersetzt wird, antibakteriell wirksam sind.

Gegenstand der vorliegenden Erfindung sind daher Verbindungen der Formel 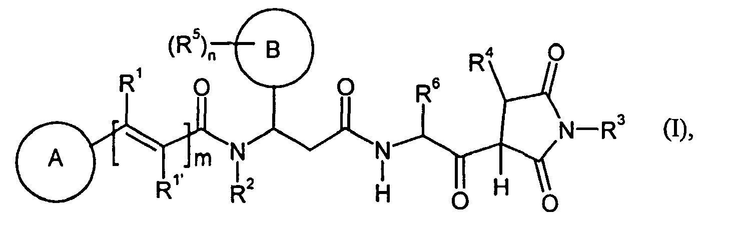 worin
- R¹: Wasserstoff, Methyl oder Halogen bedeutet,
- R^{1'}: Wasserstoff, Methyl oder Halogen bedeutet,
- R²: Wasserstoff oder Methyl bedeutet,
- R³: Wasserstoff, Hydroxy, Amino, C₁-C₃-Alkyl, C₁-C₃-Alkoxy, Benzyloxy, C₁-C₃-Alkylamino, C₁-C₃-Alkylcarbonylamino, Phenylcarbonylamino oder Benzylcarbonylamino bedeutet,
- R⁴: Wasserstoff oder C₁-C₃-Alkyl bedeutet,
- R⁵: Halogen, Trifluormethyl, Trifluormethoxy, Nitro, Amino, Alkylamino, Hydroxy, Alkyl, Alkoxy, Carboxyl, Alkoxycarbonyl, Benzyloxycarbonyl, Aminocarbonyl, Alkylaminocarbonyl, Aryl oder Heteroaryl bedeutet,
oder
zwei Substituenten R⁵ zusammen mit den Kohlenstoffatomen, an die sie gebunden sind, ein Cycloalkyl oder Heterocyclyl bilden, wobei dieses Cycloalkyl oder Heterocyclyl substituiert sein kann mit 0, 1 oder 2 Substituenten R⁵⁻¹, wobei die Substituenten R⁵⁻¹ unabhängig voneinander ausgewählt werden aus der Gruppe bestehend aus Halogen, Nitro, Amino, Trifluormethyl, Hydroxy und Alkoxy,
- R⁶: Alkyl, Cycloalkyl oder Cycloalkenyl bedeutet,
wobei R⁶ substituiert sein kann mit 0, 1, 2 oder 3 Substituenten R⁶⁻¹, wobei die Substituenten R⁶⁻¹ unabhängig voneinander ausgewählt werden aus der Gruppe bestehend aus Halogen, Nitro, Amino, Trifluormethyl, Hydroxy, Alkyl und Alkoxy,
- n: eine Zahl 0, 1, 2 oder 3 bedeutet,
wobei bei n gleich 2 oder 3 die Reste R⁵ gleich oder verschieden sein können,
- m: eine Zahl 1, 2 oder 3 bedeutet,
- A: Aryl oder Heteroaryl bedeutet,
wobei A substituiert sein kann mit 0, 1, 2 oder 3 Substituenten R^{A}, wobei die Substituenten R^{A} unabhängig voneinander ausgewählt werden aus der Gruppe bestehend aus Halogen, Alkyl, Nitro, Amino, Cyano, Trifluormethyl, Aryl, Heteroaryl, Hydroxy, Alkoxy, Alkylamino, Carboxyl, Alkoxycarbonyl, Aminocarbonyl, Alkylcarbonylamino und Alkylaminocarbonyl,
oder
zwei Substituenten R^{A} zusammen mit den Kohlenstoffatomen, an die sie gebunden sind, ein Cycloalkyl oder Heterocyclyl bilden, wobei dieses Cycloalkyl oder Heterocyclyl substituiert sein kann mit 0, 1 oder 2 Substituenten R^{A-1}, wobei die Substituenten R^{A-1} unabhängig voneinander ausgewählt werden aus der Gruppe bestehend aus Halogen, Nitro, Amino, Trifluormethyl, Hydroxy und Alkoxy,
- B: Aryl oder Heteroaryl bedeutet.

Die erfindungsgemäßen Verbindungen können auch in Form ihrer Salze, Solvate oder Solvate der Salze vorliegen.

Die erfindungsgemäßen Verbindungen können in Abhängigkeit von ihrer Struktur in stereoisomeren Formen (Enantiomere, Diastereomere) existieren. Die Erfindung betrifft daher die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 Kohlenstoffatomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dihydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkylaminocarbonyl, Alkylcarbonylamino und Alkoxycarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, tert-Butyl, n-Pentyl und n-Hexyl.

Alkoxy steht beispielhaft und vorzugsweise für Methoxy, Ethoxy, n-Propoxy, Isopropoxy, tert-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert-Butylamino, n-Pentylamino, n-Hexylamino, *N,N*-Dimethylamino, *N,N*-Diethylamino, *N*-Ethyl-*N*-methylamino, *N-*Methyl-*N*-n-propylamino, *N*-Isopropyl-*N*-n-propylamino, *N*-t-Butyl-*N*-methylamino, *N-*Ethyl-*N*-n-pentylamino und *N*-n-Hexyl-*N*-methylamino.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, tert-Butylaminocarbonyl, n-Pentylaminocarbonyl, n-Hexylaminocarbonyl, *N,N*-Dimethylaminocarbonyl, *N,N*-Diethylaminocarbonyl, *N-Ethyl-N-*methylaminocarbonyl, *N*-Methyl-*N*-n-propylaminocarbonyl, *N*-Isopropyl-*N*-n-propylaminocarbonyl, *N*-t-Butyl-*N*-methylaminocarbonyl, *N*-Ethyl-*N*-n-pentylamino-carbonyl und *N*-n-Hexyl-*N*-methylaminocarbonyl.

Alkylcarbonylamino steht beispielhaft und vorzugsweise für Methylcarbonylamino, Ethylcarbonylamino, n-Propylcarbonylamino, Isopropylcarbonylamino, tert.-Butylcarbonylamino, n-Pentylcarbonylamino und n-Hexylcarbonylamino.

Alkoxycarbonyl steht beispielhaft und vorzugsweise für Methoxycarbonyl, Ethoxycarbonyl, n-Propoxycarbonyl, Isopropoxycarbonyl, tert-Butoxycarbonyl, n-Pentoxycarbonyl und n-Hexoxycarbonyl.

Cycloalkyl steht für eine Cycloalkylgruppe mit in der Regel 3 bis 8, bevorzugt 5 bis 7 Kohlenstoffatomen, beispielhaft und vorzugsweise für Cycloalkyl sind genannt Cyclopropyl, Cyclobutyl, Cyclopentyl, Cyclohexyl und Cycloheptyl.

Cycloalkenyl steht für eine Cycloalkenylgruppe mit in der Regel 3 bis 8, bevorzugt 5 bis 7 Kohlenstoffatomen, beispielhaft und vorzugsweise für Cycloalkenyl sind genannt Cyclopropenyl, Cyclobutenyl, Cyclopentenyl, Cyclohexenyl und Cycloheptenyl.

Aryl steht für einen mono- bis tricyclischen aromatischen Rest mit in der Regel 6 bis 14 Kohlenstoffatomen; beispielhaft und vorzugsweise für Aryl sind genannt Phenyl, Naphthyl und Phenanthrenyl.

Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.

Heterocyclyl steht für einen mono- oder polycyclischen, vorzugsweise mono- oder bicyclischen, heterocyclischen Rest mit in der Regel 4 bis 10, vorzugsweise 5 bis 8 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- bis 8-gliedrige, monocyclische gesättigte Heterocyclylreste mit bis zu zwei Heteroatomen aus der Reihe O, N und S, wie beispielhaft und vorzugsweise Tetrahydrofuran-2-yl, Pyrrolidin-2-yl, Pyrrolidin-3-yl, Pyrrolinyl, Piperidinyl, Morpholinyl, Perhydroazepinyl.

Halogen steht für Fluor, Chlor, Brom und Jod, vorzugsweise für Fluor und Chlor.

Wenn Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen der Formel (I),
worin
- R¹: Wasserstoff oder Methyl bedeutet,
- R^{1'}: Wasserstoff, Methyl oder Fluor bedeutet,
- R²: Wasserstoff bedeutet,
- R³: Wasserstoff, Amino, C₁-C₃-Alkyl, C₁-C₃-Alkoxy, Benzyloxy, C₁-C₃-Alkylamino, C₁-C₃-Alkylcarbonylamino, Phenylcarbonylamino oder Benzylcarbonylamino bedeutet,
- R⁴: Methyl bedeutet,
- R⁵: Fluor, Chlor, Trifluormethyl, Trifluormethoxy, Nitro, Amino, Alkylamino, Hydroxy, Alkyl, Alkoxy, Alkoxycarbonyl, Aminocarbonyl, Phenyl oder 5- bis 6- gliedriges Heteroaryl bedeutet,
oder
zwei Substituenten R⁵ zusammen mit den Kohlenstoffatomen, an die sie gebunden sind, ein 5- bis 6-gliedriges Cycloalkyl oder 5- bis 6-gliedriges Heterocyclyl bilden,
- R⁶: C₂-C₇-Alkyl, C₃-C₇-Cycloalkyl oder C₅-C₇-Cycloalkenyl bedeutet,
wobei R⁶ substituiert sein kann mit 0, 1 oder 2 Substituent R⁶⁻¹, wobei R⁶⁻¹ ausgewählt wird aus der Gruppe bestehend aus Halogen, Trifluormethyl, Alkyl und Methoxy,
- n: eine Zahl 0, 1 oder 2 bedeutet,
wobei bei n gleich 2 die Reste R⁵ gleich oder verschieden sein können,
- m: eine Zahl 1 oder 2 bedeutet,
- A: Phenyl, Naphthyl oder 5-, 6- oder 10-gliedriges Heteroaryl bedeutet,
wobei A substituiert sein kann mit 0, 1 oder 2 Substituenten R^{A}, wobei die Substituenten R^{A} unabhängig voneinander ausgewählt werden aus der Gruppe bestehend aus Halogen, Alkyl, Amino, Cyano, Trifluormethyl, Aryl, Heteroaryl, Hydroxy, Alkoxy, Alkylamino, Alkoxycarbonyl und Aminocarbonyl,
oder
zwei Substituenten R^{A} zusammen mit den Kohlenstoffatomen, an die sie gebunden sind, ein 5- bis 6-gliedriges Cycloalkyl oder 5- bis 6-gliedriges Heterocyclyl bilden, wobei dieses Cycloalkyl oder Heterocyclyl substituiert sein kann mit 0 oder 1 Substituenten R^{A-1}, wobei die Substituenten R^{A-1} unabhängig voneinander ausgewählt werden aus der Gruppe bestehend aus Halogen, Nitro, Amino, Trifluormethyl, Hydroxy und Alkoxy,
- B: Phenyl, Naphthyl oder 5-, 6-, 9- oder 10-gliedriges Heteroaryl bedeutet.

Bevorzugt im Rahmen der vorliegenden Erfindung sind auch Verbindungen der Formel (I), welche der Formel (Ia) entsprechen, 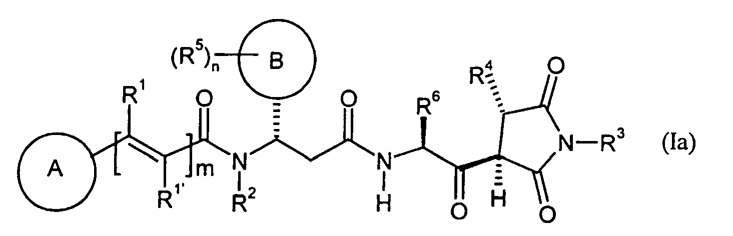 worin
- R¹: Wasserstoff bedeutet,
- R^{1'}: Wasserstoff, Methyl oder Fluor bedeutet,
- R²: Wasserstoff bedeutet,
- R³: Wasserstoff, Amino, Methyl, Methoxy, Ethoxy, Methylamino oder Dimethylamino bedeutet,
- R⁴: Methyl bedeutet,
- R⁵: Fluor, Chlor, Trifluormethyl, Alkoxy, Methoxycarbonyl, C₁-C₄-Alkyl, Phenyl oder Pyridyl bedeutet,
oder
zwei Substituenten R⁵ zusammen mit den Kohlenstoffatomen, an die sie gebunden sind, ein 5- oder 6-gliedriges Heterocyclyl bilden,
- R⁶: C₃-C₆-Alkyl, C₄-C₆-Cycloalkyl oder C₅-C₆-Cycloalkenyl bedeutet,
- n: eine Zahl 0, 1 oder 2 bedeutet,
wobei bei n gleich 2 die Reste R⁵ gleich oder verschieden sein können,
- m: die Zahl 1 bedeutet,
- A: Phenyl, Pyridyl, Imidazolyl, Thienyl, Furanyl, Oxadiazolyl, Pyrazolyl, Pyrazinyl, Pyridazinyl, Pyrimidinyl, Chinolinyl oder Isochinolinyl bedeutet,
wobei A substituiert sein kann mit 0, 1 oder 2 Substituenten R^{A}, wobei die Substituenten R^{A} unabhängig voneinander ausgewählt werden aus der Gruppe bestehend aus Halogen, Alkyl, Cyano, Trifluormethyl, Phenyl und Alkoxy,
oder
zwei Substituenten R^{A} zusammen mit den Kohlenstoffatomen, an die sie gebunden sind, ein 5- oder 6-gliedriges Heterocyclyl bilden,
- B: Phenyl, Naphthyl, Pyridyl, Thienyl, Furanyl, Chinolinyl oder Isochinolinyl bedeutet.

Bevorzugt im Rahmen der vorliegenden Erfindung sind auch Verbindungen der Formel (Ia),
worin
- R¹: Wasserstoff bedeutet,
- R^{1'}: Wasserstoff bedeutet,
- R²: Wasserstoff bedeutet,
- R³: Wasserstoff, Amino, Methylamino oder Dimethylamino bedeutet,
- R⁴: Methyl bedeutet,
- R⁵: Fluor, Chlor, Trifluormethyl, Methoxy, C₁-C₄-Alkyl, Phenyl oder Pyridyl bedeutet,
oder
zwei Substituenten R⁵ zusammen mit dem Phenylring, an den sie gebunden sind, ein 1,3-Benzodioxol oder ein 1,4-Benzodioxan bilden,
- R⁶: Isopropyl, tert-Butyl, Isobutyl, Isopentyl, Cyclobutyl oder Cyclopentyl bedeutet,
- n: eine Zahl 0, 1 oder 2 bedeutet,
wobei bei n gleich 2 die Reste R⁵ gleich oder verschieden sein können,
- m: die Zahl 1 bedeutet,
- A: Phenyl, Pyridyl, Thienyl, Chinolinyl oder Isochinolinyl bedeutet,
wobei A substituiert sein kann mit 0, 1 oder 2 Substituenten R^{A}, wobei die Substituenten R^{A} unabhängig voneinander ausgewählt werden aus der Gruppe bestehend aus Fluor, Chlor, C₁-C₃-Alkyl, Cyano, Trifluormethyl, Phenyl und C₁-C₃-Alkoxy,
oder
zwei Substituenten R^{A} zusammen mit dem Phenylring, an den sie gebunden sind, ein 1,3-Benzodioxol oder ein 1,4-Benzodioxan bilden,
- B: Phenyl, Naphthyl, Thienyl, Chinolinyl oder Isochinolinyl bedeutet.

Bevorzugt im Rahmen der vorliegenden Erfindung sind auch Verbindungen der Formel (I), worin R¹ bis R⁶, A, B, m und n wie oben definiert sind und R⁴ ungleich Wasserstoff ist.

Bevorzugt im Rahmen der vorliegenden Erfindung sind auch Verbindungen der Formel (I), worin R¹ Wasserstoff bedeutet.

Bevorzugt im Rahmen der vorliegenden Erfindung sind auch Verbindungen der Formel (I) oder (Ia), worin R^{1'} Wasserstoff bedeutet.

Bevorzugt im Rahmen der vorliegenden Erfindung sind auch Verbindungen der Formel (I), worin R² Wasserstoff bedeutet.

Bevorzugt im Rahmen der vorliegenden Erfindung sind auch Verbindungen der Formel (I) oder (Ia), worin R³ Wasserstoff oder Amino bedeutet.

Bevorzugt im Rahmen der vorliegenden Erfindung sind auch Verbindungen der Formel (I), worin R⁴ Methyl bedeutet.

Bevorzugt im Rahmen der vorliegenden Erfindung sind auch Verbindungen der Formel (I) oder (Ia), worin n die Zahl Null bedeutet.

Bevorzugt im Rahmen der vorliegenden Erfindung sind auch Verbindungen der Formel (I), worin n die Zahl 1 bedeutet, B Phenyl bedeutet und R⁵ Fluor, Chlor, Trifluormethyl, Alkoxy, C₁-C₄-Alkyl, Phenyl oder Pyridyl bedeutet, wobei R⁵ in der meta- oder para-Position zur Verknüpfungsstelle des Phenyl-Ringes vorliegt. Unter der Verknüpfungsstelle des Phenyl-Ringes wird dabei das Kohlenstoffatom des R⁵ tragenden Phenyl-Ringes verstanden, mit dem der R⁵ tragende Phenyl-Ring gemäß Formel (I) oder (Ia) als B an den Rest der Verbindung gebunden ist.

Bevorzugt im Rahmen der vorliegenden Erfindung sind auch Verbindungen der Formel (I) oder (Ia), worin R⁶ Isopropyl, tert-Butyl, Isobutyl, Isopentyl oder Cyclopentyl bedeutet.

Bevorzugt im Rahmen der vorliegenden Erfindung sind auch Verbindungen der Formel (I), worin m die Zahl 1 bedeutet.

Bevorzugt im Rahmen der vorliegenden Erfindung sind auch Verbindungen der Formel (I) oder (Ia), worin
- A: Phenyl oder Pyridyl bedeutet,
wobei A substituiert sein kann mit 0, 1 oder 2 Substituenten R^{A}, wobei die Substituenten R^{A} unabhängig voneinander ausgewählt werden aus der Gruppe bestehend aus Fluor, Chlor, Cyano, Trifluormethyl, Phenyl und Methoxy.

Bevorzugt im Rahmen der vorliegenden Erfindung sind auch Verbindungen der Formel (I) oder (Ia), worin B Phenyl bedeutet.

Bevorzugt im Rahmen der vorliegenden Erfindung sind auch folgende Verbindungen:
(2*E*)-3-(1,3-Benzodioxol-5-yl)-N-{(1*S*)-3-[((1*S*)-2-methyl-1-{[(3*R,*4*S*)-4-methyl-2,5-dioxo-3-pyrrolidinyl]carbonyl}propyl)amino]-3-oxo-1-phenylpropyl}-2-propenamid,
(2*E*)-3-(1,3-Benzodioxol-5-yl)-N-{(1*S*)-3-[((1*S*)-2,2-dimethyl-1-{[(3*R,*4*S*)-4-methyl-2,5-dioxo-3-pyrrolidinyl]carbonyl}propyl)amino]-3-oxo-1-phenylpropyl}-2-propenamid,
(2*E*)-3-(1,3-Benzodioxol-5-yl)-N-{1-(2,3-dihydro-1,4-benzodioxin-6-yl)-3-[((1*S*)-2-methyl-1-{[(3*R*,4*S*)-4-methyl-2,5-dioxo-3-pyrrolidinyl]carbonyl}propyl)amino]-3-oxopropyl}-2-propenamid,
(2*E*)-N-{1-(2,3-Dihydro-1,4-benzodioxin-6-yl)-3-[((1*S*)-2-methyl-1-{[(3*R*,4*S*)-4-methyl-2,5-dioxo-3-pyrrolidinyl]carbonyl}propyl)amino]-3-oxopropyl}-3-phenyl-2-propenamid,
(2*E*)-N-{(1*S*)-3-[((1*S*)-2-methyl-1-{([(3*R*,4*S*)-4-methyl-2,5-dioxo-3-pyrrolidinyl]-carbonyl}propyl)amino]-3-oxo-1-phenylpropyl}-3-(4-methylphenyl)-2-propenamid,
(2*E*)-3-(2H-Benzo[d]1,3-dioxolan-5-yl)-N-((1S)-2-{N-[(1S)-2-((4S,3R)-4-methyl-2,5-dioxoazolidin-3-yl)-1-cyclopentyl-2-oxoethyl]carbamoyl}-1-phenylethyl)prop-2-enamid,
(2*E*)-N-((1*S*)-2-{N-[(1*S*)-2-((4*S,*3*R*)-4-Methyl-2,5-dioxoazolidin-3-yl)-1-cyclopentyl-2-oxoethyl]carbamoyl}-1-phenylethyl)-3-(4-cyanophenyl)prop-2-enamid,
(2*E*)-N-((1*S*)-2-{N-[(1*S*)-2-((4*S,*3*R*)-1-Amino-4-methyl-2,5-dioxoazolidin-3-yl)-1-cyclopentyl-2-oxoethyl]carbamoyl}-1-phenylethyl)-3-(4-cyanophenyl)prop-2-enamid,
(2*E*)-N-(2-{N-[(1*S*)-2-((4*S*,3*R*)-1-Amino-4-methyl-2,5-dioxoazolidin-3-yl)-1-cyclopentyl-2-oxoethyl]carbamoyl}-1-(2-naphthyl)ethyl)-3-(4-cyanophenyl)prop-2-enamid,
(2*E*)-N-[(1*S*)-2-(N-{(1*S*)-2-[(4*S*,3*R*)-1-(Dimethylamino)-4-methyl-2,5-dioxoazolidin-3-yl]-1-cyclopentyl-2-oxoethyl}carbamoyl)-1-phenylethyl]-3-(4-cyanophenyl)prop-2-enamid.

Gegenstand der Erfindung sind weiterhin Verfahren zur Herstellung der Verbindungen der Formel (I), wobei
nach Verfahren [A]
Verbindungen der Formel 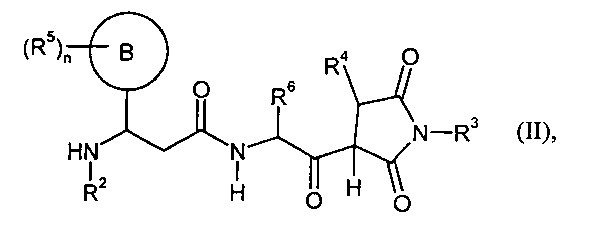 worin R² bis R⁶, B und n die oben angegebene Bedeutung aufweisen, mit Verbindungen der Formel 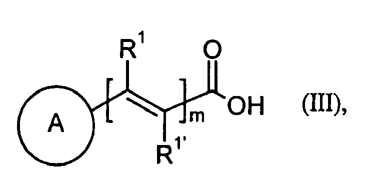 worin R¹ und R^{1'}, A und m die oben angegebene Bedeutung aufweisen,
wobei diese gegebenenfalls in aktivierter Form vorliegen können, umgesetzt werden,
oder
nach Verfahren [B]
Verbindungen der Formel 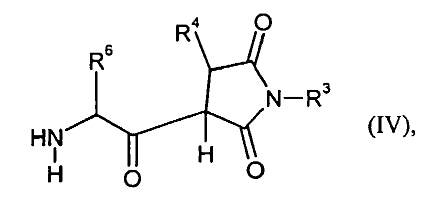 worin R³, R⁴ und R⁶ die oben angegebene Bedeutung aufweisen, mit Verbindungen der Formel 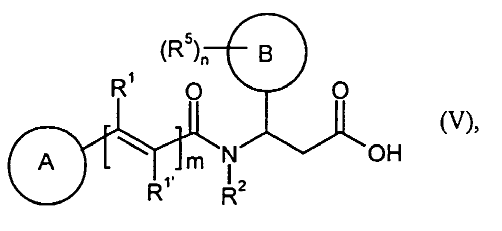 worin R¹, R^{1'}, R², R⁵, A, B, m und n die oben angegebene Bedeutung aufweisen,
wobei diese gegebenenfalls in aktivierter Form vorliegen können, umgesetzt werden.

Zur Überführung der Verbindungen in die aktivierte Form in den obengenannten Verfahren sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodümid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen mit Basen geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Bevorzugt ist die Verwendung von HATU und Diisopropylethylamin oder von EDC mit HOBt und Triethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist eine Mischung von Dichlormethan und Dimethylformamid.

### Verfahren [A]

Die Verbindungen der Formel (II) sind bekannt oder können hergestellt werden, indem Verbindungen der Formel 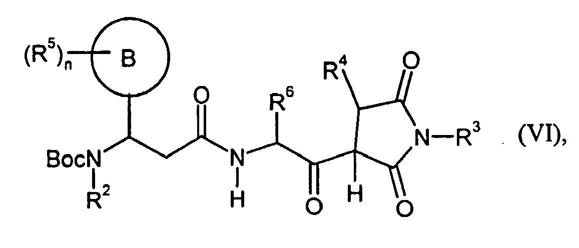 worin R² bis R⁶, B und n die oben angegebene Bedeutung aufweisen,
mit Säure, insbesondere mit Salzsäure oder Trifluoressigsäure versetzt werden. Die Verbindungen der Formel (II) fallen dabei in Form der entsprechenden Salze an, z.B. in Form ihrer Hydrochloride und können in dieser Form weiter eingesetzt werden.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist die Verwendung von Salzsäure in Dioxan oder Trifluoressigsäure in Dichlormethan.

Die Verbindungen der Formel (VI) sind bekannt oder können hergestellt werden, indem Verbindungen der Formel (IV) mit Verbindungen der Formel 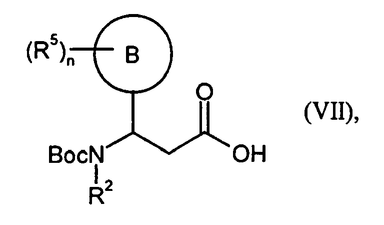 worin R², R⁵, B und n die oben angegebene Bedeutung aufweisen,
wobei diese gegebenenfalls in aktivierter Form vorliegen können, umgesetzt werden.

Zur Überführung der Verbindungen in die aktivierte Form sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP), oder Mischungen aus diesen mit Basen geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Bevorzugt ist die Verwendung von HATU und Diisopropylethylamin oder von EDC mit HOBt und Triethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist eine Mischung von Dichlormethan und Dimethylformamid.

Die Verbindungen der Formel (IV) sind literaturbekannt oder können hergestellt werden, indem Verbindungen der Formel 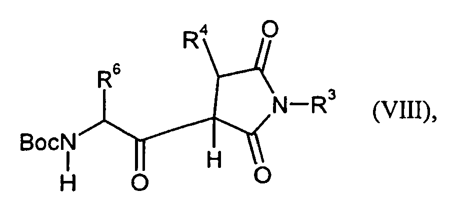 worin R³, R⁴ und R⁶ die oben angegebene Bedeutung aufweisen,
mit Säure, insbesondere mit Salzsäure oder Trifluoressigsäure versetzt werden.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist die Verwendung von Salzsäure in Dioxan oder Trifluoressigsäure in Dichlormethan.

Die Verbindungen der Formel (VII) sind bekannt oder können nach literaturbekannten Vorschriften hergestellt werden. (Bzgl. der Darstellung von aromatischen beta-Aminosäuren siehe: S. Rault, P. Dallemagne, M. Robba, *Bull. Soc. Chim. Fr.,* 1987, 1079-1083; S.G. Davies, et al., *J. Chem. Soc., Chem. Commun.* **1993,** *14,* 1153-1155; bzgl. der Umsetzung zu den tert.-Butoxycarbonyl-geschützten Verbindungen siehe T.W. Greene, P.G.M. Wuts, Protective Groups in Organic Synthesis, 3^{rd} Edt. 1999, J. Wiley & Sons, Inc.).

Die Verbindungen der Formel (VIII) sind bekannt oder können nach literaturbekannten Verfahren hergestellt werden. (Vgl. z.B. S. G. Davies, D. J. Dixon, *J. Chem. Soc., Perkin Trans. 1,* **1998,** *17,* 2635-2643; A. V. Rama Rao, A. K. Singh, Ch. V. N. S. Varaprasad, *Tetrahedron Letters,* **1991,** *32,* 4393-4396).

Die Verbindungen der Formel (III) sind bekannt oder können nach literaturbekannten Verfahren hergestellt werden (Houben-Weyl, Methoden der organischen Chemie, Bd.
E5, Carbonsäuren und Carbonsäure-Derivate, Thieme Verlag, Stuttgart, 1985).

### Verfahren [B]

Die Verbindungen der Formel (V) sind literaturbekannt oder können hergestellt werden, indem Verbindungen der Formel 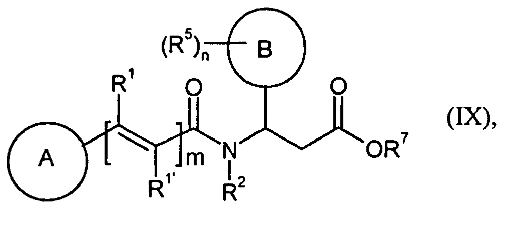 worin R¹, R^{1'}, R², R⁵, A, B, m und n die oben angegebene Bedeutung aufweisen, und R⁷ für einen Alkylrest steht, verseift werden.

Die Verseifung kann nach Standardverfahren durchgeführt werden, z.B. in einem Gemisch aus Ethanol und Wasser mit 40 %iger Natronlauge bei Raumtemperatur oder in einem Gemisch aus Dioxan und Wasser mit methanolischer Kaliumhydroxidlösung.

Die Verbindungen der Formel (IX) sind literaturbekannt oder können hergestellt werden, indem Verbindungen der Formel 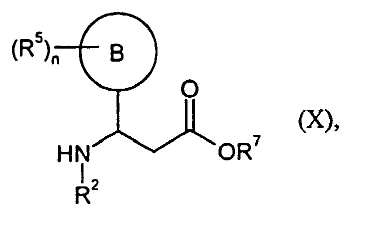 worin R², R⁵, R⁷, B und n die oben angegebene Bedeutung aufweisen,
mit Verbindungen der Formel (III), wobei diese gegebenenfalls in aktivierter Form vorliegen können, umgesetzt werden.

Zur Überführung der Verbindungen in die aktivierte Form in den obengenannten Verfahren sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen mit Basen geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Bevorzugt ist die Verwendung von HATU und Diisopropylethylamin oder von EDC mit HOBt und Triethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Benzol, Xylol, Toluol, Hexan, Cyclohexan, oder Erdölfraktionen, Nitromethan, Dimethylformamid oder Acetonitril oder Ether wie Diethylether, Tetrahydrofuran oder Dioxan. Ebenso ist es möglich, Gemische der Lösemittel einzusetzen. Besonders bevorzugt ist eine Mischung von Dichlormethan und Dimethylformamid.

Die Verbindungen der Formel (X) sind literaturbekannt oder können analog literaturbekannten Verfahren hergestellt werden (z. B. S. G. Davies et. al., *J. Chem. Soc. Chem. Comm.,* **1993,** *14,* 1153-1155; S. J. Faulconbridge et al., *Tetrahedron Letters,* **2000,** *41,* 2679-2682; M. J. Ashton et al., *Heterocycles,* **1989,** *28,* 1015-1035).

Die Synthese kann auch an einem polymeren Träger erfolgen. In diesem Fall bedeutet R² in der Synthesesequenz ein Polymer (Resin), bevorzugt ist die Verwendung von 4-(4-Formyl-3-methoxyphenoxy)butyryl-Aminomethyl-Polystyrol oder eines anderen Harzes, bei dem an ein polymeres Rückgrat wie z.B. Polystyrol oder Block-Copolymere von Polystyrol mit Ethylenglycol über eine Linkergruppe wie z. B. 3-Methoxyphenoxyethyl, 3,5-Dimethoxyphenoxyethoxymethyl oder 3-Methoxyphenoxybutyrylaminomethyl ein Formylrest oder ein anderer Rest gebunden ist, der die Anbindung von Aminen an den polymeren Träger ermöglicht.

Die Herstellung der erfindungsgemäßen Verbindungen kann durch folgende Syntheseschemata verdeutlicht werden:

### Ausgangsverbindungen:

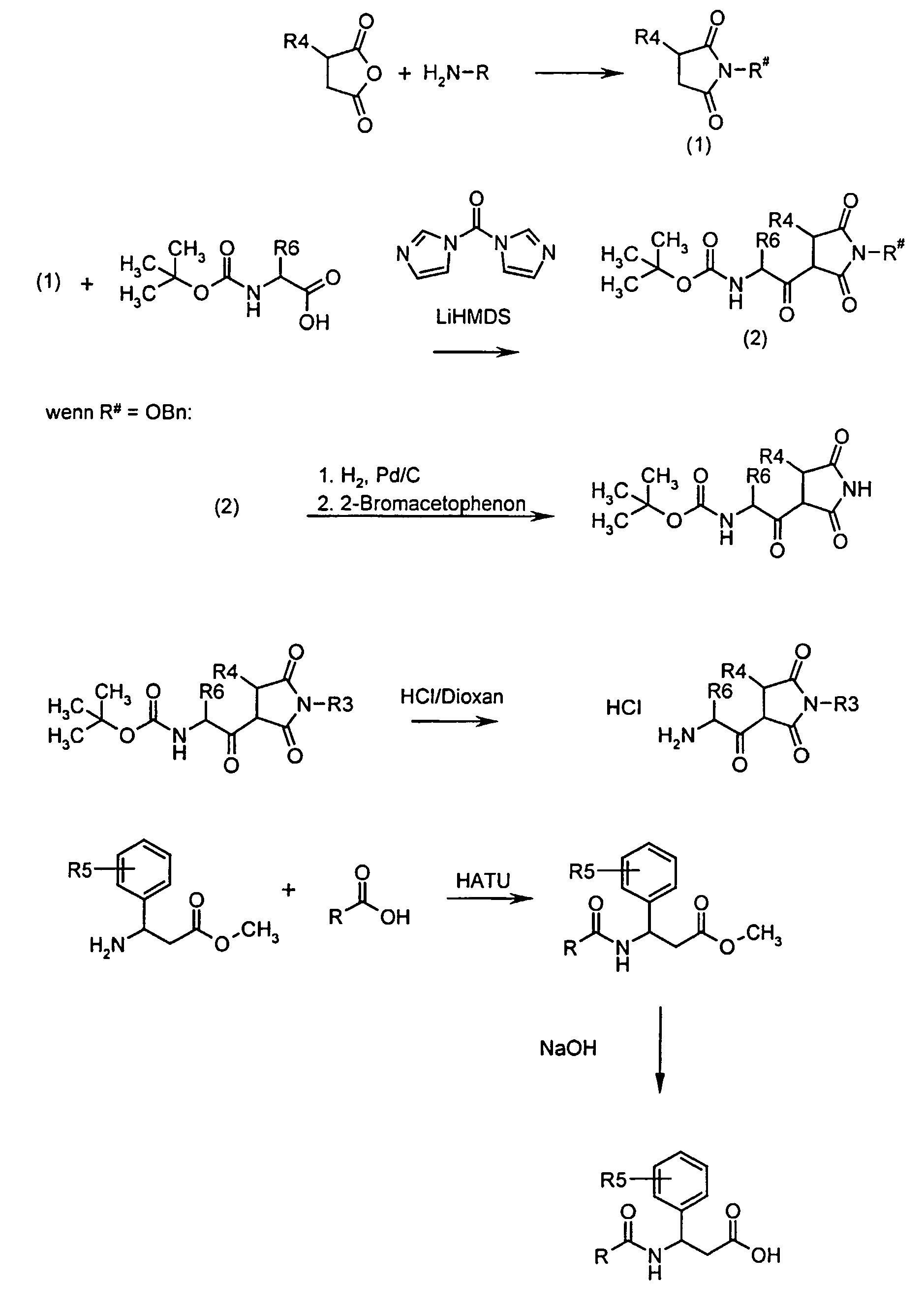

### Herstellungsbeispiele:

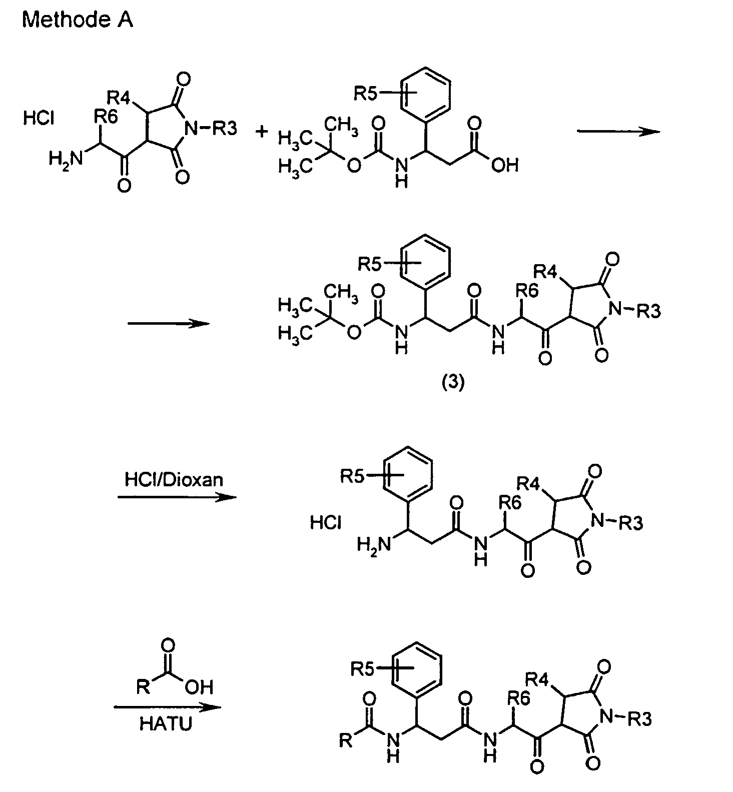

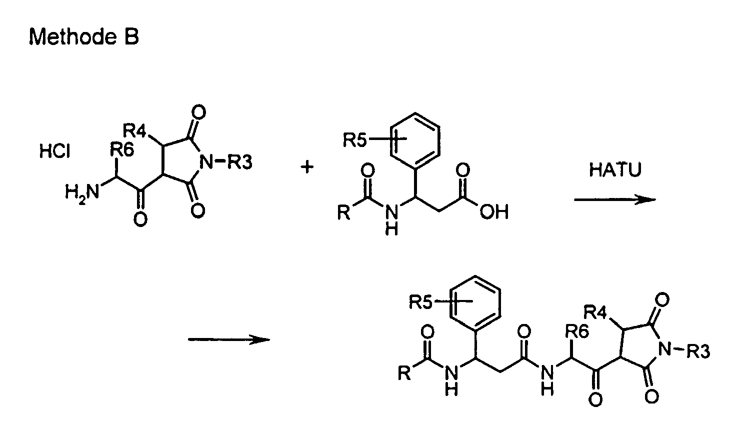

### Festphasensynthese:

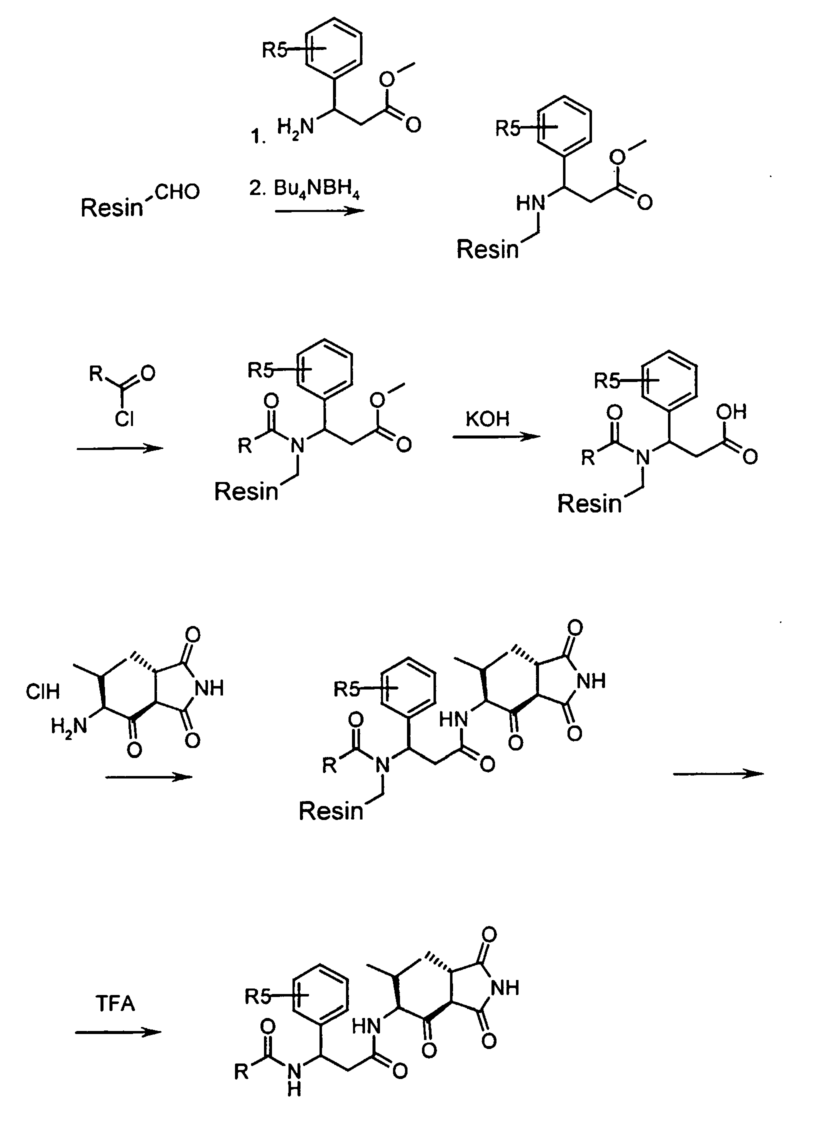

Weiterhin Gegenstand der vorliegenden Erfindung sind Verbindungen der Formel (I) zur Bekämpfung von Erkrankungen, insbesondere bakterieller Erkrankungen, sowie Arzneimittel, enthaltend Verbindungen der Formel (I) in Kombination mit mindestens einem pharmazeutisch verträglichen, pharmazeutisch unbedenklichen Trägerstoff oder sonstigen Hilfsstoff und auch die Verwendung von Verbindungen der Formel (I) zur Herstellung eines Arzneimittels zur Behandlung von bakteriellen Erkrankungen.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z.B. Staphylokokken (Staph. aureus, Staph. epidermidis), Enterokokken (E. faecalis, E. faecius) und Streptokokken (Strept. agalactiae, Strept. pneumoniae); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytocy), Enterobacter (Ent. aerogenes, Ent. agglomerans), Hafnia, Serratia (Serr. marcescens), Providencia, Yersinia, sowie die Gattung Acinetobacter. Darüber hinaus umfaßt das antibakterielle Spektrum strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:

Infektionskrankheiten beim Menschen wie z. B. septische Infektionen, Knochen- und Gelenkinfektionen, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, septische Arthritis, Mastitis, Tonsillitis, Genital-Infektionen und Augeninfektionen.

Außer beim Menschen können bakterielle Infektionen auch bei anderen Spezies behandelt werden. Beispielhaft seien genannt:
Schwein: Coli-diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsie, Yersinia, erweitert.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Bevorzugt ist die parenterale, insbesondere die intravenöse Applikation.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe (Exzipienten). Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und / oder Geruchskorrigentien.

Im Allgemeinen hat es sich als vorteilhaft erwiesen, bei parenteraler Applikation Mengen von etwa 5 bis 250 mg/kg Körpergewicht je 24 Stunden zur Erzielung wirksamer Ergebnisse zu verabreichen. Bei oraler Applikation beträgt die Menge etwa 5 bis 100 mg/kg Körpergewicht je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

Reaktionsschemata, die bei Allgemeinen Vorschriften gezeigt werden, zeigen eine Auswahl an Beispielen, sind aber jeweils für alle Beispiele anwendbar die darauf Bezug nehmen.

### Abkürzungen:

- Boc: tert.-Butoxycarbonyl
- CDCl₃: Deuterochloroform
- DCI: Direkte Chemische Ionisation
- DIEA: *N,N*-Diisopropylethylamin
- DMSO: Dimethylsulfoxid
- d. Th.: der Theorie
- EDC: N-(3-Dimethylaminoisopropyl)-N'-ethylcarbodiimid-Hydrochlorid
- eq.: Äquivalent
- ESI: Elektrospray-Ionisation (bei MS)
- Fmoc: Fluorenylmethoxycarbonyl
- h: **Stunde**
- HATU: *O*-(7-Azabenzotriazol-1-yl)-*N,N,N',N'*-tetramethyluronium-Hexafluorphosphat
- HOBt: 1-Hydroxybenzotriazol
- HPLC: **Hochdruck-, Hochleistungsflüssiechromatographie**
- LC-MS: Flüssigchromatographie-gekoppelte Massenspektroskopie
- MS: Massenspektroskopie
- NMR: Kernresonanzspektroskopie
- **PS-DIEA**: *N,N*-Diisopropylethylamin-Polystyrol (-Harz)
- R_{f}: Retentionsindex (bei DC)
- RP-HPLC: Reverse Phase HPLC
- RT: Raumtemperatur
- Rₜ: Retentionszeit (bei HPLC)
- THF: Tetrahydrofuran

### HPLC und LC-MS Methoden:

**Methode 1:** Säule: Kromasil C18, L-R Temperatur: 30°C, Fluß = 0.75 mlmin⁻¹, Eluent: A = 0.01 M HClO₄, B = Acetonitril, Gradient: → 0.5 min 98%A → 4.5 min 10%A → 6.5 min 10%A
**Methode 2:** Säule: Kromasil C18 60*2, L-R Temperatur: 30°C, Fluß = 0.75 mlmin⁻¹, Eluent: A = 0.01 M H₃PO₄, B = Acetonitril, Gradient: → 0.5 min 90%A → 4.5 min 10%A → 6.5 min 10%A
**Methode 3:** Säule: Kromasil C18 60*2, L-R Temperatur: 30°C, Fluß = 0.75 mlmin⁻¹, Eluent: A = 0.005 M HClO₄, B = Acetonitril, Gradient: → 0.5 min 98%A → 4.5 min 10%A → 6.5 min 10%A
**Methode 4:** Säule: Symmetry C18 2.1x150 mm, Säulenofen: 50°C, Fluß = 0.6 mlmin⁻¹, Eluent: A = 0.6 g 30%ige Salzsäure/ 1 Wasser, B = Acetonitril, Gradient: 0.0 min 90%A → 4.0 min 10%A → 9 min 10%A
**Methode 5:** Instrument Micromass Quattro LCZ
   Säule Symmetry C18, 50 mm x 2.1 mm, 3.5 µm, Temperatur: 40°C, Fluss = 0.5 mlmin⁻¹, Eluent A = Acetonitril + 0.1 % Ameisensäure, Eluent B = Wasser + 0.1 % Ameisensäure, Gradient: 0.0 min 10% A → 4 min 90% A → 6 min 90% A
**Methode 6:** Instrument Micromass Platform LCZ
   Säule Symmetry C18, 50 mm x 2.1 mm, 3.5 µm, Temperatur: 40°C, Fluss = 0.5 mlmin⁻¹, Eluent A = Acetonitril + 0.1% Ameisensäure, Eluent B = Wasser + 0.1 % Ameisensäure, Gradient: 0.0 min 10% A → 4 min 90% A → 6 min 90% A
**Methode 7:** Instrument Micromass Quattro LCZ
   Säule Symmetry C18, 50 mm x 2.1 mm, 3.5 µm, Temperatur: 40°C, Fluss = 0.5 mlmin⁻¹, Eluent A = Acetonitril + 0.1 % Ameisensäure, Eluent B = Wasser + 0.1 % Ameisensäure, Gradient: 0.0 min 5% A → 1 min 5% A → 5 min 90% A → 6 min 90% A
**Methode 8:** Säule: Symmetry C18 2.1x150 mm, 5 µm, Säulenofen: 70°C, Fluß = 0.9 mlmin⁻¹, Eluent: A = Acetonitril, B = 0.3 g 30%ige Salzsäure/ 1 Wasser, Gradient: 0.0 min 2% A → 2.5 min 95% A → 5 min 95% A
**Methode 9:** Säule: Symmetry C18 3.9x150 mm, Säulenofen: 40°C, Fluß = 1.5 mlmin⁻¹, Eluent: A = Wasser + 0.05% H₃PO₄, , B = Acetonitril, Gradient: 0.0 min 10% B → 0.6 min 10% B → 3.8 min 100% B → 5.0 min 100% B.
**Methode 10:** Instrument: Waters Alliance 2790 LC; Säule: Symmetry C18, 50 mm x 2.1 mm, 3.5 µm; Eluent A: Wasser + 0.1% Ameisensäure, Eluent B: Acetonitril + 0.1 % Ameisensäure; Gradient: 0.0 min 5% B → 5.0 min 10% B → 6.0 min 10% B; Temperatur: 50°C, Fluss: 1.0 ml/min, UV-Detektion: 210 nm.
**Methode 11:** Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05% Ameisensäure, Eluent A: Wasser + 0.05% Ameisensäure; Gradient: 0.0 min 10%B → 3.5 min 90%B → 5.5 min 90% B; Ofen: 50°C, Fluss: 0.8 ml/min, UV-Detektion: 210 nm.
**Methode 12:** Instrument: Waters Alliance 2790 LC; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 5% B → 4.5 min 10% B → 5.5 min 10% B; Temperatur: 50°C, Fluss: 1.0 ml/min, UV-Detektion: 210 nm.
**Methode 13:** Instrument: Micromass Quattro LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 90% A → 4.0 min 10% A → 6.0min 10% A; Ofen: 40°C, Fluss: 0.5 ml/min, UV-Detektion: 208-400 nm.
**Methode 14:** Instrument: Micromass Platform LCZ, HP1100; Säule: Symmetry C18, 50 mm x 2.1 mm, 3.5 µm; Eluent A: Wasser + 0.05% Ameisensäure, Eluent B: Acetonitril + 0.05% Ameisensäure; Gradient: 0.0 min 90%A → 4.0 min 10% A → 6.0 min 10% A; Ofen: 40°C, Fluss: 0.5 ml/min, UV-Detektion: 208-400 nm.
**Methode 15:** Instrument: Waters Alliance 2790 LC; Säule: Symmetry C 18, 50 mm x 2.1 mm, 3.5 µm; Eluent A: Wasser + 0.05% Ameisensäure, Eluent B: Acetonitril + 0.05% Ameisensäure; Gradient: 0.0 min 10% B → 4.0 min 90% B → 6.0 min 90% B; Temperatur: 50°C, Fluss: 0.0 min 0.5 ml/min → 4.0 min 0.8 ml/min, UV-Detektion: 210 nm.
**Methode 16:** Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05% Ameisensäure, Eluent A: Wasser + 0.05% Ameisensäure; Gradient: 0.0min 5%B 4.5min 90%B 5.5min 90%B; Ofen: 50 °C, Fluss: 1.0ml/, UV-Detektion: 210 nm
**Methode 17:** Gerätetyp MS: Micromass ZQ; Gerätetyp HPLC: Waters Alliance 2790; Säule: Uptisphere C 18, 50 mm x 2.0 mm, 3.0 µm; Eluent B: Acetonitril + 0.05% Ameisensäure, Eluent A: Wasser + 0.05% Ameisensäure; Gradient: 0.0min 5%B → 2.0min 40%B → 4.5min 90%B → 5.5min 90%B; Ofen: 45 °C, Fluss: 0.0min 0.75ml/min → 4.5min 0.75ml/min→ 5.5min 1.25ml/min, UV-Detektion: 210 nm
**Methode 18:** Instrument: Micromass Platform LCZ mit HPLC Agilent Serie 1100; Säule: Grom-SIL120 ODS-4 HE, 50 mm x 2.0 mm, 3 µm; Eluent A: 11 Wasser + 1ml 50%ige Ameisensäure, Eluent B: 11 Acetonitril + 1ml 50%ige Ameisensäure ; Gradient: 0.0min 100%A → 0.2min 100%A → 2.9min 30%A → 3.1min 10%A → 4.5min 10%A; Ofen: 55°C, Fluss: 0.8ml/min, UV-Detektion: 208-400 nm.
**Methode 19:** Instrument: Micromass Quattro LCZ, mit HPLC Agilent Serie 1100 ; Säule: Grom-SIL120 ODS-4 HE, 50 mm x 2.0 mm, 3 µm; Eluent A: 11 Wasser + 1ml 50%ige Ameisensäure, Eluent B: 11 Acetonitril + 1ml 50%ige Ameisensäure; Gradient: 0.0min 100%A → 0.2min 100%A → 2.9min 30%A → 3.1min 10%A → 4.5min 10%A; Ofen: 55°C, Fluss: 0.8ml/min, UV-Detektion: 208-400 nm.
**Methode 20:** Gerätetyp MS: Micromass ZQ; Gerätetyp HPLC: Waters Alliance 2790; Säule: Grom-Sil 120 ODS-4 HE 50 x 2 mm, 3.0 µm; Eluent B: Acetonitril + 0.05% Ameisensäure, Eluent A: Wasser + 0.05% Ameisensäure; Gradient: 0.0min 5%B → 2.0min 40%B → 4.5min 90%B→ 5.5min 90%B; Ofen: 45 °C; Fluss: 0.0min 0.75ml/min → 4.5min 0.75ml/min→ 5.5min 1.25ml/min; UV-Detektion: 210 nm.
**Methode 21:** Gerätetyp MS: Micromass ZQ; Gerätetyp HPLC: Waters Alliance 2790; Säule: Grom-Sil 120 ODS-4 HE 50x2 mm, 3.0 µm; Eluent B: Acetonitril + 500ul 50%ige Ameisensäure /1; Eluent A: Wasser + 500ul 50%ige Ameisensäure /1; Gradient: 0.0min 0%B → 0.2min 0%B → 2.9min 70%B→ 3.1min 90%B → 4.5min 90%B, Ofen: 50 °C, Fluss:0.8 ml/min; UV-Detektion: 210 nm.
**Methode 22:** Instrument: Micromass Quattro LCZ mit HPLC Agilent Serie 1100; Säule: UPTISPHERE HDO, 50 mm x 2.0 mm, 3 µm; Eluent A: 11 Wasser + 1ml 50%ige Ameisensäure, Eluent B: 11 Acetonitril + 1ml 50%ige Ameisensäure; Gradient: 0.0min 100%A → 0.2min 100%A → 2.9min 30%A → 3.1min 10%A → 4.5min 10%A; Ofen: 55°C, Fluss: 0.8ml/min, UV-Detektion: 208-400 nm.

### Ausgangsverbindungen;

### Beispiel 1A

### (3S)-1,3-Dimethyl-2,5-pyrrolidindion

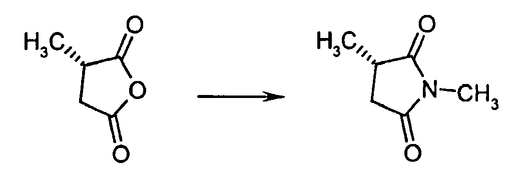

600 mg (5.26 mmol) (3*S*)-3-Methyldihydro-2,5-furandion (Darstellung: S. G. Davies, D. J. Dixon, *J. Chem. Soc., Perkin Trans. 1,* **1998,** 17, 2635 - 2643) werden zusammen mit 559 mg (0.77 ml, 5.52 mmol) Triethylamin in 5 ml Dichlormethan bei 0°C vorgelegt und mit 373 mg (5.52 mmol) Methylamin Hydrochlorid versetzt. Das Reaktionsgemisch wird über Nacht bei Raumtemperatur gerührt und dann portionsweise mit 938 mg (5.78 mmol) N,N-Carbonyldiimidazol versetzt. Es wird 1.5 h bei Raumtemperatur und 30 min bei Rückflusstemperatur gerührt. Nach Abkühlen auf Raumtemperatur wird das Reaktionsgemisch mit 5%iger Salzsäure und Wasser gewaschen, die organische Phase wird über Magnesiumsulfat getrocknet, filtriert und eingeengt und das Produkt wird am Hochvakuum getrocknet. Es werden 605 mg des Produktes erhalten (88 % der Theorie).
MS (ESI+): m/z (%) = 128 (M+H⁺) (100).
HPLC (Methode 6): Rₜ = 0.81 min.
¹H-NMR (300 MHz, CDCl₃): δ = 3.10 (dd, 1 H), 2.99 (s, 3 H), 2.90-2.82 (m, 1 H), 2.32 (dd, 1 H), 1.35 (d, 3 H).

### Beispiel 2A

### (3R,4S)-3-[(2S)-2-(tert-Butoxycarbonyl)amino-3-methylbutanoyl]-1,4-dimethyl-2,5-pyrrolidindion

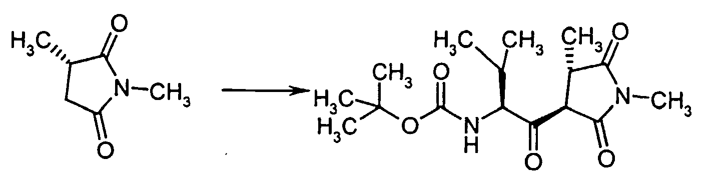

684 mg (3.15 mmol) N-(tert.-Butoxycarbonyl)-L-valin und 561 mg (3.46 mmol) N,N-Carbonyldiimidazol werden in 4 ml Tetrahydrofuran 2 h lang bei Raumtemperatur gerührt. Zu diesem Gemisch werden dann 400 mg (3.15 mmol) (3*S*)-3-1,3-Dimethyl-2,5-pyrrolidindion gegeben und die gesamte Mischung wird innerhalb von 30 min zu 6.3 ml einer auf -65°C gekühlten 1 molaren Lösung von Lithium-hexamethyldisilazid in THF getropft. Nach beendeter Zugabe wird weitere 15 min bei - 65°C gerührt, bevor 6 ml gesättigter wässriger Ammoniumchlorid-Lösung zugegeben werden. Nach Erwärmung auf Raumtemperatur wird das Reaktionsgemisch mit Diethylether verdünnt und die organische Phase mit gesättigter wässriger Natriumchlorid-Lösung gewaschen und anschließend eingeengt. Das Rohprodukt wird durch RP-HPLC (Eluens:Wasser-Acetonitril, Gradient) gereinigt. Es werden 223 mg (22 % der Theorie) des gewünschten Produkts erhalten.
MS (ESI-): m/z (%) = 325 (M-H⁺) (35).
HPLC (Methode 5): Rₜ = 3.99 min.
¹H-NMR (200 MHz, CDCl₃): δ = 5.70 (br. d, 1 H), 4.57 (dd, 1 H), 3.78 (d, 1 H), 3.47-3.30 (m, 1 H), 2.98 (s, 3 H), 2.50- 2.32 (m, 1 H), 1.46 (s, 9 H), 1.32 (d, 3 H), 1.02 (d, 3 H), 0.80 (d, 3 H).

Analog zu Beispiel 2A lassen sich durch Umsetzung der entsprechenden N-*tert*.-Butoxycarbonyl-geschützten Aminosäuren mit (3*S*)-1-(Benzyloxy)-3-methyl-2,5-pyrrolidindion (Darstellung: S. G. Davies, D. J. Dixon, *J. Chem. Soc., Perkin Trans. 1,* **1998,** *17,* 2635 - 2643) folgende Derivate (Beispiele 3A bis 5A) darstellen:

### Beispiel 3A

### (3R,4S)-1-Benzyloxy-3-[(2S)-2-(tert-butoxycarbonyl)amino-3,3-dimethylbutanoyl]-4-methyl-2,5-pyrrolidindion

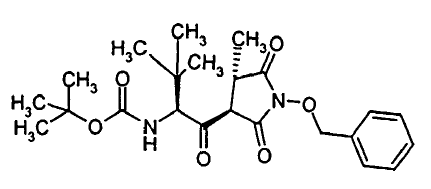

MS (ESI-): m/z (%) = 431 (M-H⁺) (100).
HPLC (Methode 6): Rₜ = 4.87 min.

### Beispiel 4A

### (3R,4S)-1-Benzyloxy-3-[(2S)-2-(tert-butoxycarbonyl)amino-4-methylpentanoyl]-4-methyl-2,5-pyrrolidindion

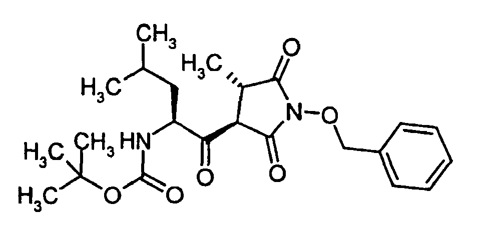

MS (ESI-): m/z (%) = 431 (M-H⁺) (100).
HPLC (Methode 6): Rₜ = 4.88 min.

### Beispiel 5A

### (3R,4S)-1-Benzyloxy-3-[(2S)-2-(tert-butoxycarbonyl)amino-butanoyl)-4-methyl-2,5-pyrrolidindion

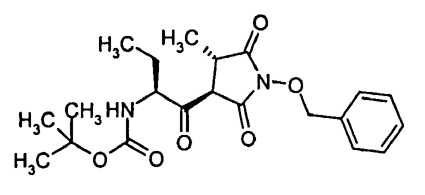

MS (ESI-): m/z (%) = 403 (M-H⁺) (100).
HPLC (Methode 6): Rₜ = 4.54 min.

### Allgemeine Vorschrift A: Reduktive Entschützung von 1-Benzyloxy-2,5-pyrrolidindionen

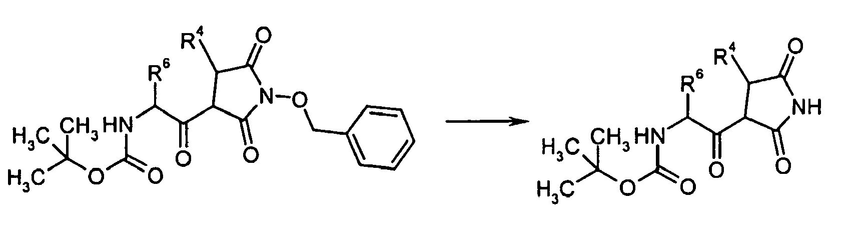

Die Entschützung erfolgt analog zu S. G. Davies, D. J. Dixon, *J. Chem. Soc., Perkin Trans. 1* 1998, 2635-2643.
Das 1-Benzyloxy-2,5-pyrrolidindion (1 eq.) wird in Methanol oder Ethanol gelöst (ca. 0.02 mol/l), mit einer katalytischen Menge Palladium-Kohle (10%) versetzt und 1 h unter Wasserstoffatmosphäre (Normaldruck) gerührt. Dann wird das Reaktionsgemisch filtriert und eingeengt. Der Rückstand wird in Acetonitril gelöst (ca. 0.05 mol/l) und zu einer Lösung von 2-Bromacetophenon (1 eq) in Acetonitril (ca. 0.03 mol/l) bei Raumtemperatur getropft. Danach werden über einen Zeitraum von 2 h 1.5 eq. Triethylamin in Acetonitril (ca. 0.35 mol/l) zu der Reaktionsmischung getropft. Das Reaktionsgemisch wird über Nacht bei Raumtemperatur gerührt, eingeengt und das Rohprodukt wird mittels RP-HPLC (Eluent: Acetonitril/Wasser oder Acetonitril/Wasser + 0.3 ml 37%ige Salzsäure/l, Gradient) gereinigt.

### Beispiel 6A

### (3R,4S)-3-[(2S)-2-(tert-Butoxycarbonyl)amino-3,3-dimethylbutanoyl]-4-methyl-2,5-pyrrolidindion

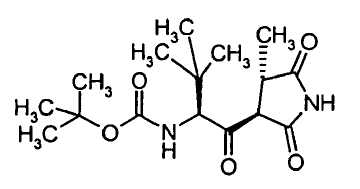

Die Darstellung erfolgt nach der Allgemeinen Vorschrift A.
MS (ESI+): m/z (%) = 327 (M+H⁺) (100).
HPLC (Methode 5): Rₜ = 3.87 min.

### Beispiel 7A

### (3R,4S)-3-[(2S)-2-(tert-Butoxycarbonyl)amino-4-methylpentanoyl)-4-methyl-2,5-pyrrolidindion

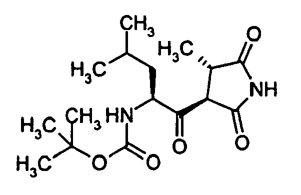

Die Darstellung erfolgt nach der Allgemeinen Vorschrift A.
MS (ESI-): m/z (%) = 325 (M-H⁺) (100).
HPLC (Methode 5): Rₜ = 3.91 min.

### Beispiel 8A

### (3R,4S)-3-[(2S)-2-(tert-Butoxycarbonyl)amino-butanoyl]-4-methyl-2,5-pyrrolidindion

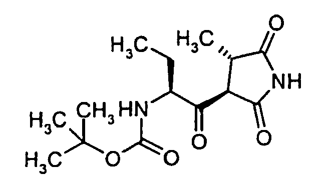

Die Darstellung erfolgt nach der Allgemeinen Vorschrift A.
MS (ESI-): m/z (%) = 297 (M-H⁺) (100).
HPLC (Methode 6): Rₜ = 3.50 min.

### Beispiel 9A

### (3R,4S)-3-[(2S)-2-Amino-3-methylbutanoyl]-4-methyl-2,5-pyrrolidindion Hydrochlorid

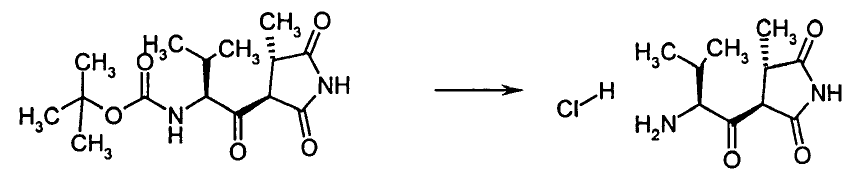

Zu einer auf 0°C abgekühlten Lösung von 4.40 g (14.09 mmol) (3*R*,4*S*)-3-[(2*S*)-2-(tert-Butoxycarbonyl)amino-3-methylbutanoyl]-4-methyl-2,5-pyrrolidindion (Darstellung: S. G. Davies, D. J. Dixon, *J. Chem. Soc., Perkin Trans. 1,* **1998,** 17, 2635 - 2643) werden 35 ml 4N Salzsäure-Lösung in 1,4-Dioxan getropft. Nach Ende der Zugabe wird die Mischung auf Raumtemperatur erwärmt und 2 h gerührt, bevor die Mischung im Vakuum eingeengt wird. Das Rohprodukt kann direkt in der nächsten Stufe eingesetzt werden. Gegebenenfalls wird der Rückstand mit Diethylether behandelt und die ausgefallenen Kristalle werden abfiltriert und am Hochvakuum getrocknet. Ausbeute: 2.99 g farblose Kristalle (86 % der Theorie).
MS (ESI+): m/z (%) = 213 (M+H⁺) (100).
HPLC (Methode 4): Rₜ = 0.41 min.

Analog zu Beispiel 9A können aus den entsprechenden tert.-Butoxycarbonylamino-Derivaten durch Behandlung mit Salzsäure/Dioxan folgende Amine (Beispiele 10A bis 13A) in Form ihrer Hydrochloride dargestellt werden:

### Beispiel 10A

### (3R,4S)-3-[(2S)-2-Amino-3-methylbutanoyl]-1,4-dimethyl-2,5-pyrrolidindion Hydrochlorid

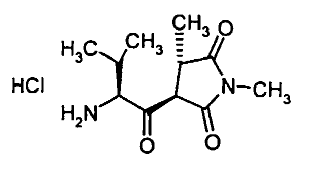

MS (ESI+): m/z (%) = 227 (M+H⁺) (80).

### Beispiel 11A

### (3R,4S)-3-[(2S)-2-Amino-3,3-dimethylbutanoyl]-4-methyl-2,5-pyrrolidindion Hydrochlorid

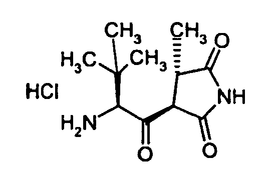

MS (ESI+): m/z (%) = 227 (M+H⁺) (100).

### Beispiel 12A

### (3R,4S)-3-[(2S)-2-Amino-4-methylpentanoyl]-4-methyl-2,5-pyrrolidindion Hydrochlorid

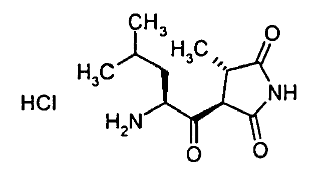

MS (ESI+): m/z (%) = 227 (M+H⁺) (100).

### Beispiel 13A

### (3R,4S)-3-[(2S)-2-Amino-butanoyl]-4-methyl-2,5-pyrrolidindion Hydrochlorid

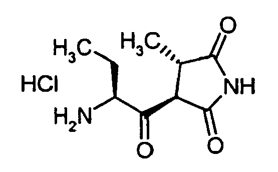

MS (ESI+): m/z (%) = 199 (M+H⁺) (100).

### Allgemeine Vorschrift B: Umsetzung von 3-Aminopropionsäurealkylestern mit Carbonsäuren

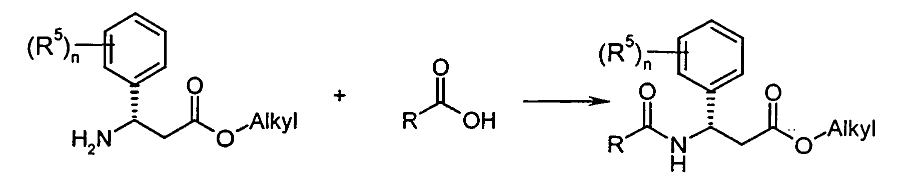

Zu einer Lösung des Carbonsäurederivates (1.2 - 1.5 eq.) in absolutem Dichlormethan oder einer Mischung (5:1 bis 1:1) von absolutem Dichlormethan und N,N-Dimethylformamid (ca. 0.1 bis 0.3 mol/l) werden bei 0°C zunächst eine äquimolare Menge HATU und dann der 3-Aminopropionsäurealkylester (1 eq., gegebenenfalls als Lösung in N,N-Dimethylformamid oder Dichlormethan/N,N-Dimethylformamid Gemischen) zugegeben. Anschließend wird bei 0°C eine Lösung von 2.5 - 3.5 eq. Diisopropylethylamin in einem 1: 1 Gemisch von absolutem Dichlormethan und N,N-Dimethylformamid (0.2 - 1 mol/l) über einen Zeitraum von 1 h zugetropft. Nach Ende der Zugabe wird die Reaktionsmischung weitere 30 min bei 0°C und anschließend über Nacht bei Raumtemperatur gerührt, bevor im Vakuum eingeengt wird. Das Produkt kann durch Chromatographie an Silicagel (Eluenten: Gemische aus Cyclohexan/Ethylacetat oder Gemische aus Dichlormethan und Ethanol) oder durch RP-HPLC (Eluenten: variable Gradienten aus Wasser und Acetonitril), alternativ durch eine Kombination beider Verfahren, erhalten werden.
((*S*)-3-Amino-3-phenyl-propionsäure-methylester, Darstellung: S. G. Davies et. al., *J. Chem. Soc., Chem. Comm.,* **1993,** *14*, 1153-1155).

Alternativ kann die Umsetzung auch nach folgendem Verfahren erfolgen:
Zu einer Lösung des 3-Aminopropionsäurealkylesters (1 eq.) in absolutem Dichlormethan oder einer Mischung (5:1 bis 1:1) von absolutem Dichlormethan und N,N-Dimethylformamid (ca. 0.1 bis 0.3 mol/l) werden das Carbonsäurederivat (1.1 - 1.5 eq.), Triethylamin (3 eq.), HOBt (3 eq.) und abschließend 1.2 eq. EDC gegeben. Die Reaktionsmischung wird bei Raumtemperatur gerührt (2 h bis über Nacht), bevor im Vakuum eingeengt wird. Der Rückstand wird in Essigsäureethylester oder Dichlormethan aufgenommen und die organische Phase wird mit Wasser, gesättigter Natriumhydrogencarbonatlösung und gesättigter Kochsalzlösung gewaschen, über Natriumsulfat getrocknet, filtriert und eingeengt. Das Produkt kann durch Chromatographie an Silicagel (Eluenten: Gemische aus Cyclohexan/ Essigsäureethylester oder Gemische aus Dichlormethan und Ethanol) oder durch RP-HPLC (Eluenten: variable Gradienten aus Wasser und Acetonitril), alternativ durch eine Kombination beider Verfahren, gereinigt werden.

### Beispiel 14A

### (3S)-3-{[(2E)-3-(1,3-Benzodioxol-5-yl)-2-propenoyl]amino}-3-phenylpropionsäuremethylester

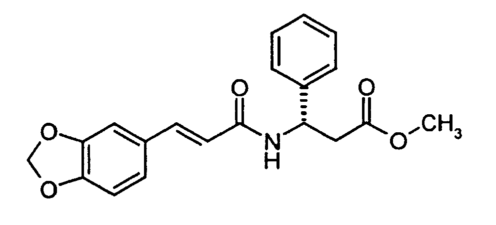

Die Darstellung erfolgt nach der Allgemeinen Vorschrift B.
¹H-NMR (300 MHz, d₆-DMSO): δ = 8.50 (d, 1 H), 7.38-7.21 (m, 6 H), 7.14 (d, 1 H), 7.06 (dd, 1 H), 6.93 (d, 1 H), 6.49 (d, 1 H), 6.05 (s, 2 H), 5.32 (q, 1 H), 3.56 (s, 3 H), 2.91-2.78 (m, 2 H).
MS (ESI+): m/z (%) = 354 (M+H⁺) (65).

### Allgemeine Vorschrift C: Verseifung der Propionsäurealkylester

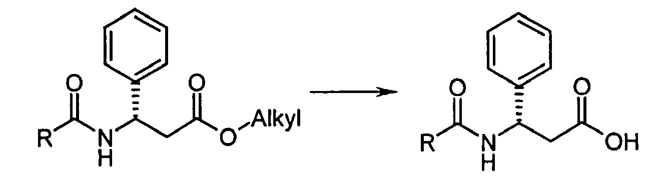

Der Propionsäurealkylester wird in einem 3:1 Gemisch aus Ethanol und Wasser vorgelegt (ca. 0.1 - 0.15 mol/l) und mit 5 eq. 40%iger Natronlauge versetzt. Das Reaktionsgemisch wird 24 h bei Raumtemperatur gerührt, mit verdünnter Salzsäure angesäuert (ca. pH 3) und eingeengt. Der Rückstand wird in Essigsäureethylester aufgenommen und mit gesättigter wässriger Natriumchloridlösung gewaschen. Die organische Phase wird über Magnesiumsulfat getrocknet, filtriert und eingeengt. Das erhaltene Produkt kann ohne weitere Reinigung in der nächsten Stufe eingesetzt werden.

Alternativ kann auch folgende Methode Verwendung finden:
Der Propionsäurealkylester wird in einem 1:1 Gemisch aus Dioxan und Wasser vorgelegt (ca. 0.1 - 0.15 mol/l) und mit 3 eq. einer Lösung von Kaliumhydroxid in Methanol (100 mg/ml) versetzt. Das Reaktionsgemisch wird 2 h bei Raumtemperatur gerührt und anschließend eingeengt. Der Rückstand wird in Wasser aufgenommen und mit verdünnter Salzsäure angesäuert. Die wässrige Phase wird dreimal mit einem 1:1 Gemisch aus Dichlormethan und Essigsäureethylester extrahiert. Die vereinigten organischen Phasen werden über Natriumsulfat getrocknet, filtriert und eingeengt. Das erhaltene Produkt kann ohne weitere Reinigung in der nächsten Stufe eingesetzt werden.

### Beispiel 15A

### (3S)-3-{[(2E)-3-(1,3-Benzodioxol-5-yl)-2-propenoyl]amino}-3-phenylpropionsäure

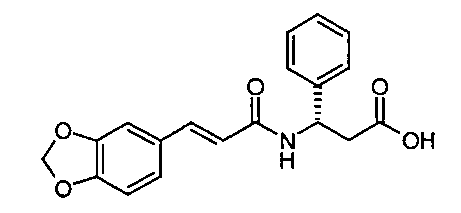

Die Darstellung erfolgt nach der Allgemeinen Vorschrift C.
¹H-NMR (300 MHz, d₆-DMSO): δ = 12.21 (s, 1 H), 8.50 (d, 1 H), 7.38-7.21 (m, 6 H), 7.14 (d, 1 H), 7.06 (dd, 1 H), 6.93 (d, 1 H), 6.50 (d, 1 H), 6.06 (s, 2 H), 5.31 (q, 1 H), 2.83-2.66 (m, 2 H).
MS (ESI+): m/z (%) = 340 (M+H⁺) (85).
HPLC (Methode 5): Rₜ = 3.47 min.

Die so erhaltenen Propionsäurederivate können nach der Allgemeinen Vorschrift D (Acylierung von 3-[2-Amino-alkanoyl]-2,5-pyrrolidindion Hydrochlorid Derivaten mit Carbonsäurederivaten) umgesetzt werden.

### Allgemeine Vorschrift D: Acylierung von 3-[2-Amino-alkanoyl]-2,5-pyrrolidindion Hydrochlorid Derivaten mit Carbonsäurederivaten

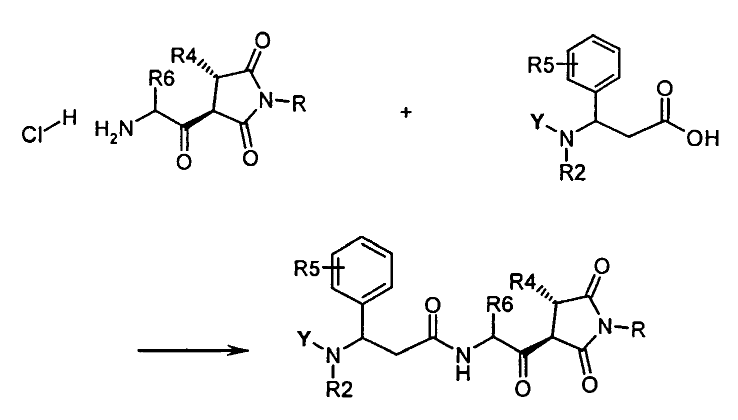

Zu einer Lösung des Carbonsäurederivates (1.2 - 1.5 eq.) in absolutem Dichlormethan oder einer Mischung (5:1 bis 1:1) von absolutem Dichlormethan und N,N-Dimethylformamid (ca. 0.1 bis 0.3 mol/l) werden bei 0°C zunächst eine äquimolare Menge HATU und dann das 3-[2-Amino-alkanoyl]-2,5-pyrrolidindion Hydrochlorid Derivat (1 eq., gegebenenfalls als Lösung in N,N-Dimethylformamid oder Dichlormethan/N,N-Dimethylformamid Gemischen) zugegeben. Anschließend wird bei 0°C eine Lösung von 2.5 - 3.5 eq. Diisopropylethylamin in einem 1:1 1 Gemisch von absolutem Dichlormethan und N,N-Dimethylformamid (0.2 - 1 mol/l) über einen Zeitraum von 1 h zugetropft. Nach Ende der Zugabe wird die Reaktionsmischung weitere 30 min bei 0°C und anschließend über Nacht bei Raumtemperatur gerührt, bevor im Vakuum eingeengt wird. Das Produkt kann durch Chromatographie an Silicagel (Eluenten: Gemische aus Cyclohexan/Essigsäureethylester oder Gemische aus Dichlormethan und Ethanol) oder durch RP-HPLC (Eluenten: variable Gradienten aus Wasser und Acetonitril), alternativ durch eine Kombination beider Verfahren, erhalten werden.

Alternativ kann die Umsetzung auch nach folgendem Verfahren erfolgen:
Zu einer Lösung des 3-[2-Amino-alkanoyl]-2,5-pyrrolidindion Hydrochlorid Derivates (1 eq.) in absolutem Dichlormethan oder einer Mischung (5:1 bis 1:1) von absolutem Dichlormethan und N,N-Dimethylformamid (ca. 0.1 bis 0.3 mol/l) werden das Carbonsäurederivat (1.1 - 1.5 eq.), Triethylamin (3 eq.), HOBt (3 eq.) und abschließend 1.2 eq. EDC gegeben. Die Reaktionsmischung wird bei Raumtemperatur gerührt (2 h bis über Nacht), bevor im Vakuum eingeengt wird. Der Rückstand wird in Essigsäureethylester oder Dichlormethan aufgenommen und die organische Phase wird mit Wasser, gesättigter Natriumhydrogencarbonatlösung und gesättigter Kochsalzlösung gewaschen, über Natriumsulfat getrocknet, filtriert und eingeengt. Das Produkt kann durch Chromatographie an Silicagel (Eluenten: Gemische aus Cyclohexan/Ethylacetat oder Gemische aus Dichlormethan und Ethanol) oder durch RP-HPLC (Eluenten: variable Gradienten aus Wasser und Acetonitril), alternativ durch eine Kombination beider Verfahren, gereinigt werden.

### Beispiel 16A

### ((S)-2-{(S)-2-Methyl-1-[1-((3R,4S)-4-methyl-2,5-dioxo-pyrrolidin-3-yl)-methanoyl]-propylcarbamoyl}-1-phenyl-ethyl)-carbamidsäure-tert-butylester

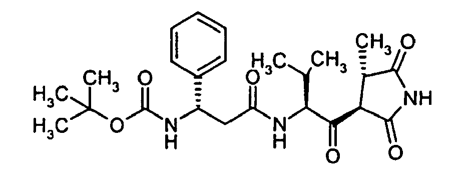

Die Darstellung erfolgt nach der Allgemeinen Vorschrift D.
¹H-NMR (400 MHz, d₆-DMSO): δ = 11.45 (s, 1H), 7.98 (d, 1 H), 7.31-7.24 (m, 5 H), 7.20 (br. s, 1 H), 4.88-4.82 (br. s, 1 H), 4.69 (br. s, 1 H), 3.98 (d, 1 H), 2.95-2.89 (m, 1 H), 2.77-2.69 (m, 1 H), 2.51-2.44 (m, 1 H), 2.35-2.29 (m, 1 H), 1.10 (d, 3 H), 0.85 (d, 3 H), 0.78 (d, 3 H).
MS (ESI+): m/z (%) = 460 (M+H⁺) (100).
HPLC (Methode 6): Rₜ = 3.90 min.

### Allgemeine Vorschrift E: Deblockierung von Boc-geschützten Derivaten

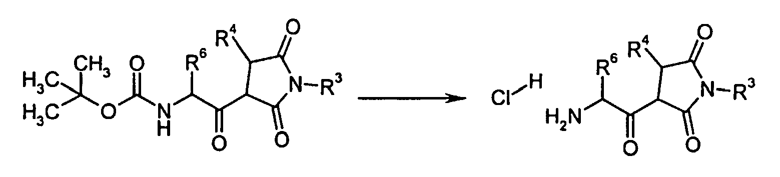

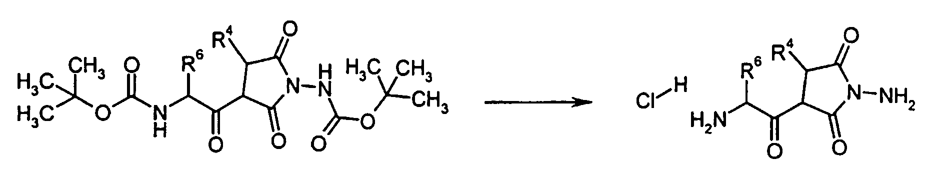

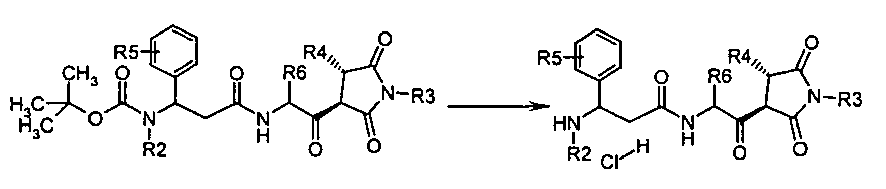

Das *tert*.-Butyloxycarbonyl (BOC) geschützte Aminderivat (gegebenenfalls als Lösung in Dioxan) wird bei 0°C oder Raumtemperatur mit 4N Salzsäure-Lösung in 1,4-Dioxan versetzt (ca. 0.1 mol/l) und 2 bis 24 h bei Raumtemperatur gerührt, bevor im Vakuum eingeengt wird. Der Rückstand kann ohne weitere Reinigung weiter umgesetzt werden oder wird gegebenenfalls mit Dichlormethan und Diethylether (ca. 1:2) behandelt. Die ausgefallenen Kristalle werden abgesaugt und am Hochvakuum getrocknet. Man erhält das Produkt als Hydrochlorid.

### Beispiel 17A

### (S)-3-Amino-{(S)-2-methyl-1-[1-((3R,4S)-4-methyl-2,5-dioxo-pyrrolidin-3-yl)-methanoyl]-propyl}-3-phenylpropionamid Hydrochlorid

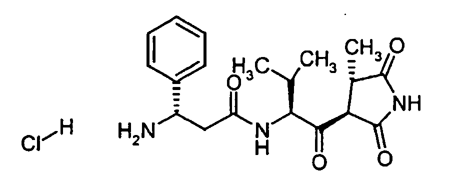

Die Darstellung erfolgt nach der Allgemeinen Vorschrift E.
¹H-NMR (200 MHz, d₆-DMSO): δ = 11.49 (br. s, 1 H), 8.5 (br. s, ca. 3 H), 7.54-7.32 (m, 5 H), 4.69-4.55 (m, 2 H), 3.89 (d, 1 H), 3.06-2.80 (m, 3 H), 2.39-2.25 (m, 1 H), 1.01 (d, 3 H), 0.81 (d, 3 H), 0.75 (d, 3 H).
MS (ESI+): m/z (%) = 360 (M-Cl)⁺ (100).
HPLC (Methode 4): Rₜ = 1.44 min.

### Beispiel 18A

### 3-Amino-3-(2,3-dihydro-1,4-benzodioxin-6-yl)propionsäuremethylester

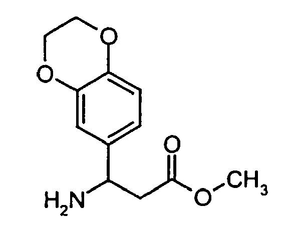

3-Amino-3-(2,3-dihydro-1,4-benzodioxin-6-yl)propionsäure [Synthese nach literaturbekannten Vorschriften (z. B. L. Lázár, T. Martinek, G. Bernáth, F. Fülöp, Synth. Comm. 1998, 28, 219-224)] wird in Methanol vorgelegt (ca. 0.5 bis 1.0 mol/l) und bei 0°C tropfenweise mit 1.2 eq Thionylchlorid versetzt. Nach beendeter Zugabe wird das Reaktionsgemisch über Nacht bei Raumtemperatur gerührt und anschließend eingeengt. Der Rückstand wir in wenig Methanol gelöst und mit Diethylether wird das Produkt ausgefällt. Der Feststoff wird abgesaugt, mehrfach mit Diethylether gewaschen und im Vakuum getrocknet.
¹H-NMR (300MHz, d₆-DMSO): δ = 8.51 (br. s, 3 H), 7.07 (d, 1 H), 6.95 (dd, 1 H), 6.88 (d, 1 H), 4.48 (dd, 1 H), 4.24 (s, 4 H), 3.57 (s, 3 H), 3.12 (dd, 1 H), 2.94 (dd, 1 H).
MS (ESI+): m/z = 238 (M+H⁺).

Analog zu Beispiel 1A können folgende Verbindungen (Beispiel 19A bis 26A) durch Umsetzung von (3*S*)-3-Methyldihydro-2,5-furandion mit den entsprechenden primären Aminen, Hydroxylamin- oder Hydrazinderivaten erhalten werden. Die Rohprodukte können durch RP-HPLC (Eluent: Wasser-Acetonitril, Gradient) gereinigt werden.

| **Beispiel** | **Struktur** | **MW** | **MS** | **HPLC** |
|---|---|---|---|---|
| 19A | 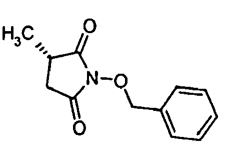 | 219.24 | | HPLC |
| | | | | (Methode 6): |
| | | | | Rₜ = 3.37 min |
| 20A | 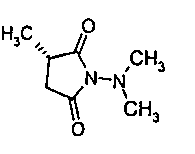 | 156.18 | MS (ESI+), | HPLC |
| | | | m/z: | (Methode 19): |
| | | | 157 (M+H)⁺ | Rₜ = 2.62 min |
| 21A | 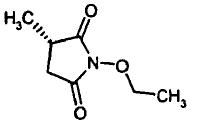 | 157.17 | MS (DCI), | HPLC |
| | | | m/z: 175 | (Methode 20): |
| | | | (M+NH₄)⁺ | Rₜ = 1.70 min |
| 22A | 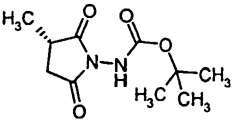 | 228.25 | MS (DCI), | HPLC |
| | | | m/z: 246 | (Methode 20): |
| | | | (M+NH₄)⁺ | Rₜ = 2.09 min |
| 23A | 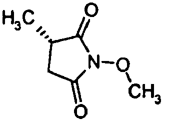 | 143.14 | MS (ESI+), | |
| | | | m/z: 144 | |
| | | | (M+H)⁺ | |
| 24A | 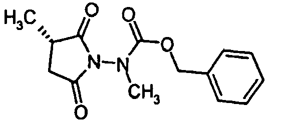 | 276.29 | MS (DCI), | HPLC |
| | | | m/z: 294 | (Methode 21): |
| | | | (M+NH₄)⁺ | Rₜ = 2.80 min |
| 25A | 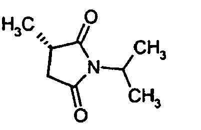 | 155.20 | MS (ESI+), | HPLC |
| | | | m/z: 156 | (Methode 19): |
| | | | (M+H)⁺ | Rₜ = 3.26 min |
| 26A | 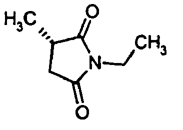 | 141.17 | MS (ESI+), | HPLC |
| | | | m/z: 142 | (Methode 19): |
| | | | (M+H)⁺ | Rₜ = 2.78 min |

### Allgemeine Vorschrift F: Umsetzung von N-tert.-Butoxycarbonyl-geschützten Aminosäuren mit 2,5-Pyrrolidindion Derivaten

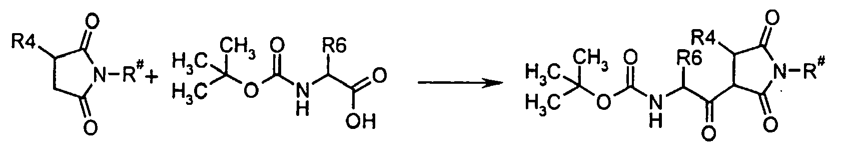

Die N-*tert.*-Butoxycarbonyl-geschützte Aminosäure (1 eq.) und N,N-Carbonyldiimidazol (1.1 eq.) werden in Tetrahydrofuran (ca. 0.1-1 mol/l) 2 h lang bei Raumtemperatur gerührt. Zu diesem Gemisch wird dann das 2,5-Pyrrolidindion (1 eq.) gegeben und die gesamte Mischung wird innerhalb von 30 min zu einer auf -65°C gekühlten 1 molaren Lösung von Lithium-hexamethyldisilazid (2 eq.) in THF getropft. Nach beendeter Zugabe wird weitere 15 min bei -65°C gerührt, bevor gesättigte wässrige Ammoniumchlorid-Lösung zugegeben wird. Nach Erwärmung auf Raumtemperatur wird das Reaktionsgemisch mit Diethylether verdünnt und die organische Phase mit gesättigter wässriger Natriumchlorid-Lösung gewaschen, über Magnesiumsulfat getrocknet, filtriert und anschließend eingeengt. Das Rohprodukt wird durch RP-HPLC (Eluent: Wasser-Acetonitril, Gradient) gereinigt.

Nach der Allgemeinen Vorschrift F können durch Umsetzung der entsprechenden N-*tert*.-Butoxycarbonyl-geschützten Aminosäuren (bzgl. der Darstellung von unnatürlichen alpha-Aminosäuren siehe z. B.: A. A. *Cordi et al., J. Med. Chem.* 2001, *44,* 787-805; K. Mai, G. Patil, *Tetrahedron Lett.* 1984, 25, 4583-4586; N. A. Hassan, E. Bayer, J. C. Jochims, *J. Chem. Soc., Perkin Trans.* 1 1998, 3747-3757; bzgl. der *tert.*-Butoxycarbonyl-Schützung siehe z. B.: T. W. Greene, P. G. M. Wuts, Protective Groups in Organic Synthesis, 3^{rd} Edt. 1999, J. Wiley & Sons, Inc.) mit 2,5-Pyrrolidindionen folgende Derivate (Beispiel 27A bis 52A) erhalten werden:

| **Beispiel** | **Struktur** | **MW** | **MS** | **HPLC** |
|---|---|---|---|---|
| 27A | 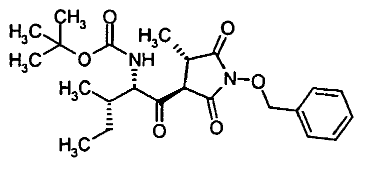 | 432.51 | MS (ESI-), | HPLC |
| | | | m/z: 431 | (Methode 10): |
| | | | (M-H)⁻ | Rₜ = 4.10 min |
| 28A | 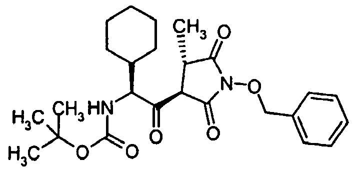 | 458.55 | MS (ESI-), | HPLC |
| | | | m/z: 457 | (Methode 12): |
| | | | (M-H)⁻ | Rₜ=4.12min |
| 29A | 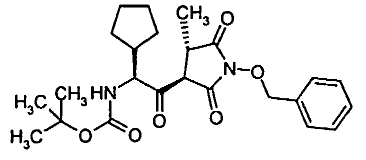 | 444.53 | MS (ESI-), | HPLC |
| | | | m/z: 443 | (Methode 18): |
| | | | (M-H)⁻ | Rₜ = 4.11 min |
| 30A | 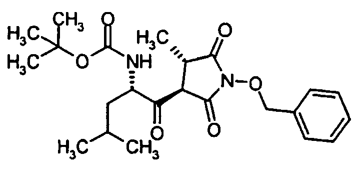 | 432.51 | MS (ESI-), | HPLC |
| | | | m/z: 431 | (Methode 6): |
| | | | (M-H)⁻ | Rₜ = 4.88 min |
| 31A | 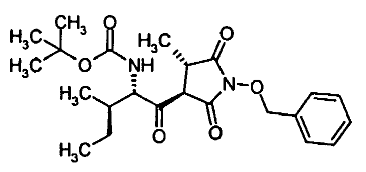 | 432.51 | MS (ESI-), | HPLC |
| | | | m/z: 431 | (Methode 13): |
| | | | (M-H)⁻ | Rₜ = 5.08 min |
| 32A | 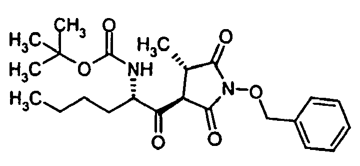 | 432.51 | MS (ESI-), | HPLC |
| | | | m/z: 431 | (Methode 11): |
| | | | (M-H)⁻ | Rₜ = 3.72 min |
| 33A | 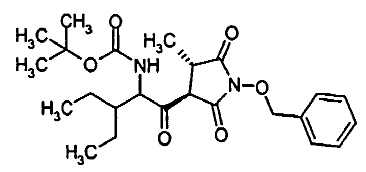 | 446.54 | MS (ESI-), | HPLC |
| | | | m/z: 445 | (Methode 17): |
| | | | (M-H)⁻ | Rₜ = 4.42 min |
| 34A | 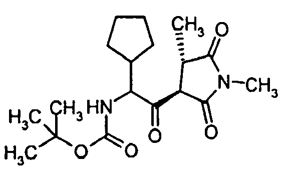 | 352.43 | MS (ESI-), | HPLC |
| | | | m/z: 351 | (Methode 14): |
| | | | (M-H)⁻ | Rₜ = 4.61 min |
| 35A | 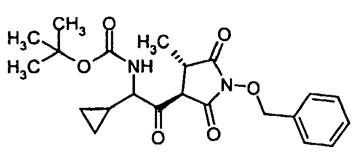 | 416.47 | MS (ESI-), | HPLC |
| | | | m/z: 415 | (Methode 16): |
| | | | (M-H)⁻ | Rₜ = 3.49 min |
| 36A | 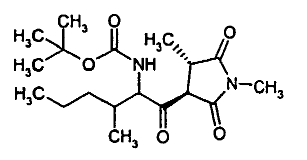 | 354.44 | MS (ESI-), | HPLC |
| | | | m/z: 353 | (Methode 16): |
| | | | (M-H)⁻ | Rₜ = 3.44 min |
| 37A | 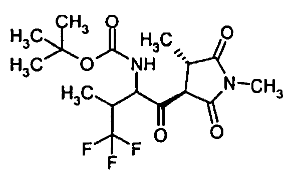 | 380.36 | MS (ESI-), | HPLC |
| | | | m/z: 379 | (Methode 17): |
| | | | (M-H)⁻ | Rₜ = 3.72 min |
| 38A | 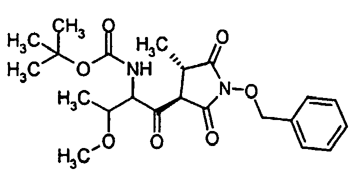 | 434.49 | MS (ESI-), | HPLC |
| | | | m/z: 433 | (Methode 17): |
| | | | (M-H)⁻ | Rₜ = 3.98 min |
| 39A | 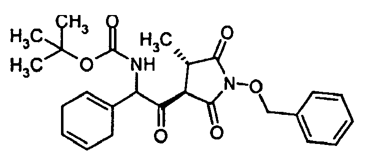 | 454.52 | MS (ESI-), | HPLC |
| | | | m/z: 453 | (Methode 22): |
| | | | (M-H)⁻ | Rₜ = 4.41 min |
| 40A | 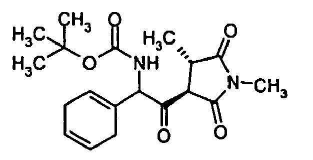 | 362.42 | MS (ESI-), | HPLC |
| | | | m/z: 361 | (Methode 18): |
| | | | (M-H)⁻ | Rₜ = 3.74 min |
| 41A | 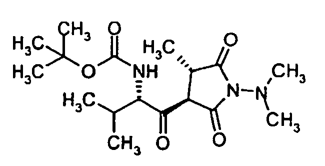 | 355.43 | MS (ESI+), | HPLC |
| | | | m/z: 378 | (Methode 19): |
| | | | (M+Na)⁺ | Rₜ = 4.12 min |
| 42A | 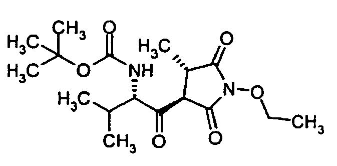 | 356.42 | MS (ESI-), | HPLC |
| | | | m/z: 355 | (Methode 20): |
| | | | (M-H)⁻ | Rₜ = 3.64 min |
| 43A | 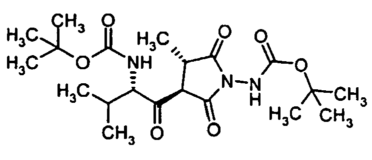 | 427.50 | MS (ESI-), | HPLC |
| | | | m/z: 426 | (Methode 20): |
| | | | (M-H)⁻ | Rₜ = 3.73 min |
| 44A | 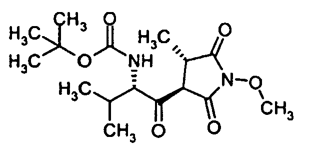 | 342.39 | MS (ESI-), | HPLC |
| | | | m/z: 341 | (Methode 19): |
| | | | (M-H)⁻ | Rₜ = 4.18min |
| 45A | 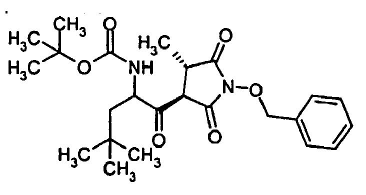 | 446.54 | MS (ESI-), | HPLC |
| | | | m/z: 445 | (Methode 20): |
| | | | (M-H)⁻ | Rₜ = 4.02 min |
| 46A | 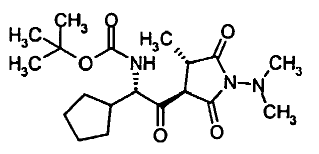 | 381.47 | MS (ESI-), | HPLC |
| | | | m/z: 380 | (Methode 21): |
| | | | (M-H)⁻ | Rₜ = 3.43 min |
| 47A | 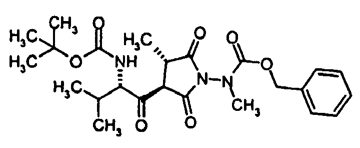 | 475.54 | MS (ESI-), | HPLC |
| | | | m/z: 474 | (Methode 20): |
| | | | (M-H)⁻ | Rₜ = 3.91 min |
| 48A | 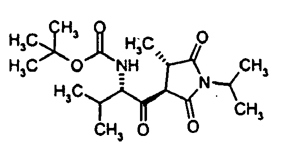 | 354.44 | MS (ESI-), | HPLC |
| | | | m/z: 353 | (Methode 18): |
| | | | (M-H)⁻ | Rₜ= 3.80 min |
| 49A | 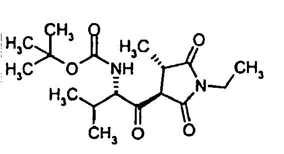 | 340.42 | MS (ESI-), | HPLC |
| | | | m/z: 339 | (Methode 20): |
| | | | (M-H)⁻ | Rₜ = 3.61 min |
| 50A | 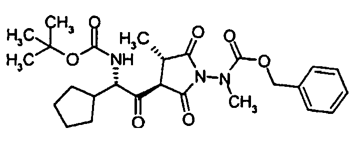 | 501.58 | MS (ESI-), | HPLC |
| | | | m/z: 500 | (Methode 21): |
| | | | (M-H)⁻ | Rₜ = 3.93 min |
| 51A | 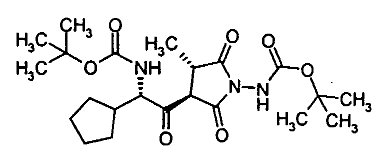 | 453.53 | MS (ESI-), | HPLC |
| | | | m/z: 452 | (Methode 21): |
| | | | (M-H)⁻ | Rₜ = 3.61 min |
| 52A | 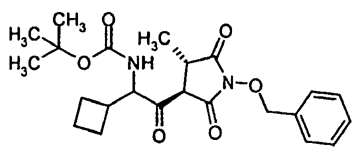 | 430.51 | MS (ESI+), | HPLC |
| | | | m/z: 453 | (Methode 20): |
| | | | (M+Na)⁺ | Rₜ = 4.32 min |

Nach der Allgemeinen Vorschrift A können folgende Verbindungen (Beispiel 53A bis 64A) erhalten werden:

| **Beispiel** | **Struktur** | **MW** | **MS** | **HPLC** |
|---|---|---|---|---|
| 53A | 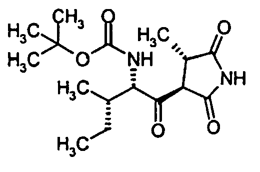 | 326.40 | MS (ESI+), | HPLC |
| | | | m/z: 349 | (Methode 12): |
| | | | (M+Na)⁺ | Rₜ = 2.97 min |
| 54A | 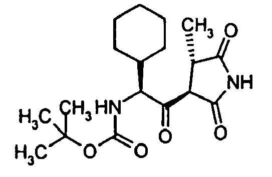 | 352.43 | MS (ESI-), | HPLC |
| | | | m/z: 351 | (Methode 12): |
| | | | (M-H)⁻ | Rₜ = 3.23 min |
| 55A | 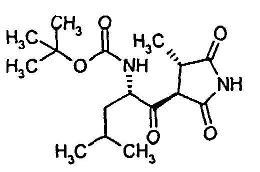 | 326.39 | MS (ESI-), | HPLC |
| | | | m/z: 325 | (Methode 5): |
| | | | (M-H)⁻ | Rₜ = 3.91 min |
| 56A | 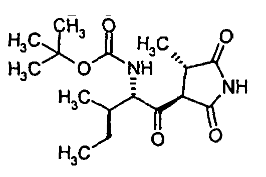 | 326.39 | MS (ESI-), | HPLC |
| | | | m/z: 325 | (Methode 12): |
| | | | (M-H)⁻ | Rₜ = 2.88 min |
| 57A | 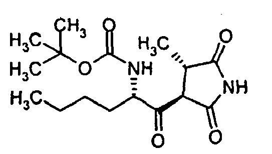 | 326.39 | MS (ESI-), | HPLC |
| | | | m/z: 325 | (Methode 13): |
| | | | (M-H)⁻ | Rₜ = 4.25 min |
| 58A | 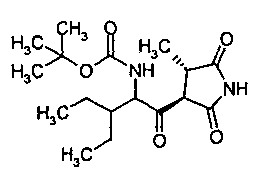 | 340.42 | MS (ESI-), | HPLC |
| | | | m/z: 339 | (Methode 17): |
| | | | (M-H)⁻ | Rₜ = 3.59 min |
| 59A | 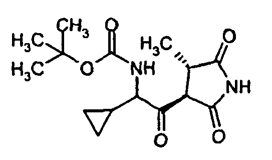 | 310.35 | MS (ESI-), | HPLC |
| | | | m/z: 309 | (Methode 16): |
| | | | (M-H)⁻ | Rₜ = 2.41 min |
| 60A | 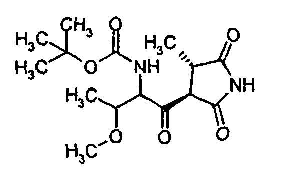 | 328.26 | MS (ESI+), | HPLC |
| | | | m/z: 351 | (Methode 20): |
| | | | (M+Na)⁺ | Rₜ = 3.18 min |
| 61A | 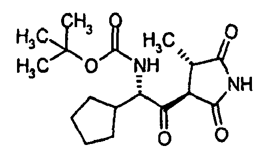 | 338.40 | MS (ESI-), | HPLC |
| | | | m/z: 337 | (Methode 17): |
| | | | (M-H)⁻ | Rₜ = 3.57 min |
| 62A | 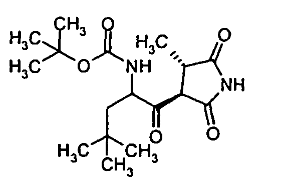 | 340.42 | MS (ESI+), | HPLC |
| | | | m/z: 341 | (Methode 20): |
| | | | (M+H)⁺ | Rₜ = 3.79 min |
| 63A | 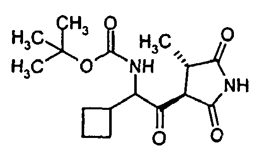 | 324.38 | MS (ESI-), | HPLC |
| | | | m/z: 323 | (Methode 19): |
| | | | (M-H)⁻ | Rₜ = 4.02 min |
| 64A | 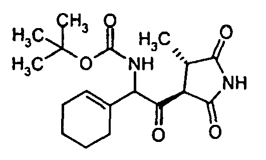 | 350.41 | MS (ESI-), | HPLC |
| | | | m/z: 349 | (Methode 18): |
| | | | (M-H)⁻ | Rₜ = 3.66 min |

Die Verbindung 64A ist bei der Umsetzung von Verbindung 39A entstanden.

### Allgemeine Vorschrift G: Deblockierung von Benzyloxycarbonyl-geschützten Hydrazin-Derivaten

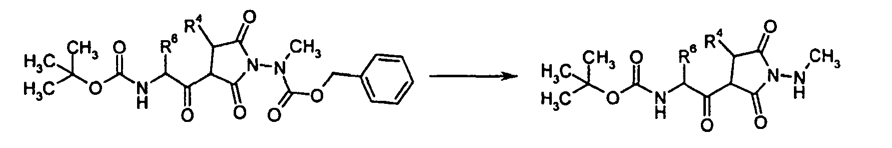

Das Benzyloxycarbonyl-geschützte Hydrazin-Derivat (1 eq.) wird in Methanol oder Ethanol gelöst (ca. 0.05 mol/l), mit einer katalytischen Menge Palladium-Kohle (10%) versetzt und 3 - 4 h unter Wasserstoffatmosphäre (Normaldruck) gerührt. Dann wird das Reaktionsgemisch filtriert und eingeengt. Das Rohprodukt kann ohne weitere Reinigung umgesetzt werden.

Nach der Allgemeinen Vorschrift G können folgende Verbindungen (Beispiel 65A und 66A) erhalten werden:

| **Beispiel** | **Struktur** | **MW** | **MS** | **HPLC** |
|---|---|---|---|---|
| 65A | 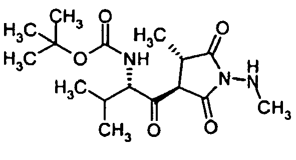 | 341.41 | MS (ESI-), | HPLC |
| | | | m/z: 340 | (Methode 19): |
| | | | (M-H)⁻ | Rₜ = 4.00 min |
| 66A | 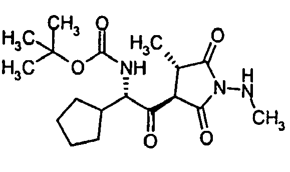 | 367.44 | MS (ESI-), | HPLC |
| | | | m/z: 366 | (Methode 20): |
| | | | (M-H)⁻ | Rₜ = 3.47 min |

Nach der Allgemeinen Vorschrift E können folgende Verbindungen (Beispiel 67A bis 93A) erhalten werden:

| **Beispiel** | **Struktur** | **MW** |
|---|---|---|
| 67A | 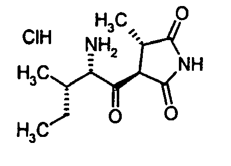 | 226.28 |
| 68A | 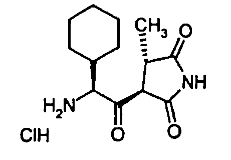 | 252.32 |
| 69A | 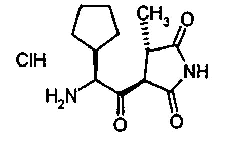 | 238.29 |
| 70A | 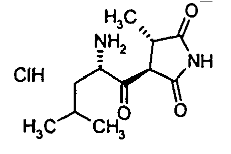 | 226.28 |
| 71A | 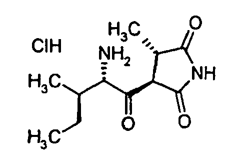 | 226.28 |
| 72A | 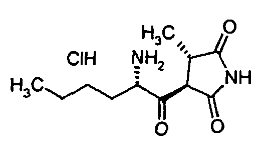 | 226.28 |
| 73A | 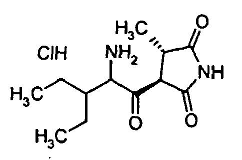 | 240.30 |
| 74A | 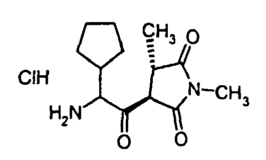 | 252.32 |
| 75A | 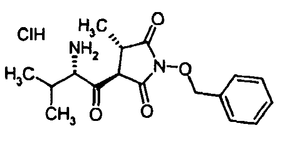 | 318.38 |
| 76A | 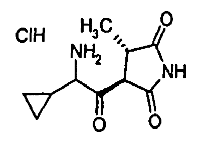 | 210.23 |
| 77A | 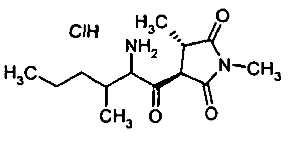 | 254.33 |
| 78A | 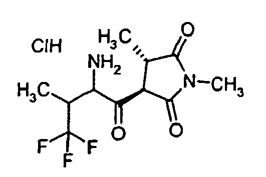 | 280.25 |
| 79A | 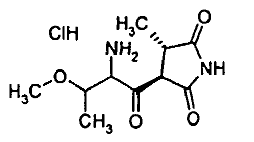 | 228.25 |
| 80A | 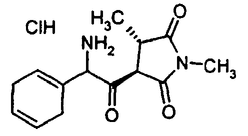 | 262.31 |
| 81A | 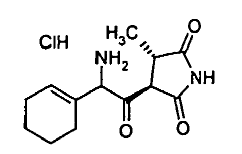 | 250.30 |
| 82A | 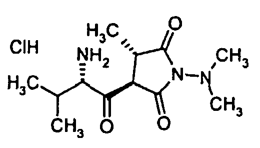 | 255.32 |
| 83A | 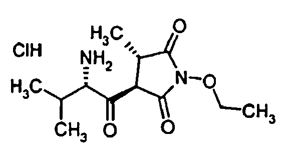 | 256.30 |
| 84A | 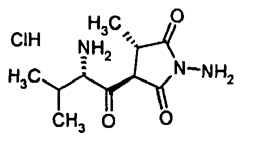 | 227.27 |
| 85A | 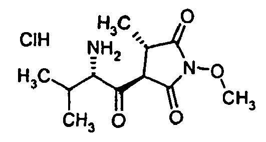 | 242.28 |
| 86A | 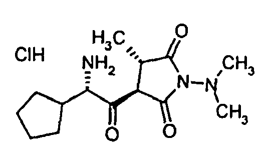 | 281.36 |
| 87A | 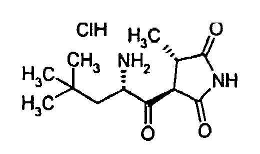 | 240.30 |
| 88A | 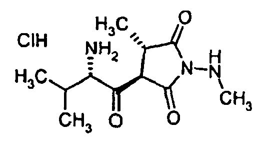 | 241.29 |
| 89A | 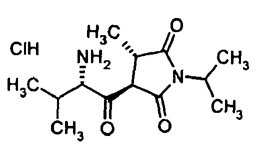 | 254.33 |
| 90A | 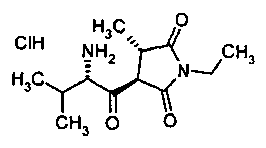 | 240.30 |
| 91A | 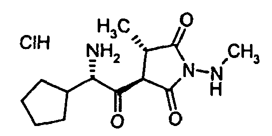 | 267.33 |
| 92A | 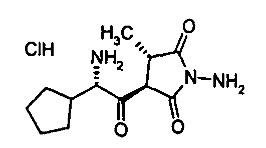 | 253.30 |
| 93A | 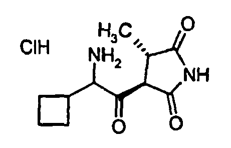 | 224.26 |

### Beispiel 94A

### (S)-3-Amino-3-phenylpropionsäuremethylester

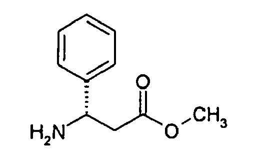

2.3 g (11.65 mmol) (*S*)-3-Amino-3-phenylpropionsäure werden in 100 ml Methanol vorgelegt und mit einer katalytischen Menge konzentrierter Schwefelsäure (0.02 eq.) versetzt. Das Reaktionsgemisch wird 24 h lang zum Rückfluss erhitzt und anschließend eingeengt. Das Rohprodukt kann ohne weitere Reinigung in der nächsten Stufe eingesetzt werden.
Ausbeute: 2.7 g (65 % d. Th.).
¹H-NMR (300 MHz, d₆-DMSO): δ = 8.50 (s, 2 H), 7.52-7.37 (m, 5 H), 4.61 (t, 1 H), 3.58 (s, 3 H), 3.13 (dd, 1 H), 2.98 (dd, 1 H).
MS (ES+): m/z (%) = 180 (M+H)⁺ (100).

### Allgemeine Vorschrift H: Synthese der beta-Aminosäuremethylester

Die beta-Aminosäure (1 eq) [Synthese nach literaturbekannten Vorschriften, z. B. S. Rault, P. Dallemagne, M. Robba, *Bull. Soc. Chim. Fr.,* **1987,** 1079-1083; L. Lázár, T. Martinek, G. Bernáth, F. Fülöp, *Synth. Comm.,* **1998,** *28,* 219-224] wird in Methanol (Konzentration 0.3-1 mol/l) bei Temperaturen zwischen -40°C und 0°C suspendiert. Thionylchlorid (2 eq) wird zugetropft, das Reaktionsgemisch wird auf Raumtemperatur erwärmt und über Nacht gerührt. Nach Eindampfen zur Trockene wird der Rückstand in Wasser aufgenommen und zweimal mit Essigsäureethylester gewaschen. Die organische Phase wird verworfen, die wässrige Phase wird mit gesättigter Natriumhydrogencarbonat-Lösung neutralisiert und erneut dreimal mit Essigsäureethylester extrahiert. Die organischen Phasen der letzten Extraktion werden über Natriumsulfat oder Magnesiumsulfat getrocknet, dekantiert und zur Trockene eingedampft. Alternativ kann die Aufarbeitung auch durch Lösen des Rohprodukts in wenig Methanol und Kristallisation des Produkts durch Zugabe von Diethylether erfolgen.

Gemäß der Allgemeinen Arbeitsvorschrift H können folgende Verbindungen (Beispiel 95A bis 108A) hergestellt werden:

| **Beispiel** | **Struktur** | **MW** | **MS** | **HPLC / NMR** |
|---|---|---|---|---|
| 95A | 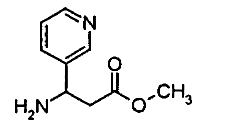 | 180.2 | MS (ESI+), | |
| | | | m/z: 180 | |
| | | | (M+H)⁺ | |
| 96A | 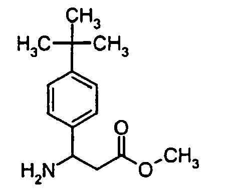 | 235.3 | MS (DCI), | |
| | | | m/z:236 | |
| | | | (M+H)⁺ | |
| 97A | 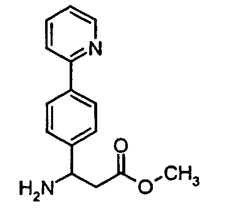 | 256.3 | MS (ESI+), | |
| | | | m/z: 257 | HPLC (Methode 5): |
| | | | (M+H)⁺ | Rₜ = 3.68 min |
| 98A | 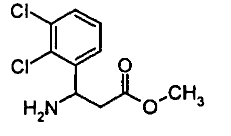 | 248.1 | MS (ESI+), | |
| | | | m/z: 248 | |
| | | | (M+H)⁺ | |
| 99A | 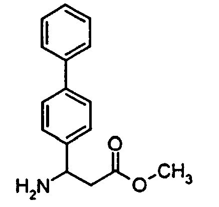 | 255.3 | MS (DCI), | |
| | | | m/z: 256 | |
| | | | (M+H)⁺ | |
| 100A | 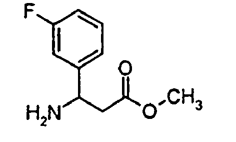 | 197.2 | MS (DCI), | |
| | | | m/z:198 | |
| | | | (M+H)⁺ | |
| 101A | 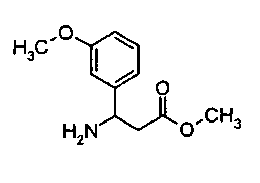 | 209.2 | | ¹H-NMR (300 MHz, CDCl₃): δ = 2.61-2.68 (m, 2 H); 3.69 (s, 3H); 3.80 (s, 3H); 4.40 (dd, 1 H); 6.80 (dd, 1 H); 6.90-6.96 (m, 2H); 7.20-7.29 (m, 1H) |
| 102A | 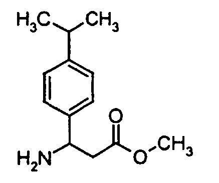 | 221.3 | MS (DCI), | |
| | | | m/z: 222 | |
| | | | (M+H)⁺ | |
| 103A | 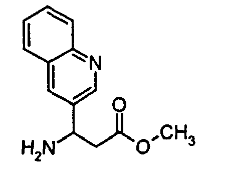 | 230.3 | MS (ESI+), | |
| | | | m/z: 231 | HPLC (Methode 20): |
| | | | (M+H)⁺ | Rₜ = 1.70 min |
| 104A | 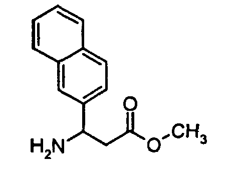 | 229.3 | MS (ESI+), | |
| | | | m/z: 230 | HPLC (Methode 19): |
| | | | (M+H)⁺ | Rₜ = 1.75 min |
| 105A | 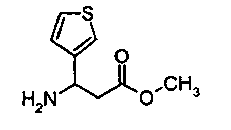 | 185.3 | MS (ESI+), | |
| | | | m/z: 186 | |
| | | | (M+H)⁺ | |
| 106A | 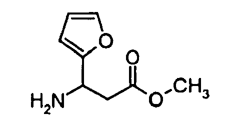 | 169.2 | MS (ESI+), | |
| | | | m/z: 170 | |
| | | | (M+H)⁺ | |
| 107A | 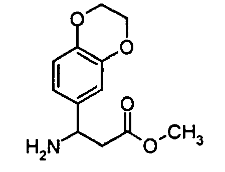 | 237.3 | MS (ESI+), | |
| | | | m/z: 238 | |
| | | | (M+H)⁺ | |
| 108A | 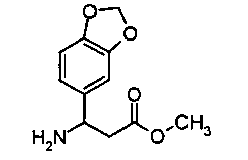 | 223.2 | MS (ESI+), | |
| | | | m/z: 224 | |
| | | | (M+H)⁺ | |

Nach der Allgemeinen Vorschrift B können folgende Verbindungen (Beispiel 109A bis 117A) erhalten werden:

| **Beispiel** | **Struktur** | **MW** | **MS** | **HPLC** |
|---|---|---|---|---|
| 109A | 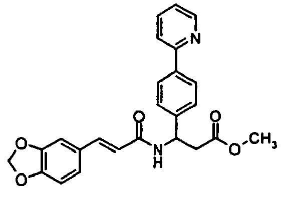 | 430.46 | MS (ESI+), | HPLC |
| | | | m/z (%): 431 | (Methode 12): |
| | | | (M+H)⁺ | Rₜ = 2.51 min |
| 110A | 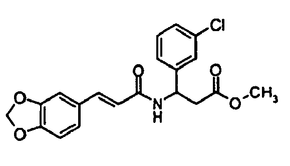 | 387.82 | MS (ESI+), | HPLC |
| | | | m/z (%): 388 | (Methode 12): |
| | | | (M+H)⁺ | Rₜ = 3.10 min |
| 111A | 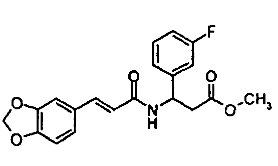 | 371.36 | MS (ESI+), | HPLC |
| | | | m/z (%): 372 | (Methode 17): |
| | | | (M+H)⁺ | Rₜ = 3.48 min |
| 112A | 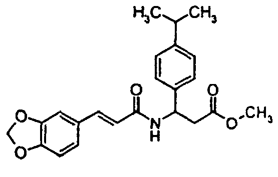 | 395.45 | MS (ESI+), | HPLC |
| | | | m/z (%): 396 | (Methode 17): |
| | | | (M+H)⁺ | Rₜ = 3.93min |
| 113A | 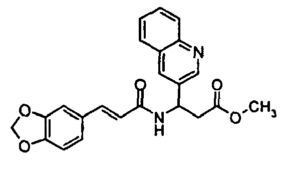 | 404.42 | MS (ESI+), | HPLC |
| | | | m/z (%): 405 | (Methode 19): |
| | | | (M+H)⁺ | Rₜ = 2.40 min |
| 114A | 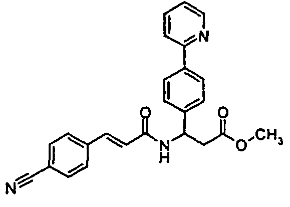 | 411.46 | MS (ESI+), | HPLC |
| | | | m/z (%): 412 | (Methode 20): |
| | | | (M+H)⁺ | Rₜ = 3.20 min |
| 115A | 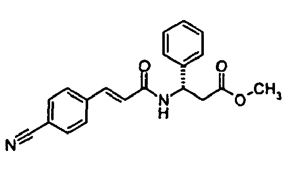 | 334.37 | MS (ESI+), | HPLC |
| | | | m/z (%): 335 | (Methode 18): |
| | | | (M+H)⁺ | Rₜ = 3.36 min |
| 116A | 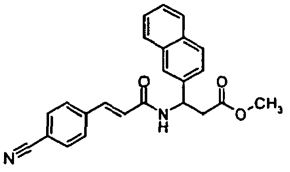 | 384.43 | MS (ESI+), | HPLC |
| | | | m/z (%): 385 | (Methode 21): |
| | | | (M+H)⁺ | Rₜ = 3.38 min |
| 117A | 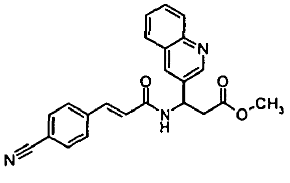 | 385.42 | MS (ESI+), | HPLC |
| | | | m/z (%): 386 | (Methode 21): |
| | | | (M+H)⁺ | Rₜ = 2.76 min |

### Allgemeine Vorschrift I: Umsetzung von 3-Amino-propionsäure-alkylestern mit Carbonsäurechloriden

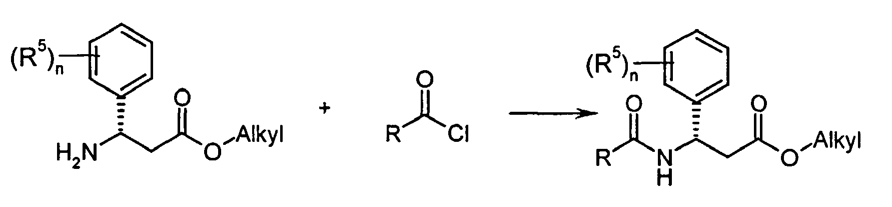

Der 3-Aminopropionsäurealkylester wird in Dichlormethan (ca. 0.1 - 0.4 mol/l) bei RT vorgelegt und mit 2 bis 3 eq. Diisopropylethylamin und 1.2 eq. des Carbonsäurechlorids versetzt. Es wird 2 bis 3 h bei Raumtemperatur gerührt. Dann wird Wasser zu dem Reaktionsgemisch gegeben, die organische Phase wird abgetrennt, über Natriumsulfat getrocknet, filtriert und eingeengt. Der Rückstand kann mit Dichlormethan und Diethylether umkristallisiert oder mittels Chromatographie an Kieselgel (Eluent: Gemische aus Dichlormethan und Essigsäureethylester) gereinigt werden.

Nach der Allgemeinen Vorschrift I können folgende Verbindungen (Beispiel 118A bis 122A) erhalten werden:

| **Beispiel** | **Struktur** | **MW** | **MS** | **HPLC** |
|---|---|---|---|---|
| 118A | 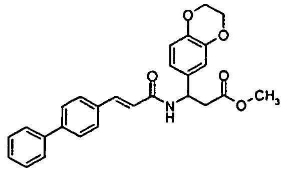 | 443.50 | MS (ES+), | |
| | | | m/z (%): 444 | |
| | | | (M+H)⁺ | |
| 119A | 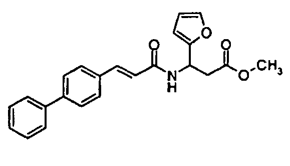 | 375.43 | MS (ES+), | HPLC |
| | | | m/z (%): 376 | (Methode 9): |
| | | | (M+H)⁺ | Rₜ = 4.59 min |
| 120A | 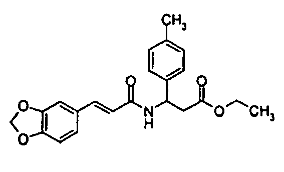 | 381.43 | MS (ES+), | HPLC |
| | | | m/z (%): 382 | (Methode 9): |
| | | | (M+H)⁺ | Rₜ = 3.65 min |
| 121A | 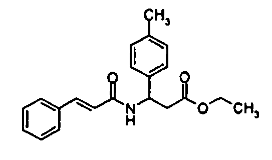 | 337.42 | MS (ES+), | HPLC |
| | | | m/z (%): 338 | (Methode 9): |
| | | | (M+H)⁺ | Rₜ = 4.52 min |
| 122A | 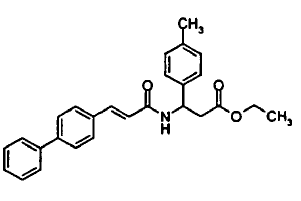 | 413.52 | MS (ES+), | HPLC |
| | | | m/z(%):414 | (Methode 9): |
| | | | (M+H)⁺ | Rₜ=4.91 min |

Nach der Allgemeinen Vorschrift C können folgende Verbindungen (Beispiel 123A bis 136A) erhalten werden:

| **Beispiel** | **Struktur** | **MW** | **MS** | **HPLC** |
|---|---|---|---|---|
| 123A | 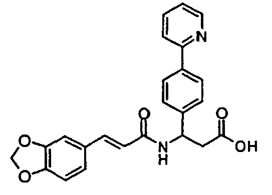 | 416.43 | MS (ESI+), | HPLC |
| | | | m/z (%): 417 | (Methode 17): |
| | | | (M+H)⁺ | Rₜ = 2.90 min |
| 124A | 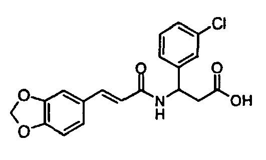 | 373.79 | MS (ESI+), | HPLC |
| | | | m/z (%): 374 | (Methode 16): |
| | | | (M+H)⁺ | Rₜ = 2.75 min |
| 125A | 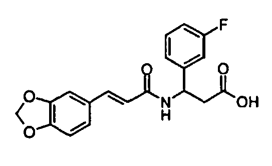 | 357.34 | MS (ESI+), | HPLC |
| | | | m/z (%): 358 | (Methode 17): |
| | | | (M+H)⁺ | Rₜ = 3.20 min |
| 126A | 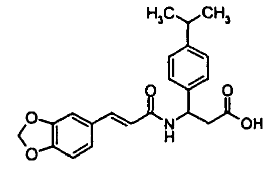 | 381.43 | MS (ESI+), | HPLC |
| | | | m/z (%): 382 | (Methode 17): |
| | | | (M+H)⁺ | Rₜ = 3.59 min |
| 127A | 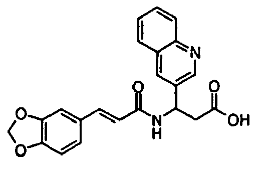 | 390.39 | MS (ESI+), | HPLC |
| | | | m/z (%): 391 | (Methode 20): |
| | | | (M+H)⁺ | Rₜ = 2.27 min |
| 128A | 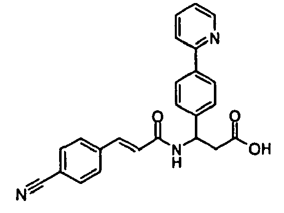 | 397.43 | MS (ESI+), | HPLC |
| | | | m/z (%): 398 | (Methode 19): |
| | | | (M+H)⁺ | Rₜ = 2.11 min |
| 129A | 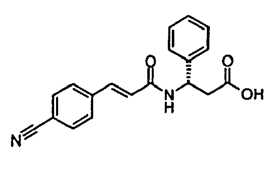 | 320.35 | MS (ESI+), | HPLC |
| | | | m/z (%): 321 | (Methode 20): |
| | | | (M+H)⁺ | Rₜ = 2.65 min |
| 130A | 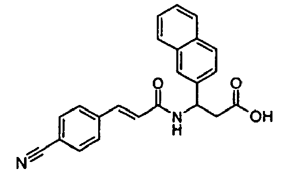 | 370.41 | MS (ESI+), | HPLC |
| | | | m/z (%): 371 | (Methode 19): |
| | | | (M+H)⁺ | Rₜ = 2.65 min |
| 131A | 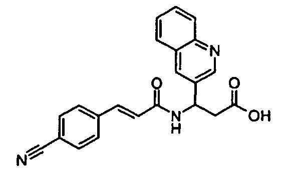 | 371.39 | MS (ESI+), | HPLC |
| | | | m/z (%): 372 | (Methode 20): |
| | | | (M+H)⁺ | Rₜ = 2.35 min |
| 132A | 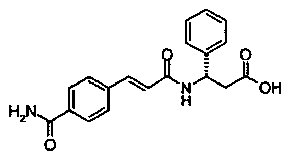 | 338.37 | MS (ESI+), | HPLC |
| | | | m/z (%): 339 | (Methode 18): |
| | | | (M+H)⁺ | Rₜ = 2.72 min |
| 133A | 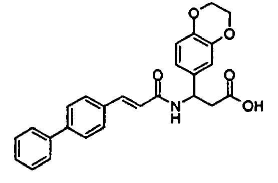 | 429.48 | MS (ES+), | HPLC |
| | | | m/z (%): 430 | (Methode 9): |
| | | | (M+H)⁺ | Rₜ = 4.31 min |
| 134A | 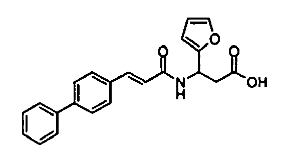 | 361.40 | MS (ES+), | |
| | | | m/z (%): 362 | |
| | | | (M+H)⁺ | |
| 135A | 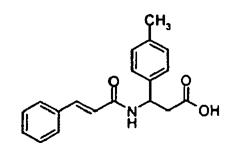 | 309.37 | MS (ES+), | HPLC |
| | | | m/z (%): 310 | (Methode 9): |
| | | | (M+H)⁺ | Rₜ = 4.04 min |
| 136A | 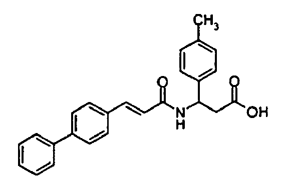 | 385.47 | MS (ES+), | |
| | | | m/z (%): 386 | |
| | | | (M+H)⁺ | |

### Die Verbindung 132A ist als Nebenprodukt bei der Darstellung von Verbindung 129A entstanden.

### Die so erhaltenen Propionsäurederivate können nach der Allgemeinen Vorschrift D (Acylierung von 3-[2-Amino-alkanoyl]-2,5-pyrrolidindion Hydrochlorid Derivaten mit Carbonsäurederivaten) umgesetzt werden.

### Allgemeine Vorschrift J: Darstellung von N-tert-Butoxycarbonyl-geschützten beta-Aminosäuren

Die beta-Aminosäure (1 eq.) [Synthese nach literaturbekannten Vorschriften, z. B. S. Rault, P. Dallemagne, M. Robba, *Bull. Soc. Chim. Fr.,* **1987,** 1079-1083; L. Lázár, T. Martinek, G. Bernáth, F. Fülöp, *Synth. Comm.,* **1998,** *28,* 219-224] wird in Wasser vorgelegt (Konzentration ca. 0.3 - 1 mol/l) und mit Triethylamin (1.5 - 3 eq.) versetzt. Dann wird eine Lösung von 2-(tert-Butoxycarbonyloximino)-phenylacetonitril (1.1 eq.) in Dioxan (0.3 - 1 mol/l) zugegeben. Das Reaktionsgemisch wird 3 h bei Raumtemperatur gerührt, mit Wasser verdünnt und mit Diethylether gewaschen. Die wässrige Phase wird mit 5 %iger Zitronensäure angesäuert (ca. pH 2) und dreimal mit Essigsäureethylester extrahiert. Die vereinigten organischen Phasen werden mit gesättigter Kochsalzlösung gewaschen, über Natriumsulfat getrocknet, filtriert und eingeengt. Das Rohprodukt kann gegebenenfalls aus Essigsäureethylester/n-Hexan umkristallisiert werden.

Nach der Allgemeinen Vorschrift J können folgende Verbindungen (Beispiel 137A bis 150A) erhalten werden:

| **Beispiel** | **Struktur** | **MW** | **MS** | **HPLC** |
|---|---|---|---|---|
| 137A | 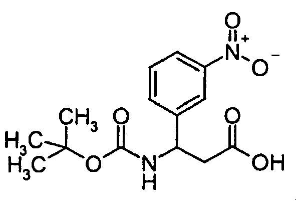 | 310.3 | MS (ESI+), | HPLC |
| | | | m/z: 311 | (Methode 8): |
| | | | (M+H)⁺ | Rₜ = 3.87 min |
| 138A | 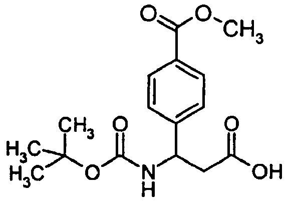 | 323.34 | MS (ESI+), | HPLC |
| | | | m/z: 324 | (Methode 8): |
| | | | (M+H)⁺ | Rₜ = 2.39 min |
| 139A | 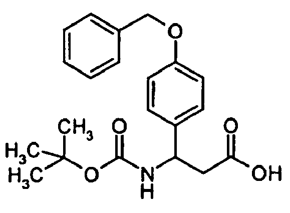 | 371.44 | MS (ESI+), | HPLC |
| | | | m/z: 372 | (Methode 9): |
| | | | (M+H)⁺ | Rₜ = 4.47 min |
| 140A | 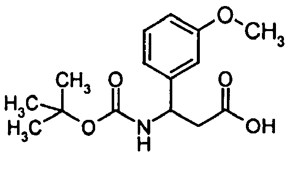 | 295.34 | MS (ESI+), | |
| | | | m/z: 296 | |
| | | | (M+H)⁺ | |
| 141A | 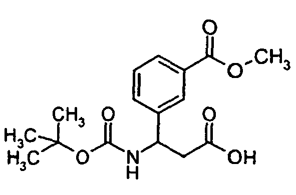 | 323.35 | | HPLC |
| | | | | (Methode 9): |
| | | | | Rₜ=3.96min |
| 142A | 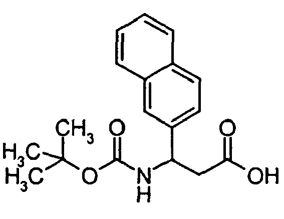 | 315.37 | MS (ESI-), | HPLC |
| | | | m/z: 314 | (Methode 21): |
| | | | (M-H)⁻ | Rₜ = 3.21 min |
| 143A | 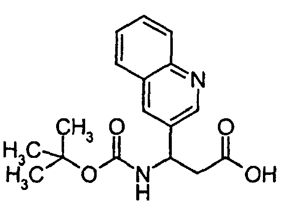 | 316.36 | MS (ESI+), | HPLC |
| | | | m/z: 317 | (Methode 19): |
| | | | (M+H)⁺ | Rₜ = 3.47 min |
| 144A | 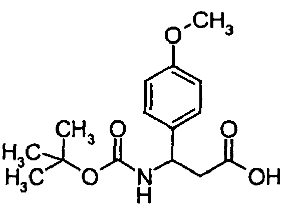 | 295.33 | MS (ESI+), | HPLC |
| | | | m/z: 296 | (Methode 20): |
| | | | (M+H)⁺ | Rₜ = 3.00 min |
| 145A | 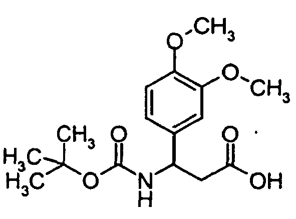 | 325.36 | | HPLC |
| | | | | (Methode 9): |
| | | | | Rₜ = 3.76 min |
| 146A | 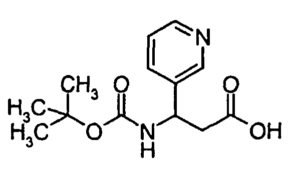 | 266.30 | MS (DCI), | HPLC |
| | | | m/z: 167 | (Methode 9): |
| | | | (M-100+H)⁺ | Rₜ = 1.92 min |
| 147A | 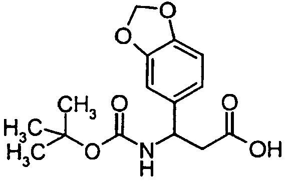 | 309.32 | MS (ESI-), | HPLC |
| | | | m/z: 308 | (Methode 13): |
| | | | (M-H)⁻ | Rₜ = 3.69 min |
| 148A | 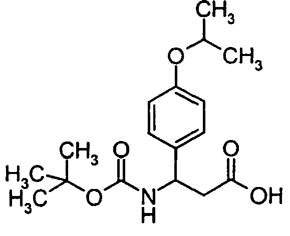 | 323.39 | MS (ESI-), | HPLC |
| | | | m/z: 322 | (Methode 14): |
| | | | (M-H)⁻ | Rₜ = 4.35 min |
| 149A | 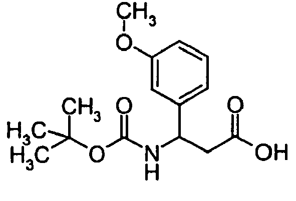 | 295.33 | MS (ESI+), | |
| | | | m/z: 296 | |
| | | | (M+H)⁺ | |
| 150A | 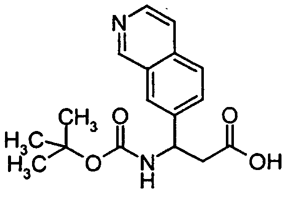 | 316.36 | MS (ESI+), | HPLC |
| | | | m/z: 317 | (Methode 21): |
| | | | (M+H)⁺ | Rₜ = 2.14 min |

### Beispiel 151A

### (3S)-3-[(tert-Butoxycarbonyl)amino]-3-phenylpropionsäure

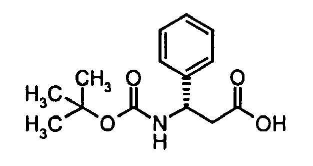

2.82 g (17 mmol) (*S*)-3-Amino-3-phenylpropionsäure werden in 60 ml Dioxan aufgeschlemmt und bei 0°C mit 4.1 g (18.8 mmol) Di-tert-butyl-dicarbonat (Boc-Anhydrid) und 43 ml einer 1N Natriumhydroxidlösung in Wasser versetzt. Das Reaktionsgemisch wird noch 30 min bei 0°C und dann 3 h bei Raumtemperatur gerührt. Anschließend wird das Reaktionsgemisch eingeengt und der Rückstand wird in Methylenchlorid aufgenommen. Die organische Phase wird mit 1N Salzsäure und gesättigter Kochsalzlösung gewaschen, über Magnesiumsulfat getrocknet und eingeengt. Das Rohprodukt (3.12 g) kann ohne weitere Reinigung weiter umgesetzt werden.
MS (ES-): m/z (%) = 264 (M-H)⁻ (100).
HPLC (Methode 14): Rₜ = 3.89 min.

### Beispiel 152A

### (3S)-3-[(tert-Butoxycarbonyl)-methyl-amino]-3-phenylpropionsäure

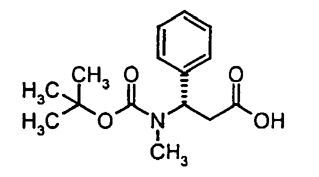

500 mg (1.88 mmol) (3S)-3-[(tert-Butoxycarbonyl)amino]-3-phenylpropionsäure werden in 6 ml Tetrahydrofuran vorgelegt und bei 0°C mit 2.14 g (15.1 mmol) Iodmethan versetzt. Dann werden portionsweise 226 mg (5.65 mmol) einer 60 %igen Natriumhydrid Dispersion in Mineralöl hinzugegeben. Das Reaktionsgemisch wird auf Raumtemperatur erwärmt und über Nacht gerührt. Anschließend werden 5 ml Wasser vorsichtig zugegeben und das Reaktionsgemisch wird eingeengt. Der Rückstand wird in Essigsäureethylester aufgenommen. Die organische Phase wird mit Wasser gewaschen, über Magnesiumsulfat getrocknet, filtriert und eingeengt. Das Rohprodukt (110 mg) kann ohne weitere Reinigung weiter umgesetzt werden.
MS (ESI-): m/z = 278 (M-H)⁻.
HPLC (Methode 14): Rₜ = 4.27 min.

Nach der Allgemeinen Vorschrift D können folgende Verbindungen (Beispiel 153A bis 170A) erhalten werden:

| **Beispiel** | **Struktur** | **MW** | **MS** | **HPLC** |
|---|---|---|---|---|
| 153A | 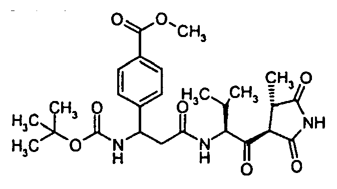 | 517.58 | MS (ESI+), | HPLC |
| | | | m/z: 518 | (Methode 8): |
| | | | (M+H)⁺ | Rₜ = 2.60 min |
| 154A | 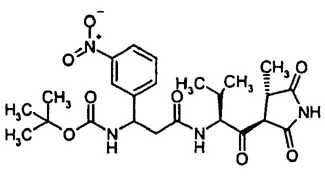 | 504.54 | MS (ESI-), | HPLC |
| | | | m/z: 503 | (Methode 6): |
| | | | (M-H)⁻ | Rₜ = 3.99 min |
| 155A | 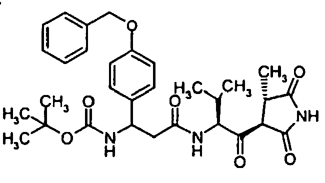 | 565.67 | MS (ESI+), | HPLC |
| | | | m/z: 566 | (Methode 16): |
| | | | (M+H)⁺ | Rₜ = 3.45 min |
| 156A | 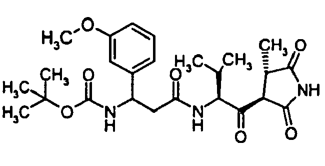 | 489.57 | MS (ESI+), | HPLC |
| | | | m/z: 490 | (Methode 16): |
| | | | (M+H)⁺ | Rₜ = 2.90 min |
| 157A | 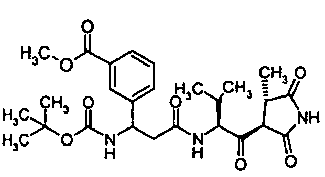 | 517.58 | MS (ESI+), | HPLC |
| | | | m/z: 518 | (Methode 16): |
| | | | (M+H)⁺ | Rₜ = 2.89 min |
| 158A | 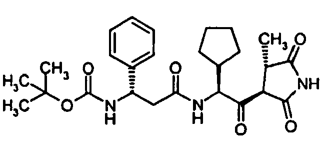 | 485.58 | MS (ESI-), | HPLC |
| | | | m/z: 484 | (Methode 20): |
| | | | (M-H)⁻ | Rₜ = 3.72 min |
| 159A | 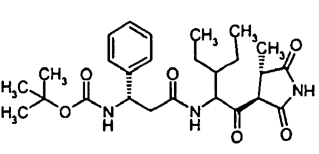 | 487.59 | MS (ESI+), | HPLC |
| | | | m/z: 488 | (Methode 17): |
| | | | (M+H)⁺ | Rₜ = 3.73 min |
| 160A | 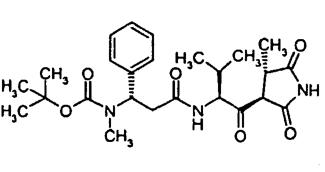 | 473.57 | MS (ESI-), | HPLC |
| | | | m/z: 472 | (Methode 12): |
| | | | (M-H)⁻ | Rₜ = 3.20 min |
| 161A | 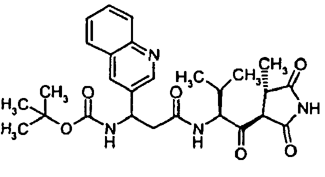 | 510.59 | MS (ESI-), | HPLC |
| | | | m/z: 509 | (Methode 19): |
| | | | (M-H)⁻ | Rₜ = 3.99 min |
| 162A | 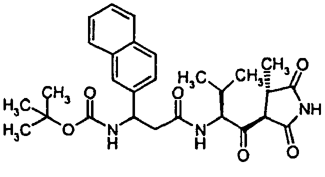 | 509.61 | MS (ESI-), | HPLC |
| | | | m/z: 508 | (Methode 20): |
| | | | (M-H)⁻ | Rₜ = 3.77 min |
| 163A | 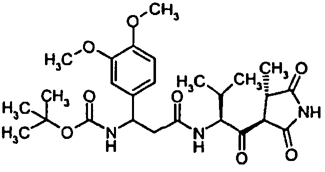 | 519.60 | MS (ESI-), | HPLC |
| | | | m/z: 518 | (Methode 18): |
| | | | (M-H)⁻ | Rₜ=3.36min |
| 164A | 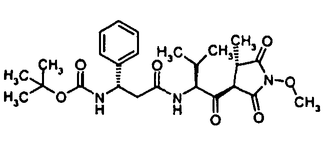 | 489.57 | MS (ESI-), | HPLC |
| | | | m/z: 488 | (Methode 19): |
| | | | (M-H)⁻ | Rₜ=4.19min |
| 165A | 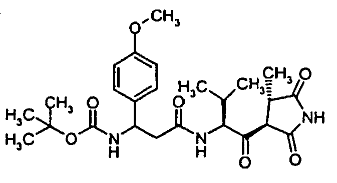 | 489.57 | MS (ESI-), | HPLC |
| | | | m/z: 488 | (Methode 20): |
| | | | (M-H)⁻ | Rₜ = 3.36 min |
| 166A | 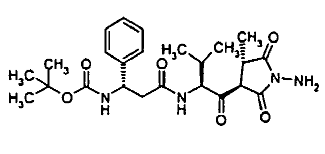 | 474.56 | MS (ESI-), | HPLC |
| | | | m/z: 473 | (Methode 19): |
| | | | (M-H)⁻ | Rₜ = 2.55 min |
| 167A | 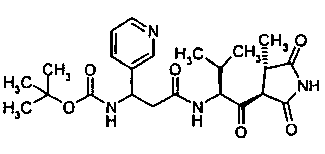 | 460.53 | MS (ESI+), | HPLC |
| | | | m/z: 461 | (Methode 5): |
| | | | (M+H)⁺ | Rₜ = 2.86 min |
| 168A | 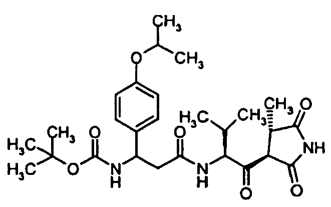 | 517.63 | MS (ESI-), | HPLC |
| | | | m/z: 516 | (Methode 14): |
| | | | (M-H)⁻ | Rₜ = 4.42 min |
| 169A | 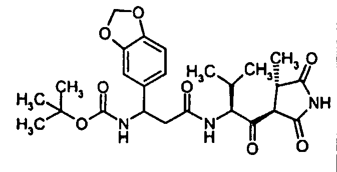 | 503.56 | MS (ESI+), | HPLC |
| | | | m/z: 504 | (Methode 14): |
| | | | (M+H)⁺ | Rₜ = 4.05 min |
| 170A | 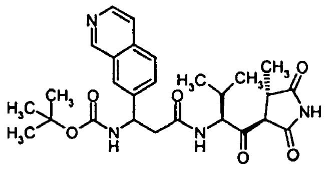 | 510.59 | MS (ESI+), | HPLC |
| | | | m/z: 511 | (Methode 21): |
| | | | (M+H)⁺ | Rₜ = 2.50 min |

Nach der Allgemeinen Vorschrift E können folgende Verbindungen (Beispiel 171A bis 188A) in Form ihrer Hydrochloride erhalten werden:

| **Beispiel** | **Struktur** | **MW** | **MS** | **HPLC** |
|---|---|---|---|---|
| 171A | 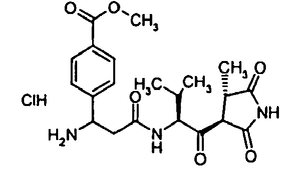 | 417.47 | MS (ESI-), | HPLC |
| | | | m/z: 416 | (Methode 5): |
| | | | (M-H)⁻ | Rₜ = 2.23 min |
| 172A | 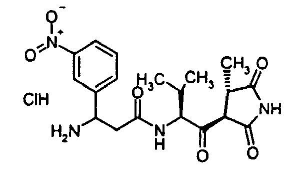 | 404.43 | | |
| 173A | 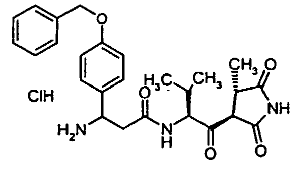 | 465.55 | MS (ESI-), | HPLC |
| | | | m/z: 464 | (Methode 17): |
| | | | (M-H)⁻ | Rₜ = 2.63 min |
| 174A | 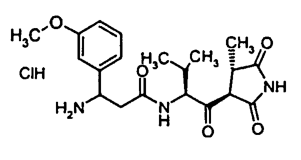 | 389.46 | MS (ESI+), | HPLC |
| | | | m/z:390 | (Methode 17): |
| | | | (M+H)⁺ | Rₜ = 2.14 u. 2.25 min |
| 175A | 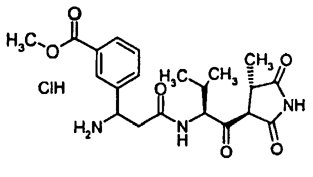 | 417.47 | | |
| 176A | 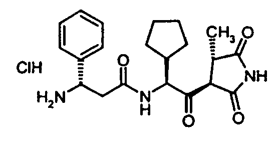 | 385.47 | MS (ESI-), | HPLC |
| | | | m/z: 384 | (Methode 20): |
| | | | (M-H)⁻ | Rₜ = 1.90 min |
| 177A | 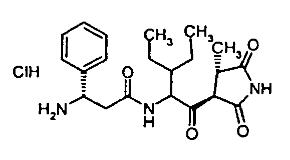 | 387.48 | | |
| 178A | 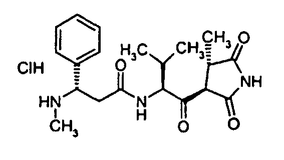 | 373.46 | MS (ESI-), | HPLC |
| | | | m/z: 372 | (Methode 12): |
| | | | (M-H)⁻ | Rₜ = 1.33 min |
| 179A | 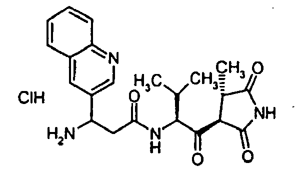 | 410.48 | | HPLC |
| | | | | (Methode 9): |
| | | | | Rₜ = 2.79 min |
| 180A | 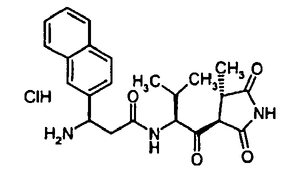 | 409.49 | MS(ESI-), | HPLC |
| | | | m/z:408 | (Methode 20): |
| | | | (M-H)⁻ | Rₜ=2.14+ 2.23 min |
| 181A | 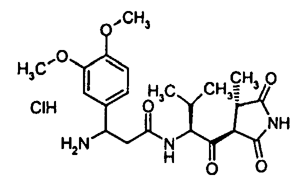 | 419.48 | | HPLC |
| | | | | (Methode 9): |
| | | | | Rₜ = 2.80 min |
| 182A | 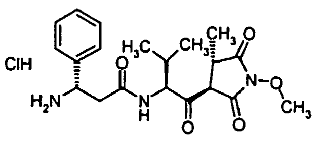 | 389.46 | MS (ESI-), | HPLC |
| | | | m/z: 388 | (Methode 19): |
| | | | (M-H)⁻ | Rₜ = 3.17 min |
| 183A | 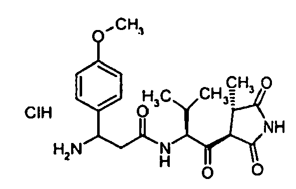 | 389.46 | | HPLC |
| | | | | (Methode 9): |
| | | | | Rₜ = 2.86 min |
| 184A | 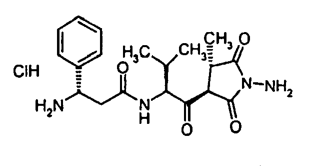 | 374.44 | | |
| 185A | 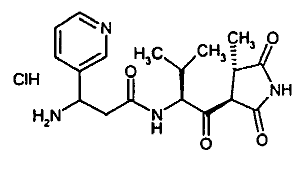 | 360.42 | MS (ESI+), | |
| | | | m/z: 361 | |
| | | | (M+H)⁺ | |
| 186A | 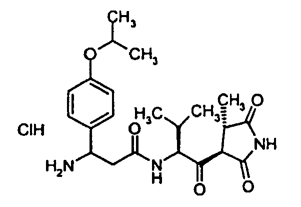 | 417.51 | MS (ESI-), | HPLC |
| | | | m/z: 416 | (Methode 9): |
| | | | (M-H)⁻ | Rₜ = 2.97 min |
| 187A | 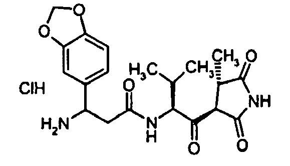 | 403.44 | MS (ESI-), | HPLC |
| | | | m/z: 402 | (Methode 14): |
| | | | (M-H)⁻ | Rₜ = 2.40 min |
| 188A | 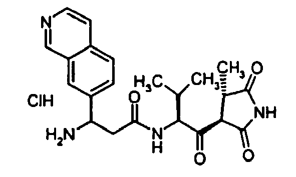 | 410.48 | | |

### Herstellungsbeispiele:

### Allgemeine Vorschrift K: Acylierung von acylalkylamino substituierten 3-[2-Amino-alkanoyl]-2,5-pyrrolidindion Hydrochlorid Derivaten mit Carbonsäurederivaten

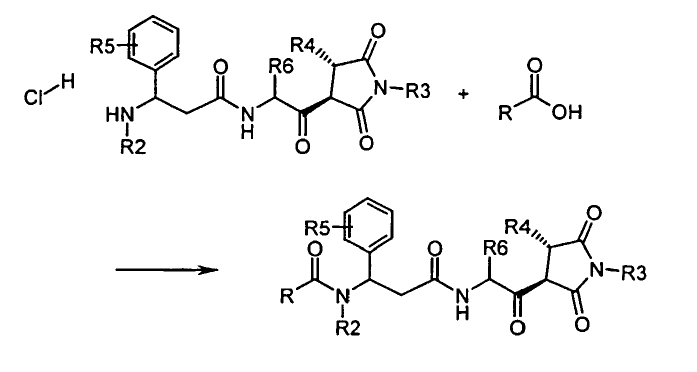

Zu einer Mischung aus Aminhydrochlorid (1.0 eq.), Carbonsäure (1.2 bis 1.3 eq.) und HATU (1.2 - 1.4 eq.) gelöst in absolutem N,N-Dimethylformamid oder in einer 1:1 Mischung aus N,N-Dimethylformamid und Dichlormethan (0.02 - 0.2 mol/l) wird bei 0°C eine 0.2 - 1.0 molare Lösung von Diisopropylethylamin (2.5 bis 3.5 eq.) in N,N-Dimethylformamid oder einer 1:1 Mischung aus N,N-Dimethylformamid und Dichlormethan über den Zeitraum von 1 h zugetropft. Nach Ende der Zugabe wird die Reaktionsmischung noch 30 min bei 0°C und über Nacht bei Raumtemperatur gerührt, bevor das Gemisch im Vakuum eingeengt wird. Das Produkt kann durch Chromatographie an Silicagel (Eluenten: Gemische aus Cyclohexan/Essigsäureethylester oder Gemische aus Dichlormethan und Ethanol) oder durch RP-HPLC (Eluenten: variable Gradienten aus Wasser und Acetonitril), alternativ durch eine Kombination beider Verfahren, erhalten werden.

Alternativ kann die Umsetzung auch nach folgendem Verfahren erfolgen: Zu einer Mischung aus Aminhydrochlorid (1.0 eq.), Carbonsäure (1.2 bis 1.3 eq.), Triethylamin (2.4 - 3 eq.) und HOBt (2.4 - 3 eq.) in absolutem Dichlormethan oder in einer Mischung aus N,N-Dimethylformamid und Dichlormethan (0.02 - 0.2 mol/l) werden abschließend 1.2 eq. EDC gegeben. Die Reaktionsmischung wird bei Raumtemperatur gerührt (2 h bis über Nacht), bevor im Vakuum eingeengt wird. Der Rückstand wird in Essigsäureethylester oder Dichlormethan aufgenommen und die organische Phase wird mit Wasser, gesättigter Natriumhydrogencarbonatlösung und gesättigter Kochsalzlösung gewaschen, über Natriumsulfat getrocknet, filtriert und eingeengt. Das Produkt kann durch Chromatographie an Silicagel (Eluenten: Gemische aus Cyclohexan/ Essigsäureethylester oder Gemische aus Dichlormethan und Ethanol) oder durch RP-HPLC (Eluenten: variable Gradienten aus Wasser und Acetonitril), alternativ durch eine Kombination beider Verfahren, gereinigt werden.

### Allgemeine Vorschrift L: Festphasengestützte Synthese

Das Aldehydharz (Nova Biochem) (0.78 mmol/g) wird in Toluol/Trimethylorthoformiat (1:1 bis 4:1) suspendiert, mit dem entsprechenden beta-Aminosäuremethylester (2.5 - 3 eq) bei Raumtemperatur versetzt und über Nacht geschüttelt. Das Harz wird zweimal mit N,N-Dimethylformamid gewaschen, in N,N-Dimethylformamid suspendiert und bei Raumtemperatur mit Tetrabutylammoniumborhydrid (2 - 5 eq) versetzt. Nach 30 Minuten Schütteln bei Raumtemperatur wird das Reaktionsgemisch bei -40°C bis Raumtemperatur langsam mit Eisessig (100 eq) versetzt, gegebenenfalls wieder auf Raumtemperatur erwärmt und für mindestens 1 h geschüttelt. Das Harz wird wiederholt mit Wasser, Methanol, Dichlormethan/10 % N,N-Diisopropylethylamin, Methanol, Dichlormethan und Diethylether gewaschen und getrocknet. Das Harz wird in Dichlormethan suspendiert, mit N,N-Diisopropylethylamin (10 - 20 eq) und dem entsprechenden Carbonsäurechlorid (5 eq) bei Raumtemperatur 1 - 2 h lang geschüttelt. Das Harz wird wiederholt mit Methanol, N,N-Dimethylformamid, Methanol, Dichlormethan und Diethylether gewaschen und getrocknet. Zur Verseifung wird das Harz mit einer Lösung von Kaliumhydroxid (30 eq) in Methanol/Dioxan (1:2, 30 mg Kaliumhydroxid/ml Lösung) versetzt und 3 h bei RT geschüttelt. Anschließend wird das Harz mit Wasser, Methanol, Dichlormethan/Eisessig, Dichlormethan, Dichlormethan/N,N-Diisopropylethylamin, Methanol, N,N-Dimethylformamid, Methanol, Dichlormethan und Diethylether gewaschen. Das Harz wird mit (Benzotriazol-1-yloxy)bisdimethylaminomethyliumfluoroborat (5eq) und N,N-Diisopropylethylamin (20 eq) in N,N-Dimethylacetamid bei RT 1h geschüttelt, zweimal mit N,N-Dimethylacetamid gewaschen und mit einer frisch zubereiteten Lösung von (3*R*,4*S*)-3-[(2*S*)-2-Amino-3-methylbutanoyl]-4-methyl-2,5-pyrrolidindion Hydrochlorid (1.5 - 2 e^{q}) und N,N-Diisopropylethylamin (20 eq) versetzt und 3 h bei RT geschüttelt. Das Harz wird abschließend wiederholt mit Methanol, N,N-Dimethylformamid, Wasser, N,N-Dimethylformamid, Methanol, Dichlormethan und Diethylether gewaschen und getrocknet. Das Harz wird mit Trifluoressigsäure oder 50 %iger Trifluoressigsäure in Dichlormethan 30 min bis 3 h bei RT bis 50°C geschüttelt. Die Rohproduktlösung wird abfiltriert, zur Trockene eingedampft und durch Reversed Phase HPLC mit einem Wasser/Acetonitril-Gradienten gereinigt. Alternativ ist auch eine Chromatographie an Silicagel (Eluenten: Gemische aus Dichlormethan und Methanol) möglich.

Gemäß den oben beschriebenen Vorschriften zur Acylierung von 3-[2-Aminoalkanoyl]-2,5-pyrrolidindion Hydrochlorid Derivaten (Allgemeine Vorschrift D) oder von acylalkylamino substituierten 3-[2-Amino-alkanoyl]-2,5-pyrrolidindion Hydrochlorid Derivaten (Allgemeine Vorschrift K) mit Carbonsäurederivaten oder der Festphasengestützten Synthese (Allgemeinen Vorschrift L) können folgende Verbindungen erhalten werden.

### Beispiel 1

### (2E)-3-(1,3-Benzodioxol-5-yl)-N-{(1S)-3-[((1S)-2-methyl-1-{[(3R,4S)-4-methyl-2,5-dioxo-3-pyrrolidinyl]carbonyl}propyl)amino]-3-oxo-1-phenylpropyl}-2-propenamid

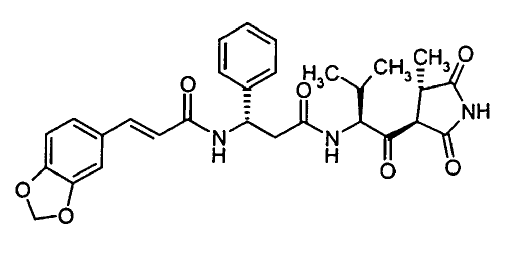

¹H-NMR (400 MHz, d₆-DMSO): δ = 11.34 (s, 1 H), 8.44 (d, 1 H), 8.12 (d, 1 H), 7.46-7.38 (m, 5 H), 7.35-7.29 (m, 1 H), 7.13 (s, 1 H), 7.05 (d, 1 H), 6.92 (d, 1 H), 6.50 (d, 1 H), 6.07 (s, 2 H), 5.47-5.30 (m, 1 H), 4.61 (dd, 1 H), 3.91 (d, 1 H), 2.95-2.90 (m, 1 H), 2.80 (dd, 1 H), 2.69 (dd, 1 H), 2.32-2.25 (m, 1 H), 1.08 (d, 3 H), 0.79 (d, 3 H), 0.74 (d, 3 H).
MS (ESI+): m/z (%) = 534 (M+H⁺) (100).
HPLC (Methode 4): Rₜ = 2.34 min.

### Beispiel 2

### (2E)-3-(1,3-Benzodioxol-5-yl)-N-{(1S)-3-[((1S)-2,2-dimethyl-1-{[(3R,4S)-4-methyl-2,5-dioxo-3-pyrrolidinyl]carbonyl}propyl)amino]-3-oxo-1-phenylpropyl}-2-propenamid

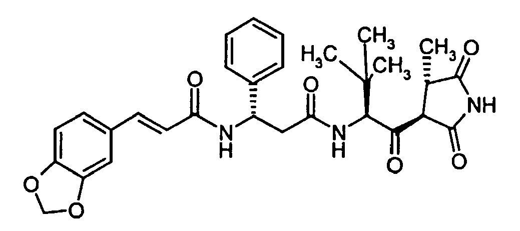

¹H-NMR (200 MHz, d₆-DMSO): δ = 11.40 (s, 1 H), 8.46 (d, 1 H), 8.19 (d, 1 H), 7.35-6.90 (m, 9 H), 6.50 (d, 1 H), 6.07 (s, 2 H), 5.37-5.25 (m, 1 H), 4.39 (br. d, 1 H), 3.85-3.75 (m, 1 H), 2.90-2.63 (m, 3 H), 1.09 (d, 3 H), 0.92 (s, 9 H).
MS (ESI+): m/z (%) = 548 (M+H⁺) (100).
HPLC (Methode 8): Rₜ = 2.45 min.

### Beispiel 3

### (2E)-3-(1,3-Benzodioxol-5-yl)-N-{(1S)-3-[((1S)-1-{[(3R,4S)-1,4-dimethyl-2,5-dioxo-3-pyrrolidinyl]carbonyl}-2-methylpropyl)amino]-3-oxo-1-phenylpropyl}-2-propenamid

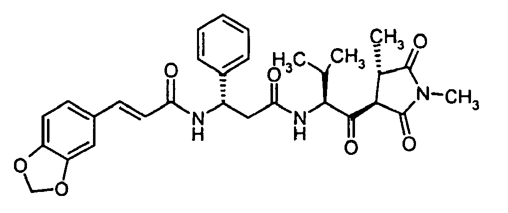

¹H-NMR (400 MHz, d₆-DMSO): δ = 8.45 (d, 1 H), 8.16 (d, 1 H), 7.36-7.25 (m, 5 H), 7.22-7.18 (m, 1 H), 7.12 (s, 1 H), 7.03 (d, 1 H), 6.94 (d, 1 H), 6.50 (d, 1 H), 6.06 (s, 2 H), 5.38-5.28 (m, 1 H), 4.66 (dd, 1 H), 3.88 (d, 1 H), 3.00-2.92 (m, 1 H), 2.82 (s, 3 H) 2.78-2.65 (m, 2 H), 2.33-2.27 (m, 1 H), 1.10 (d, 3 H), 0.80 (d, 3 H), 0.76 (d, 3 H).
MS (ESI+): m/z (%) = 548 (M+H⁺) (100).
HPLC (Methode 5): Rₜ = 2.34 min.

### Beispiel 4

### (2E)-3-(1,1'-Biphenyl-4-yl)-N-{(1S)-3-[((1S)-2-methyl-1-{[(3R,4S)-4-methyl-2,5-dioxo-3-pyrrolidinyl]carbonyl}propyl)amino]-3-oxo-1-phenylpropyl}-2-propenamid

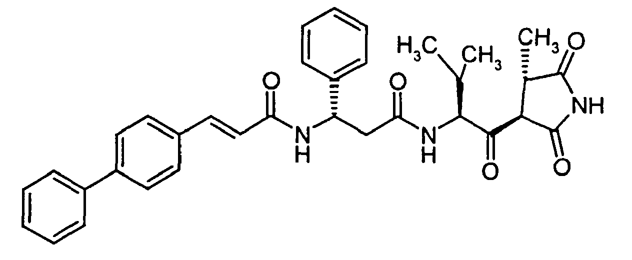

¹H-NMR (400 MHz, d₆-DMSO): δ = 11.33 (s, 1 H), 8.54 (d, 1 H), 8.13 (d, 1 H), 7.76-7.61 (m, 7 H), 7.50-7.21 (m, 8 H), 6.70 (s, 2 H), 5.40-5.30 (m, 1 H), 4.63 (dd, 1 H), 3.92 (d, 1 H), 2.95-2.89 (m, 1 H), 2.82 (dd, 1 H), 2.70 (dd, 1 H), 2.32-2.28 (m, 1 H), 1.09 (d, 3 H), 0.80 (d, 3 H), 0.75 (d, 3 H).
MS (ESI+): m/z (%) = 566 (M+H⁺) (100).
HPLC (Methode 4): Rₜ = 2.68 min.

### Beispiel 5

### (2E)-3-(1,3-Benzodioxol-5-yl)-N-{1-(2,3-dihydro-1,4-benzodioxin-6-yl)-3-[((1S)-2-methyl-1-{[(3R,4S)-4-methyl-2,5-dioxo-3-pyrrolidinyl]carbonyl}propyl)amino]-3-oxopropyl}-2-propenamid

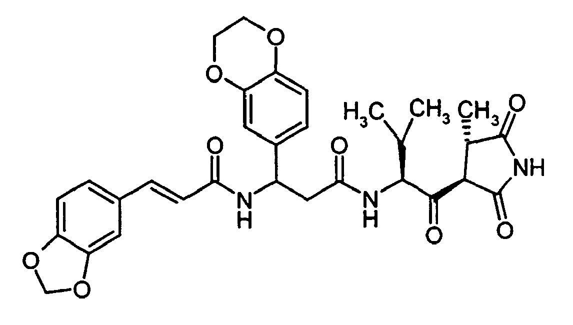

¹H-NMR (2 Diastereoisomere, 400 MHz, CDCl₃): δ = 8.41 (br. s, 1 H), 8.01 (br. d, 1 H), 7.56-7.48 (m, 1 H), 7.01-6.96 (m, 2 H), 6.85-6.75 (m, 3 H), 6.34 (d, 0.5 H), 6.28 (d, 0.5 H), 6.00 (s, 1 H), 5.99 (s, 1 H), 5.61-5-59 (m, 1 H), 5.50-5.46 (m, 1 H), 4.81 (dd, 0.5 H); 4.70 (dd, 0.5 H), 4.20-4.16 (m, 4 H), 3.98-3.93 (m, 1.5 H), 3.48-3.33 (m, 0.5 H), 3.30-3.29 (m, 0.5 H), 2.94-2.88 (m, 1.5 H), 2.49-2.35 (m, 1 H), 1.24 (d, 1.5 H), 1.17 (d, 1.5 H), 0.93 (d, 1.5 H), 0.82 (d, 1.5 H), 0.75 (d, 1.5 H), 0.69 (d, 1.5 H).
MS (ESI+): m/z = 592 (M+H⁺).

### Beispiel 6

### (2E)-N-{1-(2,3-Dihydro-1,4-benzodioxin-6-yl)-3-[((1S)-2-methyl-1-{[(3R,4S)-4-methyl-2,5-dioxo-3-pyrrolidinyl]carbonyl}propyl)amino]-3-oxopropyl}-3-phenyl-2-propenamid

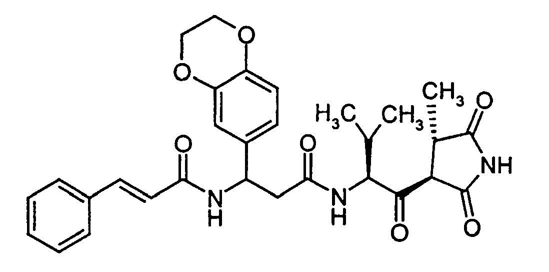

MS (ESI+): m/z (%) = 548 (M+H⁺) (100).
HPLC (Methode 9): Rₜ = 4.00 min.

| **Beispiel** | **Struktur** | **MW** | **MS** | **HPLC** |
|---|---|---|---|---|
| 7 | 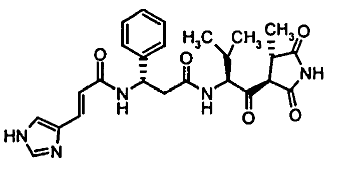 | 479,53 | MS (ES+), m/z | HPLC |
| | | | (%): 480 | (Methode 6): |
| | | | (M+H)⁺ (100) | Rt = 2.41 min |
| 8 | 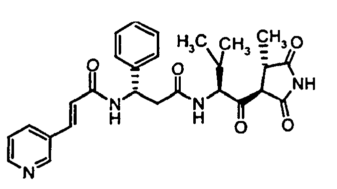 | 490,56 | MS (ES+), m/z | HPLC |
| | | | (%): 491 | (Methode 6): |
| | | | (M+H)⁺ (100) | Rt = 2.83 min |
| 9 | 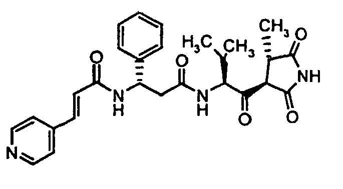 | 490,56 | MS (ES+), m/z | HPLC |
| | | | (%): 491 | (Methode 6): |
| | | | (M+H)⁺ (100) | Rt = 2.59 min |
| 10 | 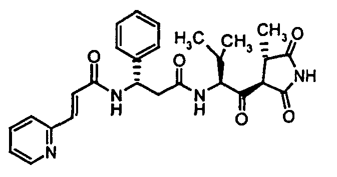 | 490,56 | MS (ES+), m/z | HPLC |
| | | | (%): 491 | (Methode 6): |
| | | | (M+H)⁺ (100) | Rt = 3.12 min |
| 11 | 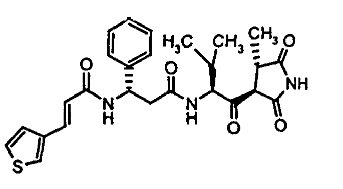 | 495,60 | MS (ES+), m/z | HPLC |
| | | | (%): 496 | (Methode 6): |
| | | | (M+H)⁺ (100) | Rt = 3.74 min |
| 12 | 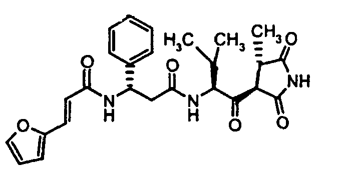 | 479,53 | MS (ES+), m/z | HPLC |
| | | | (%): 480 | (Methode 6): |
| | | | (M+H)⁺ (100) | Rt = 3.62 min |
| 13 | 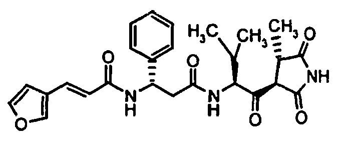 | 479,53 | MS (ES+), m/z | HPLC |
| | | | (%): 480 | (Methode 6): |
| | | | (M+H)⁺ (100) | Rt = 3.59 min |
| 14 | 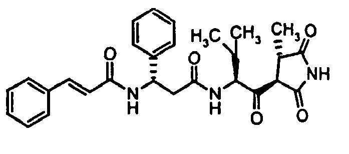 | 489,57 | MS (ES+), m/z | HPLC |
| | | | (%): 490 | (Methode 6): |
| | | | (M+H)⁺ (100) | Rt = 3.83 min |
| 15 | 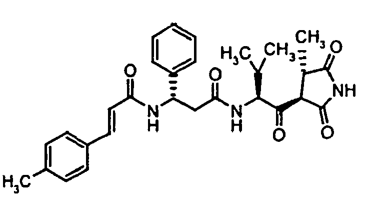 | 503,60 | MS (ES+), m/z | HPLC |
| | | | (%): 504 | (Methode 6): |
| | | | (M+H)⁺ (100) | Rt = 4.00 min |
| 16 | 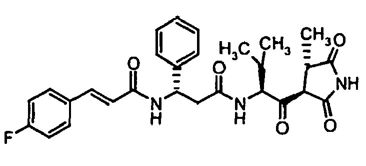 | 507,56 | MS (ES+), m/z | HPLC |
| | | | (%): 508 | (Methode 6): |
| | | | (M+H)⁺ (100) | Rt = 3.88 min |
| 17 | 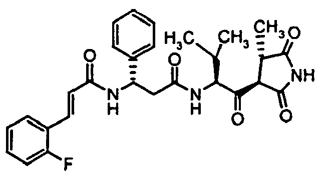 | 507,56 | MS (ES+), m/z | HPLC |
| | | | (%): 508 | (Methode 6): |
| | | | (M+H)⁺ (100) | Rt = 3.88 min |
| 18 | 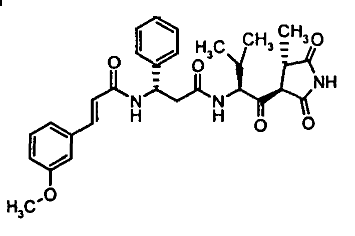 | 519,59 | MS (ES+), m/z | HPLC |
| | | | (%): 520 | (Methode 6): |
| | | | (M+H)⁺ (100) | Rt = 3.86 min |
| 19 | 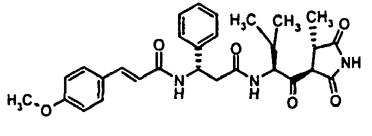 | 519,59 | MS (ES+), m/z | HPLC |
| | | | (%): 520 | (Methode 6): |
| | | | (M+H)⁺ (100) | Rt = 3.81 min |
| 20 | 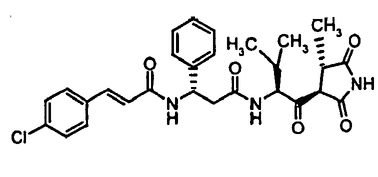 | 524,01 | MS (ES+), m/z | HPLC |
| | | | (%): 524 | (Methode 6): |
| | | | (M+H)⁺ (100) | Rt = 4.07 min |
| 21 | 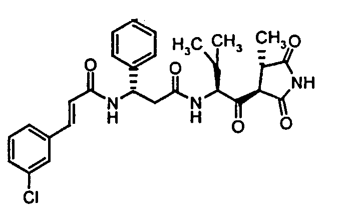 | 524,01 | MS (ES+), m/z | HPLC |
| | | | (%): 524 | (Methode 6): |
| | | | (M+H)⁺ (100) | Rt = 4.06 min |
| 22 | 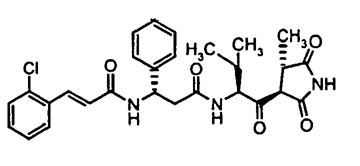 | 524,01 | MS (ES+), m/z | HPLC |
| | | | (%): 524 | (Methode 6): |
| | | | (M+H)⁺ (100) | Rt = 4.00 min |
| 23 | 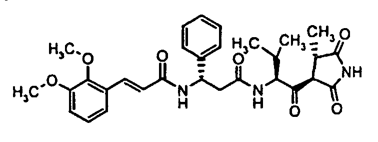 | 549,62 | MS (ES+), m/z | HPLC |
| | | | (%): 550 | (Methode 6): |
| | | | (M+H)⁺ (100) | Rt = 3.81 min |
| 24 | 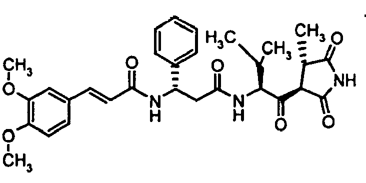 | 549,62 | MS (ES+), m/z | HPLC |
| | | | (%): 550 | (Methode 6): |
| | | | (M+H)⁺ (100) | Rt = 3.64 min |
| 25 | 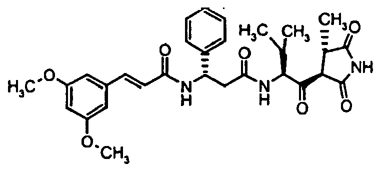 | 549,62 | MS (ES+), m/z | HPLC |
| | | | (%): 550 | (Methode 6): |
| | | | (M+H)⁺ (100) | Rt = 3.90 min |
| 26 | 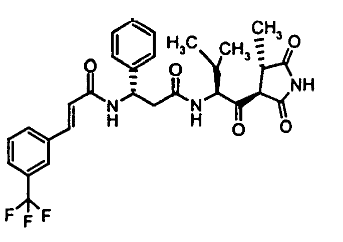 | 557,57 | MS (ES+), m/z | HPLC |
| | | | (%): 558 | (Methode 6): |
| | | | (M+H)⁺(100) | Rt = 4.17 min |
| 27 | 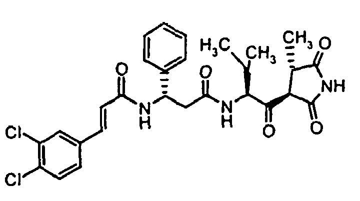 | 558,46 | MS (ES+), m/z | HPLC |
| | | | (%): 558 | (Methode 6): |
| | | | (M+H)⁺ (100) | Rt = 4.29 min |
| 28 | 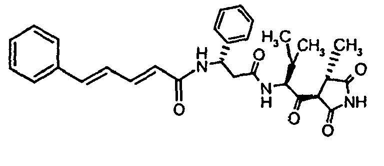 | 515,61 | MS (ES+), m/z | HPLC |
| | | | (%): 516 | (Methode 6): |
| | | | (M+H)⁺ (100) | Rt = 4.11 min |
| 29 | 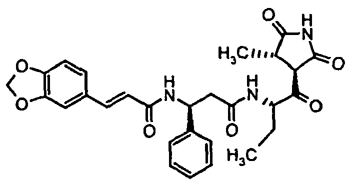 | 519,55 | MS (ES+), m/z | HPLC |
| | | | (%): 520 | (Methode 5): |
| | | | (M+H)⁺ (100) | Rt = 3.63 min |
| 30 | 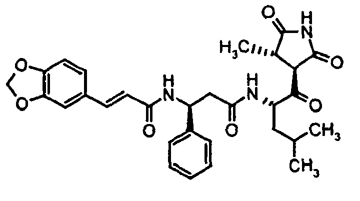 | 547,60 | MS (ES+), m/z | HPLC |
| | | | (%): 548 | (Methode 5): |
| | | | (M+H)⁺ (100) | Rt = 3.98 min |

### Beispiel 31

### (2E)-3-(2H-Benzo[d]1,3-dioxolan-5-yl)-N-((1S)-2-{N-[(1S)-2-((4S,3R)-4-methyl-2,5-dioxoazolidin-3-yl)-1-cyclopentyl-2-oxoethyl]carbamoyl}-1-phenylethyl)prop-2-enamid

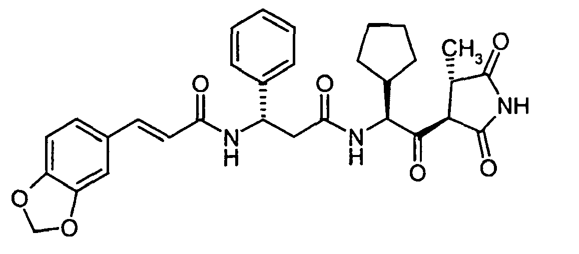

¹H-NMR (400 MHz, d₆-DMSO): δ = 11.33 (s, 1 H), 8.49 (br. s, 1 H), 8.40 (br. d, 1 H), 7.37-7.18 (m, 6 H), 7.12 (s, 1 H), 7.04 (d, 1 H), 6.93 (d, 1 H), 6.51 (d, 1 H), 6.06 (s, 2 H), 5.48-5.27 (m, 1 H), 4.52 (t, 1 H), 3.89 (m, 1 H), 2.91-2.78 (m, 2 H), 2.69-2.60 (m, 1 H), 2.48-2.27 (m, 1 H), 1.60-1.32 (m, 6 H), 1.26-1.03 (m, 3 H), 1.05 (d, 2 H).
MS (ESI+): m/z = 560 (M+H⁺).
HPLC (Methode 22): Rₜ = 4.15 min.

### Beispiel 32

### (2E)-N-((1S)-2-{N-[(1S)-2-((4S,3R)-4-Methyl-2,5-dioxoazolidin-3-yl)-1-cyclopentyl-2-oxoethyl]carbamoyl}-1-phenylethyl)-3-(2,2-difluorbenzo[3,4-d] 1,3-dioxolen-5-yl)prop-2-enamid

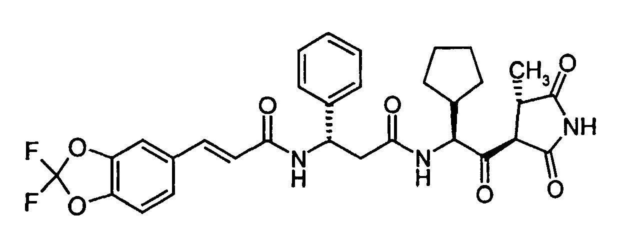

¹H-NMR (200 MHz, d₆-DMSO): δ = 11.34 (s, 1 H), 8.57 (br. s, 1 H), 8.29 (br. d, 1 H), 7.63 (s, 1 H), 7.50-7.12 (m, 7 H), 6.67 (br. d, 1 H), 5.43-5.24 (m, 1 H), 4.63-4.41 (m, 1 H), 4.40-4.20 (m, 1 H), 3.88 (d, 1 H), 2.97-2.57 (m, 3 H), 2.41-2.12 (m, 1 H), 1.54-1.28 (m, 6 H), 1.28-0.97 (m, 5 H).
MS (ESI+): m/z = 596.3 (M+H⁺).
HPLC (Methode 19): Rₜ = 4.24 min.

### Beispiel 33

### (2E)-N-((1S)-2-{N-[(1S)-2-((4S,3R)-4-Methyl-2,5-dioxoazolidin-3-yl)-1-cyclopentyl-2-oxoethyl]carbamoyl}-1-phenylethyl)-3-(4-cyanophenyl)prop-2-enamid

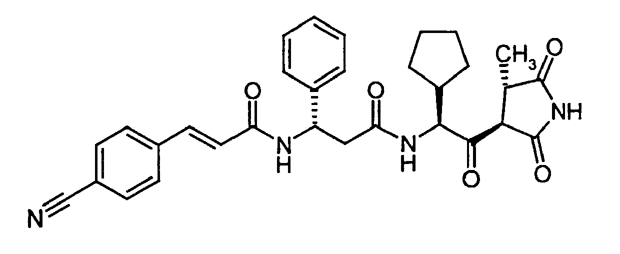

¹H-NMR (300 MHz, d₆-DMSO): δ = 11.31 (s, 1 H), 8.70-8.56 (m, 1 H), 8.27 (d, 1 H), 7.87 (d, 2 H), 7.72 (d, 2H), 7.50-7.16 (m, 6 H), 6.80 (d, 1 H), 5.34 (dd, 1 H), 4.57-4.22 (m, 1 H), 3.87 (m, 1 H), 2.93-2.61 (m, 3 H), 2.46-2.17 (m, 2 H), 1.61-1.29 (m, 6 H), 1.28-0.96 (m, 4 H).
MS (ESI+): m/z = 541 (M+H⁺).
HPLC (Methode 19): Rₜ= 4.10 min.

### Beispiel 34

### (2E)-N-((1S)-2-{N-[(1S)-2-((4S,3R)-1-Amino-4-methyl-2,5-dioxoazolidin-3-yl)-1-cyclopentyl-2-oxoethyl]carbamoyl}-1-phenylethyl)-3-(4-cyanophenyl)prop-2-enamid

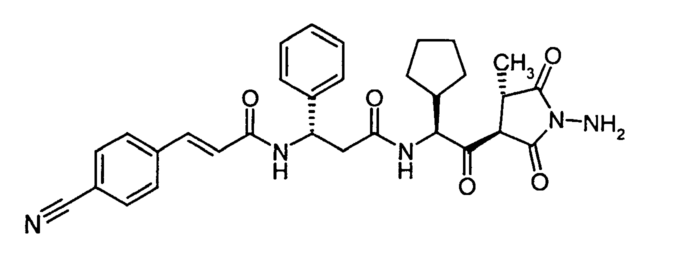

¹H-NMR (300 MHz, d₆-DMSO): δ = 8.72-8.57 (m, 1 H), 8.54-8.23 (m, 1 H), 7.88 (d, 2 H), 7.72 (d, 2 H), 7.45 (d, 1H), 7.39-7.16 (m, 5 H), 6.81 (d, 1 H), 5.43-5.27 (m, 1 H), 4.53-4.31 (m, 1 H), 3.71 (d, 1 H), 2.94-2.63 (m, 3 H), 2.42-2.23 (m, 2 H), 2.04-1.93 (m, 1H), 1.67-1.32 (m, 6 H), 1.21-1.02 (m, 5 H).
MS (ESI+): m/z = 556 (M+H⁺).
HPLC (Methode 19): Rₜ = 4.08 min.

| **Beispiel** | **Struktur** | **MW** | **MS** | **HPLC** |
|---|---|---|---|---|
| 35 | 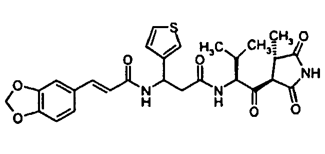 | 539.61 | MS (ESI+), | HPLC |
| | | | m/z: 540 | (Methode 9): |
| | | | (M+H)⁺ | Rₜ = 3.97 min |
| 36 | 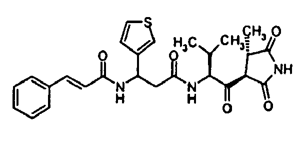 | 495.60 | MS (ESI+), | HPLC |
| | | | m/z: 496 | (Methode 9): |
| | | | (M+H)⁺ | Rₜ = 4.04 min |
| 37 | 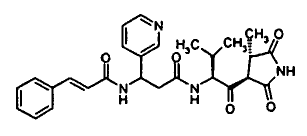 | 490.56 | MS (ESI+), | HPLC |
| | | | m/z:491 | (Methode 5): |
| | | | (M+H)⁺ | Rₜ = 2.74 u. 2.78 min |
| 38 | 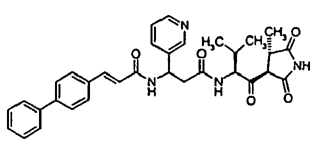 | 566.66 | MS (ESI+), | HPLC |
| | | | m/z: 567 | (Methode 5): |
| | | | (M+H)⁺ | Rₜ=3.37min |
| 39 | 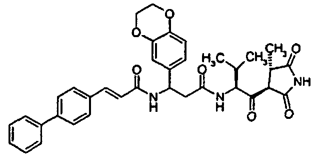 | 623.70 | MS (ESI+), | HPLC |
| | | | m/z: 624 | (Methode 5): |
| | | | (M+H)⁺ | Rₜ = 4.30 min |
| 40 | 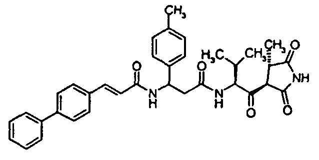 | 579.69 | MS (ESI+), | HPLC |
| | | | m/z: 580 | (Methode 9): |
| | | | (M+H)⁺ | Rₜ = 4.60 min |
| 41 | 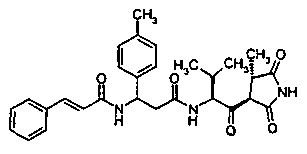 | 503.60 | MS (ESI+), | HPLC |
| | | | m/z: 504 | (Methode 9): |
| | | | (M+H)⁺ | Rₜ = 4.19 min |
| 42 | 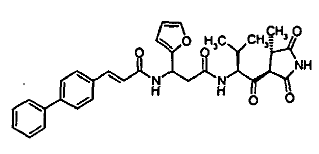 | 555.63 | MS (ESI+), | HPLC |
| | | | m/z: 556 | (Methode 9): |
| | | | (M+H)⁺ | Rₜ = 4.40 min |
| 43 | 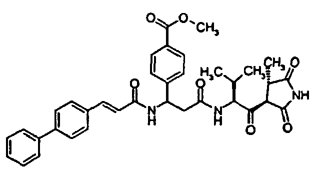 | 623.70 | MS (ESI+), | HPLC |
| | | | m/z: 624 | (Methode 9): |
| | | | (M+H)⁺ | Rₜ = 4.45 min |
| 44 | 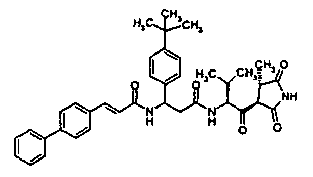 | 621.77 | MS (ESI+), | HPLC |
| | | | m/z: 622 | (Methode 5): |
| | | | (M+H)⁺ | Rₜ = 5.01 min |
| 45 | 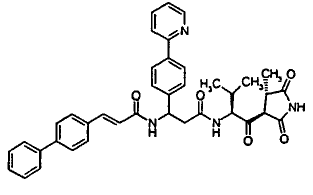 | 642.75 | MS (ESI+), | HPLC |
| | | | m/z: 643 | (Methode 5): |
| | | | (M+H)⁺ | Rₜ = 4.21 min |
| 46 | 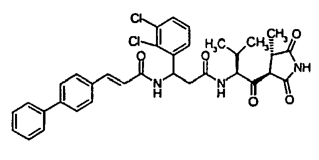 | 634.56 | MS (ESI+), | HPLC |
| | | | m/z: 634 | (Methode 5): |
| | | | (M+H)⁺ | Rₜ = 4.79 min |
| 47 | 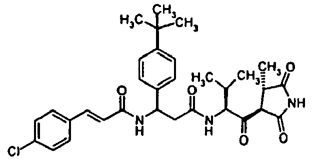 | 580.12 | MS (ESI+), | HPLC |
| | | | m/z: 580 | (Methode 5): |
| | | | (M+H)⁺ | Rₜ = 4.77 min |
| 48 | 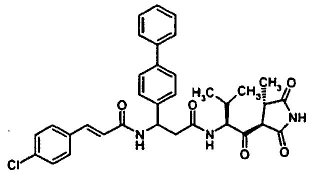 | 600.11 | MS (ESI+), | HPLC |
| | | | m/z: 600 | (Methode 5): |
| | | | (M+H)⁺ | Rₜ = 4.65 min |
| 49 | 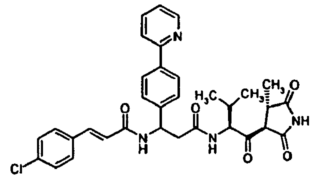 | 601.10 | MS (ESI+), | HPLC |
| | | | m/z: 601 | (Methode 14): |
| | | | (M+H)⁺ | Rₜ=4.13min |
| 50 | 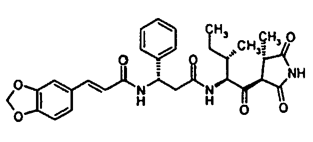 | 547.61 | MS (ESI+), | HPLC |
| | | | m/z: 548 | (Methode 12): |
| | | | (M+H)⁺ | Rₜ = 2.92 min |
| 51 | 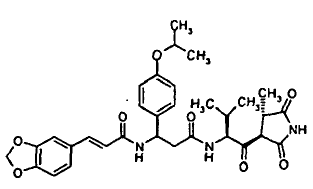 | 591.66 | MS (ESI+), | HPLC |
| | | | m/z: 592 | (Methode 14): |
| | | | (M+H)⁺ | Rₜ = 4.39 min |
| 52 | 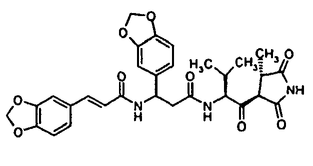 | 577.59 | MS (ESI+), | HPLC |
| | | | m/z: 578 | (Methode 10): |
| | | | (M+H)⁺ | Rₜ = 2.88 min |
| 53 | 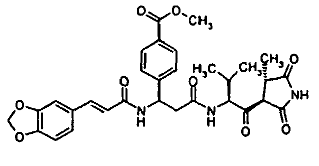 | 591.61 | MS (ESI+), | HPLC |
| | | | m/z: 592 | (Methode 14): |
| | | | (M+H)⁺ | Rₜ = 4.25 min |
| 54 | 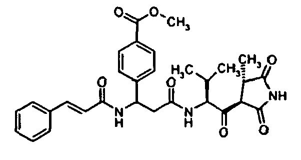 | 547.61 | MS (ESI+), | HPLC |
| | | | m/z: 548 | (Methode 6): |
| | | | (M+H)⁺ | Rₜ = 3.84 min |
| 55 | 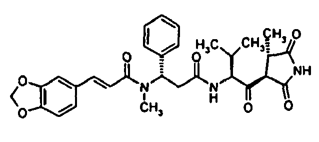 | 547.61 | MS (ESI+), | HPLC |
| | | | m/z: 570 | (Methode 13): |
| | | | (M+Na)⁺ | Rₜ = 4.13min |
| 56 | 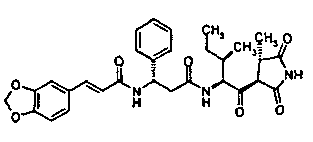 | 547.61 | MS (ESI+), | HPLC |
| | | | m/z: 548 | (Methode 12): |
| | | | (M+H)⁺ | Rₜ = 2.99 min |
| 57 | 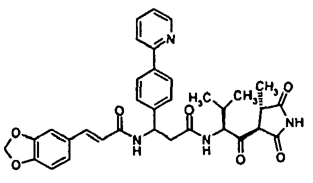 | 610.66 | MS (ESI+), | HPLC |
| | | | m/z: 611 | (Methode 12): |
| | | | (M+H)⁺ | Rₜ = 2.45 min |
| 58 | 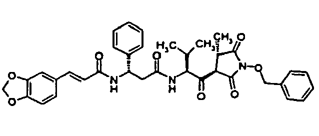 | 639.70 | MS (ESI+), | HPLC |
| | | | m/z: 640 | (Methode 12): |
| | | | (M+H)⁺ | Rₜ = 3.50 min |
| 59 | 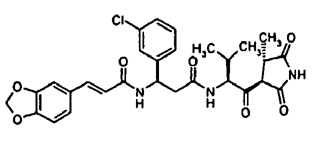 | 568.02 | MS (ESI+), | HPLC |
| | | | m/z: 568 | (Methode 16): |
| | | | (M+H)⁺ | Rₜ = 2.99 min |
| 60 | 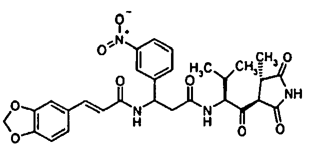 | 578.58 | MS (ESI+), | HPLC |
| | | | m/z: 579 | (Methode 9): |
| | | | (M+H)⁺ | Rₜ = 4.06 min |
| 61 | 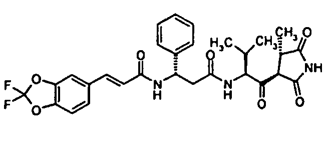 | 569.56 | MS (ESI+), | HPLC |
| | | | m/z: 570 | (Methode 14): |
| | | | (M+H)⁺ | Rₜ = 4.47 min |
| 62 | 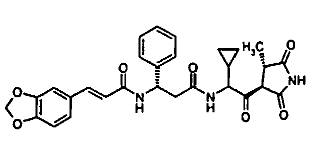 | 531.56 | MS (ESI+), | HPLC |
| | | | m/z: 532 | (Methode 16): |
| | | | (M+H)⁺ | Rₜ = 2.65 min |
| 63 | 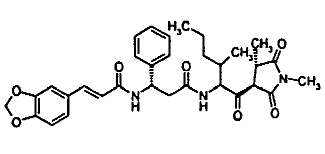 | 575.66 | MS (ESI+), | HPLC |
| | | | m/z: 576 | (Methode 16): |
| | | | (M+H)⁺ | Rₜ = 3.29 min |
| 64 | 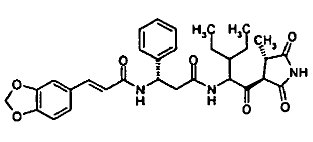 | 561.63 | MS (ESI+), | HPLC |
| | | | m/z: 562 | (Methode 16): |
| | | | (M+H)⁺ | Rₜ = 3.06 min |
| 65 | 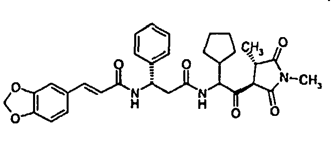 | 573.64 | MS (ESI+), | HPLC |
| | | | m/z: 574 | (Methode 17): |
| | | | (M+H)⁺ | Rₜ=3.72 min |
| 66 | 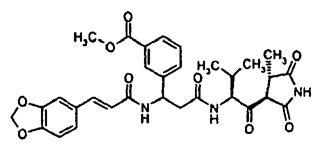 | 591.61 | MS (ESI+), | HPLC |
| | | | m/z: 592 | (Methode 14): |
| | | | (M+H)⁺ | Rₜ = 4.33 min |
| 67 | 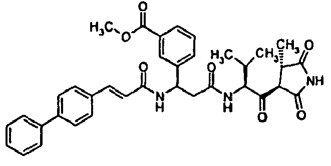 | 623.70 | MS (ESI+), | HPLC |
| | | | m/z: 624 | (Methode 17): |
| | | | (M+H)⁺ | Rₜ = 3.92 min |
| 68 | 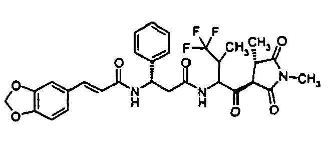 | 601.58 | MS (ESI+), | HPLC |
| | | | m/z: 602 | (Methode 17): |
| | | | (M+H)⁺ | Rₜ = 3.68 min |
| 69 | 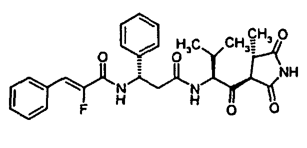 | 507.56 | MS (ESI+), | HPLC |
| | | | m/z: 508 | (Methode 17): |
| | | | (M+H)⁺ | Rₜ=3.64min |
| 70 | 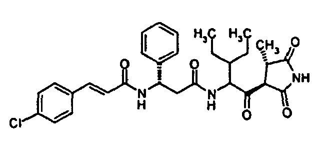 | 552.07 | MS (ESI+), | HPLC |
| | | | m/z: 552 | (Methode 18): |
| | | | (M+H)⁺ | Rₜ=3.84min |
| 71 | 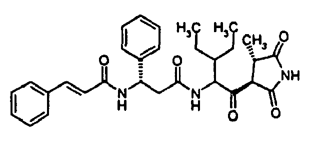 | 517.62 | MS (ESI+), | HPLC |
| | | | m/z: 518 | (Methode 18): |
| | | | (M+H)⁺ | Rₜ = 3.74 min |
| 72 | 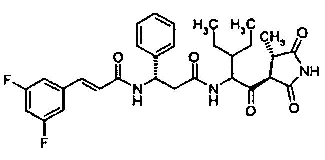 | 553.60 | MS (ESI+), | HPLC |
| | | | m/z: 554 | (Methode 18): |
| | | | (M+H)⁺ | Rₜ = 3.83 min |
| 73 | 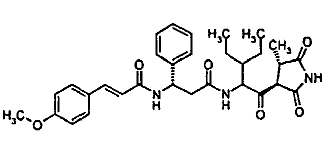 | 547.65 | MS (ESI+), | HPLC |
| | | | m/z: 548 | (Methode 18): |
| | | | (M+H)⁺ | Rₜ = 3.75 min |
| 74 | 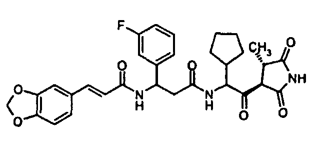 | 577.61 | MS (ESI+), | HPLC |
| | | | m/z: 578 | (Methode 18): |
| | | | (M+H)⁺ | Rₜ = 3.45 min |
| 75 | 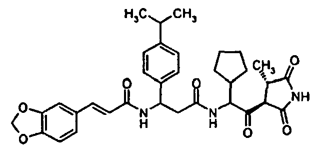 | 601.70 | MS (ESI+), | HPLC |
| | | | m/z: 602 | (Methode 18): |
| | | | (M+H)⁺ | Rₜ = 3.62 min |
| 76 | 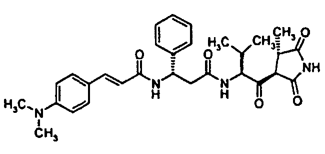 | 532.64 | MS (ESI+), | HPLC |
| | | | m/z:533 | (Methode17): |
| | | | (M+H)⁺ | Rₜ = 3.45 min |
| 77 | 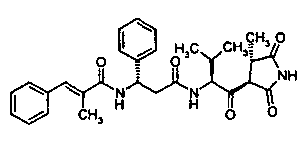 | 503.60 | MS (ESI+), | HPLC |
| | | | m/z: 504 | (Methode 17): |
| | | | (M+H)⁺ | Rₜ = 3.57 min |
| 78 | 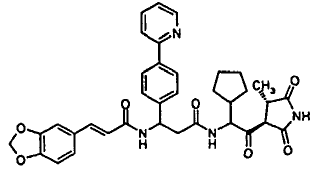 | 636.70 | MS (ESI+), | HPLC |
| | | | m/z: 637 | (Methode 17): |
| | | | (M+H)⁺ | Rₜ=3.27 min |
| 79 | 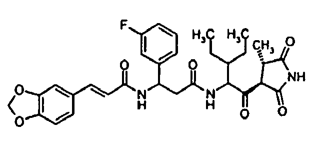 | 579.62 | MS (ESI+), | HPLC |
| | | | m/z: 580 | (Methode 17): |
| | | | (M+H)⁺ | Rₜ = 3.72 min |
| 80 | 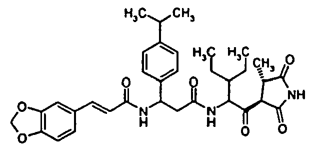 | 603.72 | MS (ESI+), | HPLC |
| | | | m/z: 604 | (Methode 17): |
| | | | (M+H)⁺ | Rₜ = 4.09 min |
| 81 | 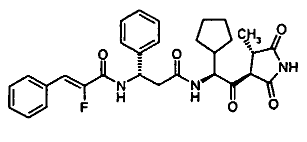 | 533.60 | MS (ESI+), | HPLC |
| | | | m/z: 534 | (Methode 22): |
| | | | (M+H)⁺ | Rₜ = 4.10 min |
| 82 | 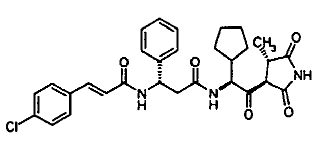 | 550.05 | MS (ESI+), | HPLC |
| | | | m/z: 550 | (Methode 19): |
| | | | (M+H)⁺ | Rₜ = 4.22 min |
| 83 | 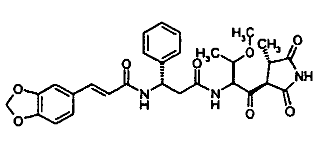 | 549.58 | MS (ESI+), | HPLC |
| | | | m/z: 550 | (Methode 19): |
| | | | (M+H)⁺ | Rₜ = 4.02 min |
| 84 | 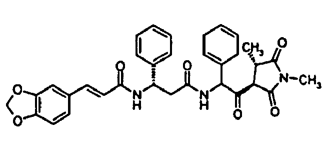 | 583.64 | MS (ESI+), | HPLC |
| | | | m/z: 584 | (Methode 18): |
| | | | (M+H)⁺ | Rₜ = 3.70 min |
| 85 | 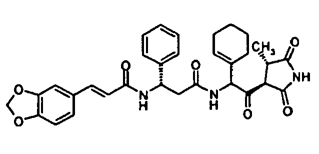 | 571.63 | MS (ESI+), | HPLC |
| | | | m/z: 572 | (Methode 20): |
| | | | (M+H)⁺ | Rₜ = 3.59 min |
| 86 | 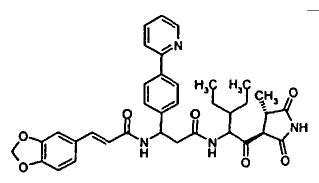 | 638.72 | MS (ESI+), | HPLC |
| | | | m/z: 639 | (Methode 20): |
| | | | (M+H)⁺ | Rₜ = 3.39 min |
| 87 | 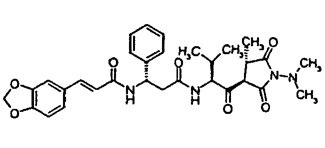 | 576.65 | MS (ESI+), | HPLC |
| | | | m/z: 577 | (Methode 20): |
| | | | (M+H)⁺ | Rₜ=3.60min |
| 88 | 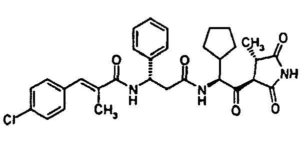 | 564.08 | MS (ESI+), | HPLC |
| | | | m/z: 564 | (Methode 18): |
| | | | (M+H)⁺ | Rₜ = 3.90 min |
| 89 | 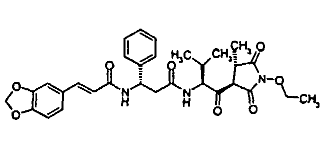 | 577.63 | MS (ESI+), | HPLC |
| | | | m/z: 578 | (Methode 20): |
| | | | (M+H)⁺ | Rₜ = 3.73 min |
| 90 | 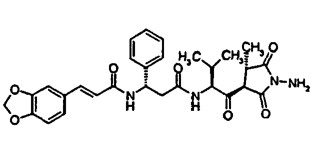 | 548.59 | MS (ESI+), | HPLC |
| | | | m/z: 549 | (Methode 21): |
| | | | (M+H)⁺ | Rₜ = 2.96 min |
| 91 | 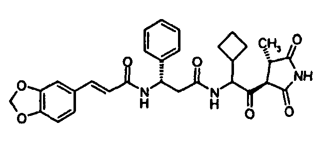 | 545.59 | MS (ESI+), | HPLC |
| | | | m/z: 546 | (Methode 20): |
| | | | (M+H)⁺ | Rₜ = 3.37 min |
| 92 | 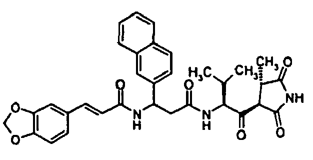 | 583.64 | MS (ESI+), | HPLC |
| | | | m/z: 584 | (Methode 19): |
| | | | (M+H)⁺ | Rₜ = 4.25 min |
| 93 | 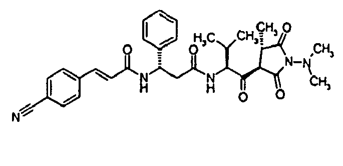 | 557.65 | MS (ESI+), | HPLC |
| | | | m/z: 558 | (Methode 19): |
| | | | (M+H)⁺ | Rₜ = 4.08 min |
| 94 | 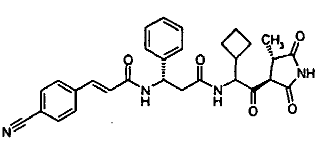 | 526.59 | MS (ESI+), | HPLC |
| | | | m/z: 527 | (Methode 20): |
| | | | (M+H)⁺ | Rₜ = 3.48 min |
| 95 | 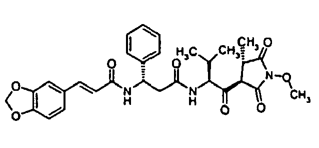 | 563.60 | MS (ESI+), | HPLC |
| | | | m/z: 564 | (Methode 20): |
| | | | (M+H)⁺ | Rₜ = 3.46 min |
| 96 | 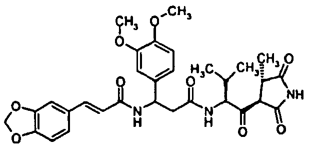 | 593.63 | MS (ESI+), | HPLC |
| | | | m/z: 594 | (Methode 20): |
| | | | (M+H)⁺ | Rₜ = 3.16 min |
| 97 | 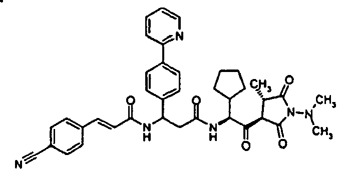 | 660.77 | MS (ESI+), | HPLC |
| | | | m/z: 661 | (Methode 21): |
| | | | (M+H)⁺ | Rₜ=3.10min |
| 98 | 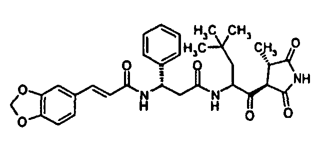 | 561.63 | MS (ESI+), | HPLC |
| | | | m/z: 562 | (Methode 19): |
| | | | (M+H)⁺ | Rₜ = 4.19min |
| 99 | 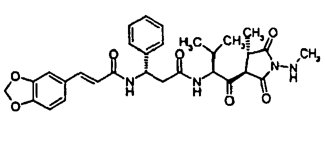 | 562.62 | MS (ESI+), | HPLC |
| | | | m/z: 563 | (Methode 19): |
| | | | (M+H)⁺ | Rₜ = 4.14 min |
| 100 | 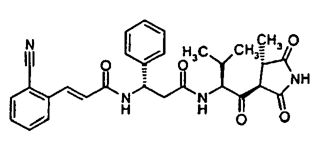 | 514.58 | MS (ESI+), | HPLC |
| | | | m/z: 515 | (Methode 19): |
| | | | (M+H)⁺ | Rₜ = 4.00 min |
| 101 | 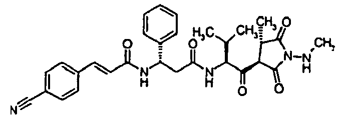 | 543.62 | MS (ESI+), | HPLC |
| | | | m/z: 544 | (Methode 21): |
| | | | (M+H)⁺ | Rₜ = 3.09 min |
| 102 | 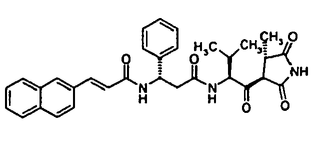 | 539.63 | MS (ESI+), | HPLC |
| | | | m/z: 540 | (Methode 19): |
| | | | (M+H)⁺ | Rₜ=4.12min |
| 103 | 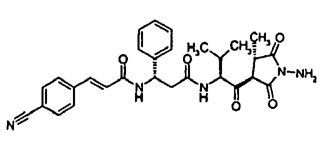 | 529.59 | MS (ESI+), | HPLC |
| | | | m/z: 530 | (Methode 18): |
| | | | (M+H)⁺ | Rₜ = 3.17 min |
| 104 | 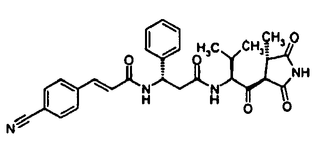 | 514.58 | MS (ESI+), | HPLC |
| | | | m/z: 515 | (Methode 18): |
| | | | (M+H)⁺ | Rₜ = 3.30 min |
| 105 | 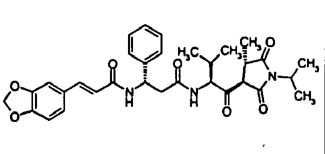 | 575.66 | MS (ESI+), | HPLC |
| | | | m/z: 576 | (Methode 21): |
| | | | (M+H)⁺ | Rₜ = 3.52 min |
| 106 | 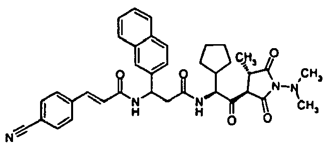 | 633.75 | MS (ESI+), | HPLC |
| | | | m/z: 634 | (Methode 20): |
| | | | (M+H)⁺ | Rₜ = 3.33 min |
| 107 | 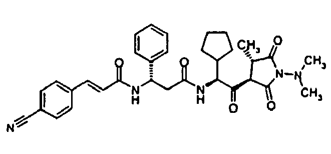 | 583.69 | MS (ESI+), | HPLC |
| | | | m/z: 584 | (Methode 18): |
| | | | (M+H)⁺ | Rₜ = 3.58 min |
| 108 | 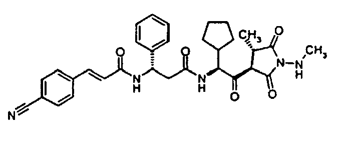 | 569.66 | MS (ESI+), | HPLC |
| | | | m/z: 570 | (Methode 19): |
| | | | (M+H)⁺ | Rₜ = 2.76 min |
| 109 | 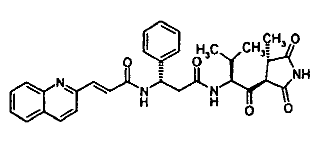 | 540.62 | MS (ESI+), | HPLC |
| | | | m/z: 541 | (Methode 20): |
| | | | (M+H)⁺ | Rₜ=3.19min |
| 110 | 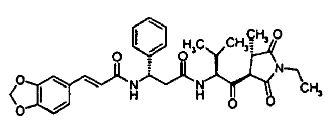 | 561.63 | MS (ESI+), | HPLC |
| | | | m/z: 562 | (Methode 18): |
| | | | (M+H)⁺ | Rₜ = 3.58 min |
| 111 | 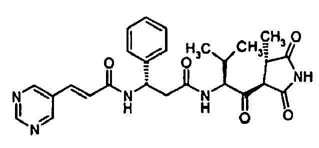 | 491.55 | MS (ESI+), | HPLC |
| | | | m/z: 492 | (Methode 20): |
| | | | (M+H)⁺ | Rₜ = 2.68 min |
| 112 | 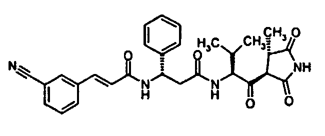 | 514.58 | MS (ESI+), | HPLC |
| | | | m/z: 515 | (Methode 20): |
| | | | (M+H)⁺ | Rₜ = 3.25 min |
| 113 | 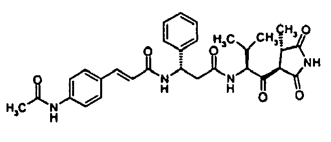 | 546.62 | MS (ESI+), | HPLC |
| | | | m/z: 547 | (Methode 20): |
| | | | (M+H)⁺ | Rₜ = 2.86 min |
| 114 | 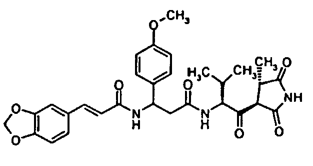 | 563.60 | MS (ESI+), | HPLC |
| | | | m/z: 564 | (Methode 20): |
| | | | (M+H)⁺ | Rₜ = 3.28 min |
| 115 | 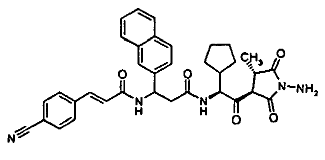 | 605.69 | MS (ESI+), | HPLC |
| | | | m/z: 606 | (Methode 19): |
| | | | (M+H)⁺ | Rₜ = 2.85 min |
| 116 | 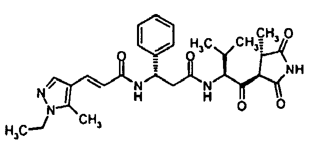 | 521.62 | MS (ESI+), | HPLC |
| | | | m/z: 522 | (Methode 20): |
| | | | (M+H)⁺ | Rₜ = 2.59 min |
| 117 | 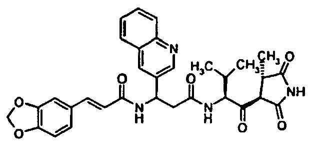 | 584.63 | MS (ESI+), | HPLC |
| | | | m/z: 585 | (Methode 21): |
| | | | (M+H)⁺ | Rₜ = 2.81 min |
| 118 | 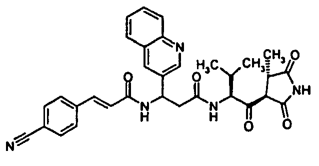 | 565.63 | MS (ESI+), | HPLC |
| | | | m/z: 566 | (Methode 19): |
| | | | (M+H)⁺ | Rₜ = 2.29 min |
| 119 | 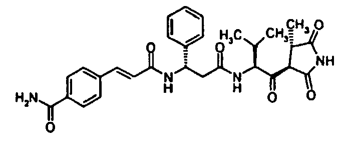 | 532.59 | MS (ESI+), | HPLC |
| | | | m/z: 533 | (Methode 21): |
| | | | (M+H)⁺ | Rₜ = 2.58 min |
| 120 | 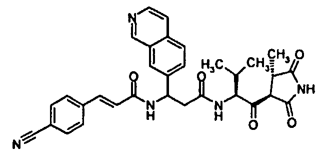 | 565.63 | MS (ESI+), | HPLC |
| | | | m/z: 566 | (Methode 19): |
| | | | (M+H)⁺ | Rₜ=2.12min |

### Beispiel 1121

### 4-(1-((2E)-3-(2H-Benzo[3,4-d]1,3-dioxolen-5-yl)prop-2-enoylamino)-2-{N-[(1S)-2-((4S,3R)-4-methyl-2,5-dioxoazolidin-3-yl)-1-(methylethyl)-2-oxoethyl]carbamoyl}-ethyl)benzoesäure

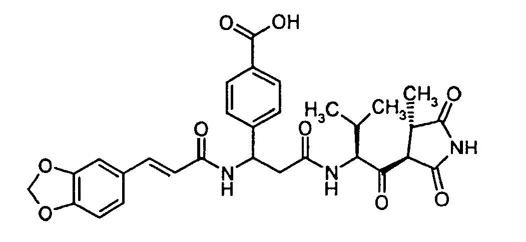

Der Benzoesäuremethylester Beispiel 53 (240 mg, 0.41 mmol) wird in 10 ml eines Dioxan/Wasser (1/1) Gemisches gelöst und mit 50 mg (0.89 mol) Kaliumhydroxid versetzt. Nach 2 h bei Raumtemperatur werden nochmals 25 mg Kaliumhydroxid zugegeben, und es wird weitere 3 h gerührt. Das Reaktionsgemisch wird eingeengt, der Rückstand in Wasser aufgenommen und mit 1 N Salzsäure angesäuert. Das Produkt fällt dabei kristallin aus und wird abfiltriert. Es werden 96 mg Produkt erhalten.
MS (ESI+): m/z = 578 (M+H⁺).
HPLC (Methode 14): Rₜ = 3.68 min.

### Allgemeine Vorschrift M: Darstellung von Benzeosäureestern

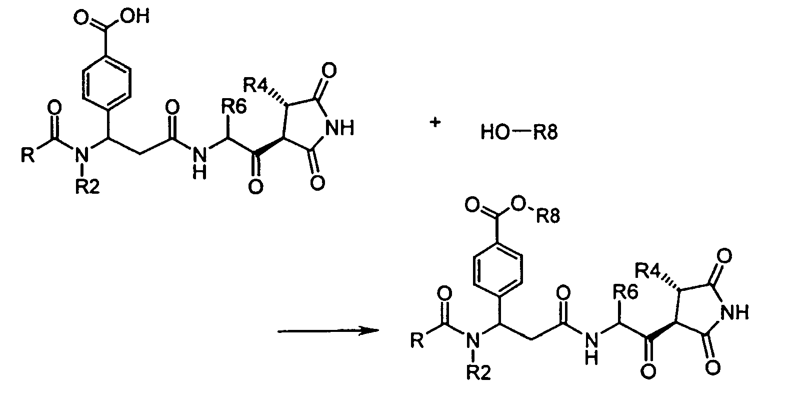

Das Benzoesäurederivat Beispiel 121 wird in Dichlormethan gelöst und mit 2 bis 3 eq. des entsprechenden Alkohols versetzt. Alternativ kann auch der Alkohol als Lösungsmittel verwendet werden. Zu der Lösung werden 2.2 eq. 4-Dimethylaminopyridin und 1.1 eq. EDC gegeben. Das Reaktionsgemisch wird über Nacht bei Raumtemperatur gerührt und eingeengt. Aus dem Rückstand kann durch Behandeln mit Dichlormethan und Diethylether das Produkt kristallisiert werden. Weitere Reinigung des Produktes erfolgt durch Chromatographie an Kieselgel mit Dichlormethan/Methanol Gemischen.

Nach der Allgemeinen Vorschrift M können folgende Verbindungen (Beispiel 122 und 123) erhalten werden:

| **Beispiel** | **Struktur** | **MW** | **MS** | **HPLC** |
|---|---|---|---|---|
| 122 | 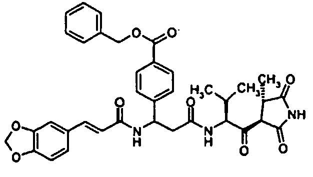 | 667.71 | MS (ESI+), | HPLC |
| | | | m/z: 668 | (Methode 14): |
| | | | (M+H)⁺ | Rₜ = 4.52 min |
| 123 | 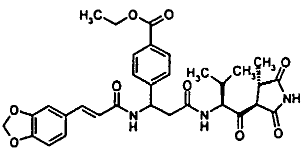 | 605.64 | MS (ESI+), | HPLC |
| | | | m/z: 606 | (Methode 14): |
| | | | (M+H)⁺ | Rₜ = 4.22 min |

### Allgemeine Vorschrift N: Acylierung von N-Amino-pyrrolidindion-Derivaten

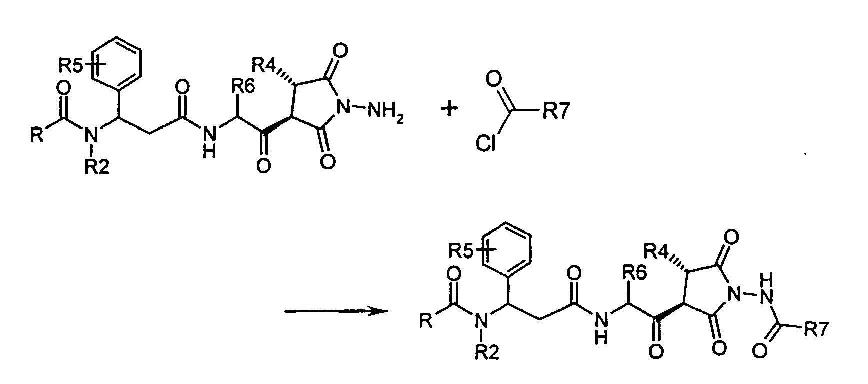

Das N-Amino-pyrrolidindion-Derivat wird in Pyridin gelöst (ca. 0.1 mol/l) und mit 1.1 eq des entsprechenden Carbonsäurechlorids versetzt. Das Reaktionsgemisch wird über Nacht bei Raumtemperatur gerührt und unter reduziertem Druck eingeengt. Der Rückstand wird mit Wasser und Dichlormethan versetzt und über Extrelut filtriert. Die organische Phase wird eingeengt und das Rohprodukt durch Chromatographie an Kieselgel mit Dichlormethan/Ethanol Gemischen gereinigt.

Nach der Allgemeinen Vorschrift N können folgende Verbindungen (Beispiel 124 bis 126) erhalten werden:

| **Beispiel** | **Struktur** | **MW** | **MS** | **HPLC** |
|---|---|---|---|---|
| 124 | 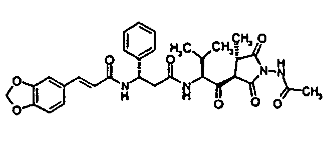 | 590.63 | MS (ESI+), | HPLC |
| | | | m/z: 591 | (Methode 19): |
| | | | (M+H)⁺ | Rₜ = 2.59 min |
| 125 | 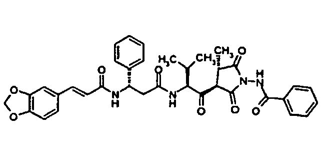 | 652.70 | MS (ESI+), | HPLC |
| | | | m/z: 653 | (Methode 21): |
| | | | (M+H)⁺ | Rₜ=3.30min |
| 126 | 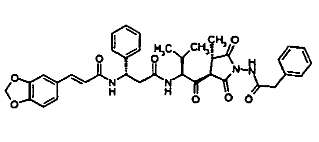 | 666.73 | MS (ESI+), | HPLC |
| | | | m/z: 667 | (Methode 19): |
| | | | (M+H)⁺ | Rₜ = 2.78 min |

### B) Bewertung der physiologischen Wirksamkeit

Die Eignung der erfindungsgemäßen Verbindungen zur Behandlung bakterieller Erkrankungen kann in folgenden Tiermodellen gezeigt werden:

### Bestimmung der Minimalen Hemmkonzentration (MHK):

Die MHK wird im Flüssigdilutionstest bestimmt. Übernachtkulturen der Testkeime werden auf eine Zellzahl von 10⁵ Keimen pro ml in Isosensitest-Medium (Fa. Difco, Irvine, USA) verdünnt und mit Verdünnungen der Testsubstanzen (Verdünnungsstufen 1:2) inkubiert. Ausnahmen sind die Tests mit S. pneumoniae G9A, die in BHI-Bouillon (Fa. Difco) plus 20 % Rinderserum, und mit H. influenzae, die in BHI-Bouillon (Fa. Difco) plus 20 % Rinderserum, 10 µg/ml Haemin und 1 % Isovitale (Fa. Becton Dickinson, New Jersey, USA) durchgeführt werden.
Die Kulturen werden bei 37°C für 18-24 Stunden inkubiert; S. pneumoniae und H. influenzae in Gegenwart von 8 -10 % CO₂.

### Ergebnisse:

Die jeweils niedrigste Substanzkonzentration, bei der kein sichtbares Bakterienwachstum mehr auftritt, wird als MHK definiert. Die MHK-Werte in µmol/l einiger erfindungsgemäßer Verbindungen gegenüber einer Reihe von Testkeimen sind in der nachstehenden Tabelle beispielhaft aufgeführt.

| **Bsp. Nr.** | **Staphylokokkus aureus 133** | **Haemophilus influenzae Spain 7** |
|---|---|---|
| 1 | 3.9 | 31.3 |
| 2 | 7.8 | 31.3 |
| 5 | 7.8 | 3.9 |
| 11 | 7.8 | 62.5 |
| 31 | <1 | 31.3 |
| 34 | <1 | 7.8 |
| 92 | <1 | 15.6 |

### Systemische Infektion mit S. aureus 133

S. aureus 133 Zellen werden über Nacht in BH-Bouillon (Fa. Oxoid, New York, USA) angezüchtet. Die Übernachtkultur wird 1:100 in frische BH-Bouillon verdünnt und für 3 Stunden hochgedreht. Die in der logarithmischen Wachstumsphase befindlichen Bakterien werden abzentrifugiert und 2 x mit gepufferter, physiologischer Kochsalzlösung gewaschen. Danach wird am Photometer (Modell LP 2W, Fa. Dr. Lange, Berlin, Deutschland) eine Zellsuspension in Kochsalzlösung mit einer Extinktion von 50 Einheiten eingestellt. Nach einem Verdünnungsschritt (1:15) wird diese Suspension 1:1 mit einer 10 % igen Mucinsuspension gemischt. Von dieser Infektionslösung wird 0,25 ml/20 g Maus i.p. appliziert. Dies entspricht einer Zellzahl von etwa 1 x 10E⁶ Keimen/Maus. Die i.p.- oder i.v.Therapie erfolgt 30 Minuten nach der Infektion. Für den Infektionsversuch werden weibliche CFW1-Mäuse verwendet. Das Überleben der Tiere wird über 6 Tage protokolliert.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.
Tablettengewicht 212 mg. Durchmesser 8 mm, Wölbungsradius 12 mm.

### Herstellung:

Die Mischung aus der Verbindung des Beispiels 1, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96 %), 400 mg Rhodigel (Xanthan gum) (Fa. FMC, USA) und 99 g Wasser.
Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

100-200 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0,22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 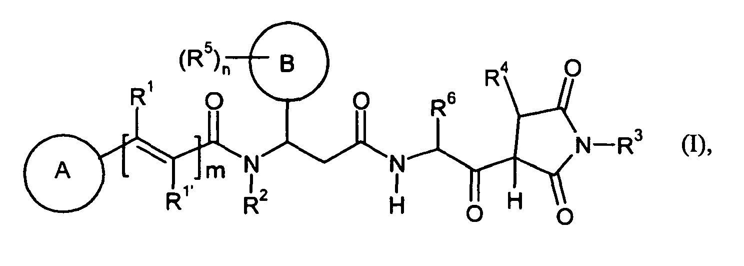 worin
R¹ Wasserstoff, Methyl oder Halogen bedeutet,
R^{1'} Wasserstoff, Methyl oder Halogen bedeutet,
R² Wasserstoff oder Methyl bedeutet,
R³ Wasserstoff, Hydroxy, Amino, C₁-C₃-Alkyl, C₁-C₃-Alkoxy, Benzyloxy, C₁-C₃-Alkylamino, C₁-C₃-Alkylcarbonylamino, Phenylcarbonylamino oder Benzylcarbonylamino bedeutet,
R⁴ Wasserstoff oder C₁-C₃-Alkyl bedeutet,
R⁵ Halogen, Trifluormethyl, Trifluormethoxy, Nitro, Amino, C₁-C₆-Alkylamino, Hydroxy, C₁-C₆-Alkyl, C₁-C₆-Alkoxy, Carboxyl, C₁-C₆-Alkoxycarbonyl, Benzyloxycarbonyl, Aminocarbonyl, C₁-C₆-Alkylaminocarbonyl, C₆-C₁₄-Aryl oder 5- bis 10-gliedriges Heteroaryl bedeutet,
oder
zwei Substituenten R⁵ zusammen mit den Kohlenstoffatomen, an die sie gebunden sind, ein 3- bis 8-gliedriges Cycloalkyl oder 4- bis 10-gliedriges Heterocyclyl bilden, wobei dieses Cycloalkyl oder Heterocyclyl substituiert sein kann mit 0, 1 oder 2 Substituenten R⁵⁻¹, wobei die Substituenten R⁵⁻¹ unabhängig voneinander ausgewählt werden aus der Gruppe bestehend aus Halogen, Nitro, Amino, Trifluormethyl, Hydroxy und C₁-C₆-Alkoxy,
R⁶ C₁-C₆-Alkyl, C₃-C₈-Cycloalkyl oder C₃-C₈-Cycloalkenyl bedeutet,
wobei R⁶ substituiert sein kann mit 0, 1, 2 oder 3 Substituenten R⁶⁻¹, wobei die Substituenten R⁶⁻¹ unabhängig voneinander ausgewählt werden aus der Gruppe bestehend aus Halogen, Nitro, Amino, Trifluormethyl, Hydroxy, C₁-C₆-Alkyl und C₁-C₆-Alkoxy,
n eine Zahl 0, 1, 2 oder 3 bedeutet,
wobei bei n gleich 2 oder 3 die Reste R⁵ gleich oder verschieden sein können,
m eine Zahl 1, 2 oder 3 bedeutet,
A C₆-C₁₄-Aryl oder 5- bis 10-gliedriges Heteroaryl bedeutet,
wobei A substituiert sein kann mit 0, 1, 2 oder 3 Substituenten R^{A}, wobei die Substituenten R^{A} unabhängig voneinander ausgewählt werden aus der Gruppe bestehend aus Halogen, C₁-C₆-Alkyl, Nitro, Amino, Cyano, Trifluormethyl, C₆-C₁₄-Aryl, 5- bis 10-gliedriges Heteroaryl, Hydroxy, C₁-C₆-Alkoxy, C₁-C₆-Alkylamino, Carboxyl, C₁-C₆-Alkoxycarbonyl, Aminocarbonyl, C₁-C₆-Alkylcarbonylamino und C₁-C₆-Alkylaminocarbonyl,
oder
zwei Substituenten R^{A} zusammen mit den Kohlenstoffatomen, an die sie gebunden sind, ein 3- bis 8-gliedriges Cycloalkyl oder 4- bis 10-gliedriges Heterocyclyl bilden, wobei dieses Cycloalkyl oder Heterocyclyl substituiert sein kann mit 0, 1 oder 2 Substituenten R^{A-1}, wobei die Substituenten R^{A-1} unabhängig voneinander ausgewählt werden aus der Gruppe bestehend aus Halogen, Nitro, Amino, Trifluormethyl, Hydroxy und C₁-C₆-Alkoxy,
B C₆-C₁₄-Aryl oder 5- bis 10-gliedriges Heteroaryl bedeutet,
sowie deren pharmazeutisch verträgliche Salze, Solvate und Hydrate.

2. Verbindung nach Anspruch 1, **dadurch gekennzeichnet, dass**
R¹ Wasserstoff oder Methyl bedeutet,
R¹' Wasserstoff, Methyl oder Fluor bedeutet,
R² Wasserstoff bedeutet,
R³ Wasserstoff, Amino, C₁-C₃-Alkyl, C₁-C₃-Alkoxy, Benzyloxy, C₁-C₃-Alkylamino, C₁-C₃-Alkylcarbonylamino, Phenylcarbonylamino oder Benzylcarbonylamino bedeutet,
R⁴ Methyl bedeutet,
R⁵ Fluor, Chlor, Trifluormethyl, Trifluormethoxy, Nitro, Amino, C₁-C₆-Alkylamino, Hydroxy, C₁-C₆-Alkyl, C₁-C₆-Alkoxy, C₁-C₆-Alkoxycarbonyl, Aminocarbonyl, Phenyl oder 5- bis 6- gliedriges Heteroaryl bedeutet,
oder
zwei Substituenten R⁵ zusammen mit den Kohlenstoffatomen, an die sie gebunden sind, ein 5- bis 6-gliedriges Cycloalkyl oder 5- bis 6-gliedriges Heterocyclyl bilden,
R⁶ C₂-C₇-Alkyl, C₃-C₇-Cycloalkyl oder C₅-C₇-Cycloalkenyl bedeutet,
wobei R⁶ substituiert sein kann mit 0, 1 oder 2 Substituent R⁶⁻¹, wobei R⁶⁻¹ ausgewählt wird aus der Gruppe bestehend aus Halogen, Trifluormethyl, C₁-C₆-Alkyl und Methoxy,
n eine Zahl 0, 1 oder 2 bedeutet,
wobei bei n gleich 2 die Reste R⁵ gleich oder verschieden sein können,
m eine Zahl 1 oder 2 bedeutet,
A Phenyl, Naphthyl oder 5-, 6- oder 10-gliedriges Heteroaryl bedeutet,
wobei A substituiert sein kann mit 0, 1 oder 2 Substituenten R^{A}, wobei die Substituenten R^{A} unabhängig voneinander ausgewählt werden aus der Gruppe bestehend aus Halogen, C₁-C₆-Alkyl, Amino, Cyano, Trifluormethyl, C₆-C₁₄-Aryl, 5- bis 10-gliedriges Heteroaryl, Hydroxy, C₁-C₆-Alkoxy, C₁-C₆-Alkylamino, C₁-C₆-Alkoxycarbonyl und Aminocarbonyl,
oder
zwei Substituenten R^{A} zusammen mit den Kohlenstoffatomen, an die sie gebunden sind, ein 5- bis 6-gliedriges Cycloalkyl oder 5- bis 6-gliedriges Heterocyclyl bilden, wobei dieses Cycloalkyl oder Heterocyclyl substituiert sein kann mit 0 oder 1 Substituenten R^{A-1}, wobei die Substituenten R^{A-1} unabhängig voneinander ausgewählt werden aus der Gruppe bestehend aus Halogen, Nitro, Amino, Trifluormethyl, Hydroxy und C₁-C₆-Alkoxy,
B Phenyl, Naphthyl oder 5-, 6-, 9- oder 10-gliedriges Heteroaryl bedeutet.

3. Verbindung nach einem der Ansprüche 1 oder 2, **dadurch gekennzeichnet, dass** sie Formel (Ia) entsprechen, 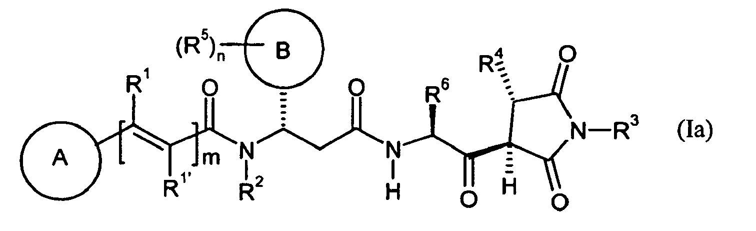 worin
R¹ Wasserstoff bedeutet,
R^{1'} Wasserstoff, Methyl oder Fluor bedeutet,
R² Wasserstoff bedeutet,
R³ Wasserstoff, Amino, Methyl, Methoxy, Ethoxy, Methylamino oder Dimethylamino bedeutet,
R⁴ Methyl bedeutet,
R⁵ Fluor, Chlor, Trifluormethyl, C₁-C₆-Alkoxy, Methoxycarbonyl, C₁-C₄-Alkyl, Phenyl oder Pyridyl bedeutet,
oder
zwei Substituenten R⁵ zusammen mit den Kohlenstoffatomen, an die sie gebunden sind, ein 5- oder 6-gliedriges Heterocyclyl bilden,
R⁶ C₃-C₆-Alkyl, C₄-C₆-Cycloalkyl oder C₅-C₆-Cycloalkenyl bedeutet,
n eine Zahl 0, 1 oder 2 bedeutet,
wobei bei n gleich 2 die Reste R⁵ gleich oder verschieden sein können,
m die Zahl 1 bedeutet,
A Phenyl, Pyridyl, Imidazolyl, Thienyl, Furanyl, Oxadiazolyl, Pyrazolyl, Pyrazinyl, Pyridazinyl, Pyrimidinyl, Chinolinyl oder Isochinolinyl bedeutet,
wobei A substituiert sein kann mit 0, 1 oder 2 Substituenten R^{A}, wobei die Substituenten R^{A} unabhängig voneinander ausgewählt werden aus der Gruppe bestehend aus Halogen, C₁-C₆-Alkyl, Cyano, Trifluormethyl, Phenyl und C₁-C₆-Alkoxy,
oder
zwei Substituenten R^{A} zusammen mit den Kohlenstoffatomen, an die sie gebunden sind, ein 5- oder 6-gliedriges Heterocyclyl bilden,
B Phenyl, Naphthyl, Pyridyl, Thienyl, Furanyl, Chinolinyl oder Isochinolinyl bedeutet.

4. Verbindung nach Anspruch 3, **dadurch gekennzeichnet, dass**
R¹ Wasserstoff bedeutet,
R^{1'} Wasserstoff bedeutet,
R² Wasserstoff bedeutet,
R³ Wasserstoff, Amino, Methylamino oder Dimethylamino bedeutet,
R⁴ Methyl bedeutet,
R⁵ Fluor, Chlor, Trifluormethyl, Methoxy, C₁-C₄-Alkyl, Phenyl oder Pyridyl bedeutet,
oder
zwei Substituenten R⁵ zusammen mit dem Phenylring, an den sie gebunden sind, ein 1,3-Benzodioxol oder ein 1,4-Benzodioxan bilden,
R⁶ Isopropyl, tert-Butyl, Isobutyl, Isopentyl, Cyclobutyl oder Cyclopentyl bedeutet,
n eine Zahl 0, 1 oder 2 bedeutet,
wobei bei n gleich 2 die Reste R⁵ gleich oder verschieden sein können,
m die Zahl 1 bedeutet,
A Phenyl, Pyridyl, Thienyl, Chinolinyl oder Isochinolinyl bedeutet,
wobei A substituiert sein kann mit 0, 1 oder 2 Substituenten R^{A}, wobei die Substituenten R^{A} unabhängig voneinander ausgewählt werden aus der Gruppe bestehend aus Fluor, Chlor, C₁-C₃-Alkyl, Cyano, Trifluormethyl, Phenyl und C₁-C₃-Alkoxy,
oder
zwei Substituenten R^{A} zusammen mit dem Phenylring, an den sie gebunden sind, ein 1,3-Benzodioxol oder ein 1,4-Benzodioxan bilden,
B Phenyl, Naphthyl, Thienyl, Chinolinyl oder Isochinolinyl bedeutet.

5. Verbindung nach einem der Ansprüche 1 oder 2, **dadurch gekennzeichnet, dass** R¹ Wasserstoff bedeutet.

6. Verbindung nach einem der Ansprüche 1, 2, 3 oder 5, **dadurch gekennzeichnet, dass** R^{1'} Wasserstoff bedeutet.

7. Verbindung nach einem der Ansprüche 1, 5 oder 6, **dadurch gekennzeichnet, dass** R² Wasserstoff bedeutet.

8. Verbindung nach einem der Ansprüche 1, 5, 6 oder 7, **dadurch gekennzeichnet, dass** R⁴ Methyl bedeutet.

9. Verbindung nach einem der Ansprüche 1, 2, 5, 6 oder 7, **dadurch gekennzeichnet, dass** m die Zahl 1 bedeutet.

10. Verbindung nach einem der vorstehenden Ansprüche, **dadurch gekennzeichnet, dass** R³ Wasserstoff oder Amino bedeutet.

11. Verbindung nach einem der vorstehenden Ansprüche, **dadurch gekennzeichnet, dass** n die Zahl Null bedeutet.

12. Verbindung nach einem der vorstehenden Ansprüche, **dadurch gekennzeichnet, dass** n die Zahl 1 bedeutet, B Phenyl bedeutet und R⁵ Fluor, Chlor, Trifluormethyl, C₁-C₆-Alkoxy, C₁-C₄-Alkyl, Phenyl oder Pyridyl bedeutet, wobei R⁵ in der meta- oder para-Position zur Verknüpfungsstelle des Phenyl-Ringes vorliegt.

13. Verbindung nach einem der vorstehenden Ansprüche, **dadurch gekennzeichnet, dass** R⁶ Isopropyl, tert-Butyl, Isobutyl, Isopentyl oder Cyclopentyl bedeutet.

14. Verbindung nach einem der vorstehenden Ansprüche, **dadurch gekennzeichnet, dass** A Phenyl oder Pyridyl bedeutet, wobei A substituiert sein kann mit 0, 1 oder 2 Substituenten R^{A}, wobei die Substituenten R^{A} unabhängig voneinander ausgewählt werden aus der Gruppe bestehend aus Fluor, Chlor, Cyano, Trifluormethyl, Phenyl und Methoxy.

15. Verfahren zur Herstellung von Verbindungen nach Anspruch 1, **dadurch gekennzeichnet, dass**
nach Verfahren [A]
eine Verbindung der Formel 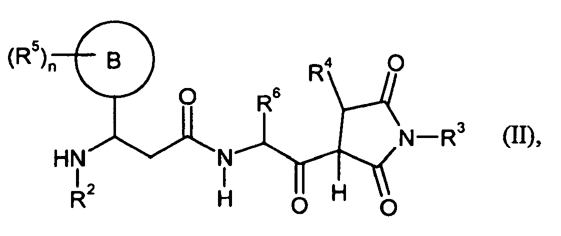 worin R² bis R⁶, B und n die in Anspruch 1 angegebene Bedeutung aufweisen,
mit einer Verbindung der Formel 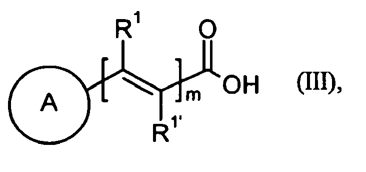 worin R¹ und R^{1'}, A und m die in Anspruch 1 angegebene Bedeutung aufweisen,
wobei diese gegebenenfalls in aktivierter Form vorliegen können, umgesetzt werden,
oder
nach Verfahren [B]
eine Verbindung der Formel 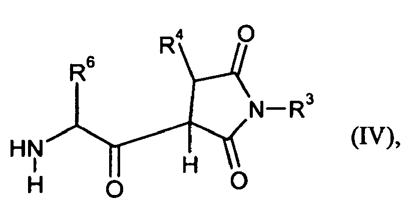 worin R³, R⁴ und R⁶ die in Anspruch 1 angegebene Bedeutung aufweisen, mit einer Verbindung der Formel 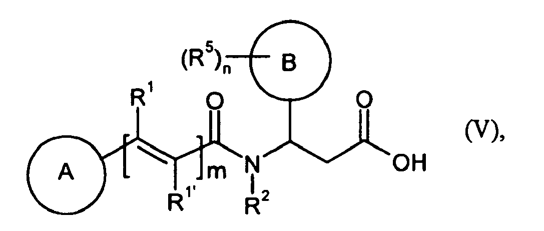 worin R¹, R^{1'}, R², R⁵, A, B, m und n die in Anspruch 1 angegebene Bedeutung aufweisen,
wobei diese gegebenenfalls in aktivierter Form vorliegen können, umgesetzt werden.

16. Verbindung nach einem der Ansprüche 1 bis 14 zur Behandlung und/oder Prophylaxe von Krankheiten.

17. Arzneimittel enthaltend mindestens eine Verbindung nach einem der Ansprüche 1 bis 14 in Kombination mit mindestens einem pharmazeutisch verträglichen, pharmazeutisch unbedenklichen Trägerstoff oder sonstigen Exzipienten.

18. Verwendung einer Verbindung nach einem der Ansprüche 1 bis 14 zur Herstellung eines Arzneimittels zur Behandlung und/oder Prophylaxe von bakteriellen Infektionen.

19. Arzneimittel nach Anspruch 17 zur Behandlung und/oder Prophylaxe von bakteriellen Infektionen.

## Claims

1. Compound of the formula 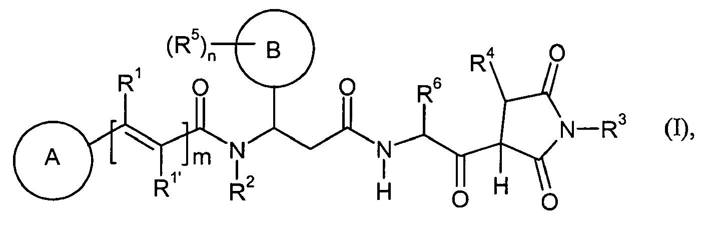 in which
R¹ is hydrogen, methyl or halogen,
R^{1'} is hydrogen, methyl or halogen,
R² is hydrogen or methyl,
R³ is hydrogen, hydroxyl, amino, C₁-C₃ alkyl, C₁-C₃ alkoxy, benzyloxy, C₁-C₃ alkylamino, C₁-C₃ alkylcarbonylamino, phenylcarbonylamino or benzylcarbonylamino,
R⁴ is hydrogen or C₁-C₃ alkyl,
R⁵ is halogen, trifluoromethyl, trifluoromethoxy, nitro, amino, C₁-C₆ alkylamino, hydroxyl, C₁-C₆ alkyl, C₁-C₆ alkoxy, carboxyl, C₁-C₆ alkoxycarbonyl, benzyloxycarbonyl, aminocarbonyl, C₁-C₆ alkylaminocarbonyl, C₆-C₁₄ aryl or 5- to 10-membered heteroaryl,
or
two substituents R⁵ together with the carbon atoms to which they are attached form a 3- to 8-membered cycloalkyl or 4- to 10-membered heterocyclyl, each of which may be substituted by 0, 1 or 2 substituents R⁵⁻¹, the substituents R⁵⁻¹ being selected independently of one another from the group consisting of halogen, nitro, amino, trifluoromethyl, hydroxyl and C₁-C₆ alkoxy,
R⁶ is C₁-C₆ alkyl, C₃-C₈ cycloalkyl or C₃-C₈ cycloalkenyl,
it being possible for R⁶ to be substituted by 0, 1, 2 or 3 substituents R⁶⁻¹, the substituents R⁶⁻¹ being selected independently of one another from the group consisting of halogen, nitro, amino, trifluoromethyl, hydroxyl, C₁-C₆ alkyl and C₁-C₆ alkoxy,
n is a number 0, 1, 2 or 3,
it being possible for the radicals R⁵ to be identical or different when n is 2 or 3,
m is a number 1, 2 or 3,
A is C₆-C₁₄ aryl or 5- to 10-membered heteroaryl,
it being possible for A to be substituted by 0, 1, 2 or 3 substituents R^{A}, the substituents R^{A} being selected independently of one another from the group consisting of halogen, C₁-C₆ alkyl, nitro, amino, cyano, trifluoromethyl, C₆-C₁₄ aryl, 5- to 10-membered heteroaryl, hydroxyl, C₁-C₆ alkoxy, C₁-C₆ alkylamino, carboxyl, C₁-C₆ alkoxycarbonyl, aminocarbonyl, C₁-C₆ alkylcarbonylamino and C₁-C₆ alkylaminocarbonyl,
or
two substituents R^{A} together with the carbon atoms to which they are attached form a 3- to 8-membered cycloalkyl or 4- to 10-membered heterocyclyl each of which may be substituted by 0, 1 or 2 substituents R^{A-1}, the substituents R^{A-1} being selected independently of one another from the group consisting of halogen, nitro, amino, trifluoromethyl, hydroxyl and C₁-C₆ alkoxy,
B is C₆-C₁₄ aryl or 5- to 10-membered heteroaryl,
and also its pharmaceutically compatible salts, solvates and hydrates.

2. Compound according to Claim 1, **characterized in that**
R¹ is hydrogen or methyl,
R^{1'} is hydrogen, methyl or fluorine,
R² is hydrogen,
R³ is hydrogen, amino, C₁-C₃ alkyl, C₁-C₃ alkoxy, benzyloxy, C₁-C₃ alkylamino, C₁-C₃ alkylcarbonylamino, phenylcarbonylamino or benzylcarbonylamino,
R⁴ is methyl,
R⁵ is fluorine, chlorine, trifluoromethyl, trifluoromethoxy, nitro, amino, C₁-C₆ alkylamino, hydroxyl, C₁-C₆ alkyl, C₁-C₆ alkoxy, C₁-C₆ alkoxycarbonyl, aminocarbonyl, phenyl or 5- to 6-membered heteroaryl,
or
two substituents R⁵ together with the carbon atoms to which they are attached form a 5- to 6-membered cycloalkyl or 5- to 6-membered heterocyclyl,
R⁶ is C₂-C₇ alkyl, C₃-C₇ cycloalkyl or C₅-C₇ cycloalkenyl,
it being possible for R⁶ to be substituted by 0, 1 or 2 substituent R⁶⁻¹, R⁶⁻¹ being selected from the group consisting of halogen, trifluoromethyl, C₁-C₆ alkyl and methoxy,
n is a number 0, 1 or 2,
it being possible for the radicals R⁵ to be identical or different if n is 2,
m is a number 1 or 2,
A is phenyl, naphthyl or 5-, 6- or 10-membered heteroaryl,
it being possible for A to be substituted by 0, 1 or 2 substituents R^{A}, the substituents R^{A} being selected independently of one another from the group consisting of halogen, C₁-C₆ alkyl, amino, cyano, trifluoromethyl, C₆-C₁₄ aryl, 5- to 10-membered heteroaryl, hydroxyl, C₁-C₆ alkoxy, C₁-C₆ alkylamino, C₁-C₆ alkoxycarbonyl and aminocarbonyl,
or
two substituents R^{A} together with the carbon atoms to which they are attached form a 5- to 6-membered cycloalkyl or 5- to 6-membered heterocyclyl each of which may be substituted by 0 or 1 substituents R^{A-1}, the substituents R^{A-1} being selected independently of one another from the group consisting of halogen, nitro, amino, trifluoromethyl, hydroxyl and C₁-C₆ alkoxy,
B is phenyl, naphthyl or 5-, 6-, 9- or 10-membered heteroaryl.

3. Compound according to one of Claims 1 or 2, **characterized in that** it conform to formula (Ia) 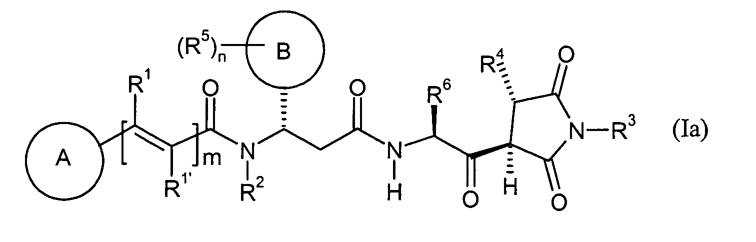 in which
R¹ is hydrogen,
R^{1'} is hydrogen, methyl or fluorine,
R² is hydrogen,
R³ is hydrogen, amino, methyl, methoxy, ethoxy, methylamino or dimethylamino,
R⁴ is methyl,
R⁵ is fluorine, chlorine, trifluoromethyl, C₁-C₆ alkoxy, methoxycarbonyl, C₁-C₄ alkyl, phenyl or pyridyl,
or
two substituents R⁵ together with the carbon atoms to which they are attached form a 5- or 6-membered heterocyclyl,
R⁶ is C₃-C₆ alkyl, C₄-C₆ cycloalkyl or C₅-C₆ cycloalkenyl,
n is a number 0, 1 or 2,
it being possible for the radicals R⁵ to be identical or different if n is 2,
m is the number 1,
A is phenyl, pyridyl, imidazolyl, thienyl, furanyl, oxadiazolyl, pyrazolyl, pyrazinyl, pyridazinyl, pyrimidinyl, quinolinyl or isoquinolinyl,
it being possible for A to be substituted by 0, 1 or 2 substituents R^{A}, the substituents R^{A} being selected independently of one another from the group consisting of halogen, C₁-C₆ alkyl, cyano, trifluoromethyl, phenyl and C₁-C₆ alkoxy,
or
two substituents R^{A} together with the carbon atoms to which they are attached form a 5- or 6-membered heterocyclyl,
B is phenyl, naphthyl, pyridyl, thienyl, furanyl, quinolinyl or isoquinolinyl.

4. Compound according to Claim 3, **characterized in that**
R¹ is hydrogen,
R^{1'} is hydrogen,
R² is hydrogen,
R³ is hydrogen, amino, methylamino or dimethylamino,
R⁴ is methyl,
R⁵ is fluorine, chlorine, trifluoromethyl, methoxy, C₁-C₄ alkyl, phenyl or pyridyl,
or
two substituents R⁵ together with the phenyl ring to which they are attached form a 1,3-benzodioxole or a 1,4-benzodioxane,
R⁶ is isopropyl, tert-butyl, isobutyl, isopentyl, cyclobutyl or cyclopentyl,
n is a number 0, 1 or 2,
it being possible for the radicals R⁵ to be identical or different if n is 2,
m is the number 1,
A is phenyl, pyridyl, thienyl, quinolinyl or isoquinolinyl,
it being possible for A to be substituted by 0, 1 or 2 substituents R^{A}, the substituents R^{A} being selected independently of one another from the group consisting of fluorine, chlorine, C₁-C₃ alkyl, cyano, trifluoromethyl, phenyl and C₁-C₃ alkoxy,
or
two substituents R^{A} together with the phenyl ring to which they are attached form a 1,3-benzodioxole or a 1,4-benzodioxane,
B is phenyl, naphthyl, thienyl, quinolinyl or isoquinolinyl.

5. Compound according to one of Claims 1 or 2, **characterized in that** R¹ is hydrogen.

6. Compound according to one of Claims 1, 2, 3 or 5, **characterized in that** R^{1'} is hydrogen.

7. Compound according to one of Claims 1, 5 or 6, **characterized in that** R² is hydrogen.

8. Compound according to one of Claims 1, 5, 6 or 7, **characterized in that** R⁴ is methyl.

9. Compound according to one of Claims 1, 2, 5, 6 or 7, **characterized in that** m is the number 1.

10. Compound according to one of the preceding claims, **characterized in that** R³ is hydrogen or amino.

11. Compound according to one of the preceding claims, **characterized in that** n is the number zero.

12. Compound according to one of the preceding claims, **characterized in that** n is the number 1, B is phenyl and R⁵ is fluorine, chlorine, trifluoromethyl, C₁-C₆ alkoxy, C₁-C₄ alkyl, phenyl or pyridyl, R⁵ being positioned meta or para to the linkage site of the phenyl ring.

13. Compound according to one of the preceding claims, **characterized in that** R⁶ is isopropyl, tert-butyl, isobutyl, isopentyl or cyclopentyl.

14. Compound according to one of the preceding claims, **characterized in that** A is phenyl or pyridyl, it being possible for A to be substituted by 0, 1 or 2 substituents R^{A}, the substituents R^{A} being selected independently of one another from the group consisting of fluorine, chlorine, cyano, trifluoromethyl, phenyl and methoxy.

15. Process for preparing compounds according to Claim 1, **characterized in that**
by process [A]
a compound of the formula 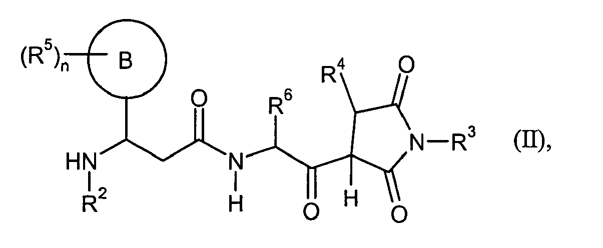 in which R² to R⁶, B and n are as defined in Claim 1,
are reacted with a compound of the formula 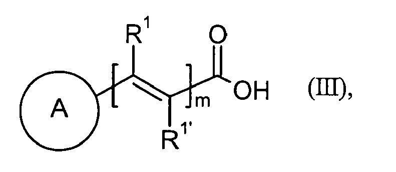 in which R¹ and R^{1'}, A and m are as defined in Claim 1,
it being possible for these to be in activated form if desired,
or
by process [B]
a compound of the formula 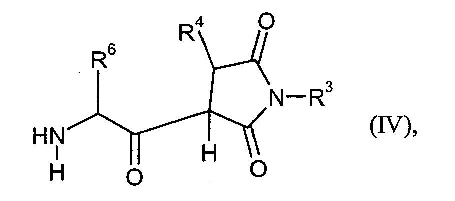 in which R³, R⁴ and R⁶ are as defined in Claim 1
are reacted with a compound of the formula 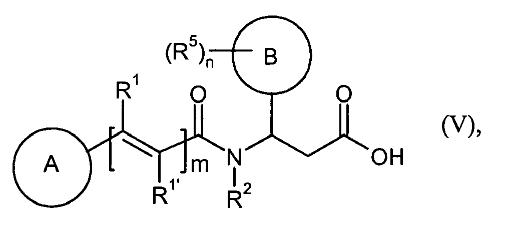 in which R¹, R^{1'}, R², R⁵, A, B, m and n are as defined in Claim 1,
it being possible for these to be in activated form if desired.

16. Compound according to one of Claims 1 to 14 for the treatment and/or prophylaxis of diseases.

17. Medicinal product comprising at least one compound according to one of Claims 1 to 14 in combination with at least one pharmaceutically compatible, pharmaceutically acceptable carrier or other excipients.

18. Use of a compound according to one of Claims 1 to 14 for producing a medicinal product for the treatment and/or prophylaxis of bacterial infections.

19. Medicinal product according to Claim 17 for the treatment and/or prophylaxis of bacterial infections.

## Revendications

1. Composé de formule 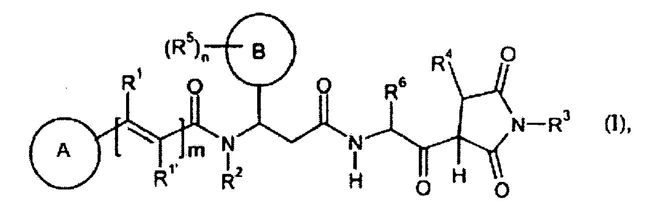 dans laquelle
R¹ représente l'hydrogène, un reste méthyle ou un halogène,
R^{1'} représente l'hydrogène, un reste méthyle ou un halogène,
R² représente l'hydrogène ou un reste méthyle,
R³ représente l'hydrogène, un groupe hydroxy, amino, un reste alkyle en C₁ à C₃, alkoxy en C₁ à C₃, benzyloxy, alkylamino en C₁ à C₃, (alkyle en C₁ à C₃)carbonylamino, phénylcarbonylamino ou benzylcarbonylamino,
R⁴ représente l'hydrogène ou un reste alkyle en C₁ à C₃,
R⁵ représente un halogène, un reste trifluorométhyle, trifluorométhoxy, un groupe nitro, amino, un reste alkylamino en C₁ à C₆, un groupe hydroxy, un reste alkyle en C₁ à C₆, alkoxy en C₁ à C₆, un groupe carboxyle, un reste (alkoxy en C₁ à C₆)carbonyle, benzyloxycarbonyle, aminocarbonyle, (alkyle en C₁ à C₆)aminocarbonyle, aryle en C₆ à C₁₄ ou un reste hétéroaryle pentagonal à décagonal,
ou bien
deux substituants R⁵ forment, conjointement avec les atomes de carbone auxquels ils sont liés, un reste cycloalkyle trigonal à octogonal ou un reste hétérocyclyle tétragonal à décagonal, ce reste cycloalkyle ou hétérocyclyle pouvant être substitué avec 0, 1 ou 2 substituants R⁵⁻¹, les substituants R⁵⁻¹ étant choisis indépendamment l'un de l'autre dans le groupe comprenant les substituants halogéno, nitro, amino, trifluorométhyle, hydroxy et alkoxy en C₁ à C₆,
R⁶ est un reste alkyle en C₁ à C₆, cycloalkyle en C₃ à C₈ ou cycloalcényle en C₃ à C₈,
R⁶ pouvant être substitué avec 0, 1, 2 ou 3 substituants R⁶⁻¹, les substituants R⁶⁻¹ étant choisis indépendamment les uns des autres dans le groupe comprenant les substituants halogéno, nitro, amino, trifluorométhyle, hydroxy, alkyle en C₁ à C₆ et alkoxy en C₁ à C₆,
n représente le nombre 0, 1, 2 ou 3,
les restes R⁵ pouvant être identiques ou différents lorsque n a la valeur 2 ou 3,
m représente le nombre 0, 1, 2 ou 3,
A est un reste aryle en C₆ à C₁₄ ou un reste hétéroaryle pentagonal à décagonal,
A pouvant être substitué avec 0, 1, 2 ou 3 substituants R^{A}, les substituants R^{A} étant choisis indépendamment les uns des autres dans le groupe comprenant les substituants halogéno, alkyle en C₁ à C₆, nitro, amino, cyano, trifluorométhyle, aryle en C₆ à C₁₄, hétéroaryle pentagonal à décagonal, hydroxy, alkoxy en C₁ à C₆, alkylamino en C₁ à C₆, carboxyle, (alkoxy en C₁ à C₆) carbonyle, aminocarbonyle, (alkyle en C₁ à C₆)carbonylamino ou (alkyle en C₁ à C₆) - aminocarbonyle,
ou bien
deux substituants R^{A} forment, conjointement avec les atomes de carbone auxquels ils sont liés, un reste cycloalkyle trigonal à octogonal ou hétérocyclyle tétragonal à décagonal, ce reste cycloalkyle ou hétérocyclyle pouvant être substitué avec 0, 1 ou 2 substituants R^{A-1}, les substituants R^{A-1} étant choisis indépendamment l'un de l'autre dans le groupe comprenant les substituants halogéno, nitro, amino, trifluorométhyle, hydroxy et alkoxy en C₁ à C₆,
B est un reste aryle en C₆ à C₁₄ ou hétéroaryle pentagonal à décagonal,
ainsi que leurs sels, leurs produits de solvatation et leurs hydrates pharmaceutiquement acceptables.

2. Composé suivant la revendication 1, **caractérisé en ce que**
R¹ représente l'hydrogène ou un groupe méthyle,
R^{1'} représente l'hydrogène, un groupe méthyle ou le fluor,
R² représente l'hydrogène,
R³ représente l'hydrogène, un groupe amino, un reste alkyle en C₁ à C₃, alkoxy en C₁ à C₃, benzyloxy, alkylamino en C₁ à C₃, (alkyle en C₁ à C₃)carbonylamino, phénylcarbonylamino ou benzylcarbonylamino,
R⁴ est un reste méthyle,
R⁵ représente le fluor, le chlore, un reste trifluorométhyle, trifluorométhoxy, un groupe nitro, amino, un reste alkylamino en C₁ à C₆, un groupe hydroxy, un reste alkyle en C₁ à C₆, alkoxy en C₁ à C₆, (alkoxy en C₁ à C₆)carbonyle, aminocarbonyle, phényle ou hétéroaryle pentagonal ou hexagonal,
ou bien
deux substituants R⁵ forment, conjointement avec les atomes de carbone auxquels ils sont liés, un reste cycloalkyle pentagonal ou hexagonal ou un reste hétérocyclyle pentagonal
ou hexagonal,
R⁶ est un reste alkyle en C₂ à C₇, cycloalkyle en C₃ à C₇
ou cycloalcényle en C₅ à C₇,
R⁶ pouvant être substitué avec 0, 1 ou 2 substituants R⁶⁻¹, les substituants R⁶⁻¹ étant choisis dans le groupe comprenant les substituants halogéno, trifluorométhyle, alkyle en C₁ à C₆ et méthoxy,
n représente un nombre 0, 1, ou 2,
les restes R⁵ pouvant être identiques ou différents lorsque n est égal à 2,
m représente le nombre 1 ou 2,
A est un reste phényle, naphtyle ou un reste hétéroaryle pentagonal, hexagonal ou décagonal,
A pouvant être substitué avec 0, 1 ou 2 substituants R^{A}, les substituants R^{A} étant choisis indépendamment les uns des autres dans le groupe comprenant les substituants halogéno, alkyle en C₁ à C₆, amino, cyano, trifluorométhyle, aryle en C₆ à C₁₄, hétéroaryle pentagonal à décagonal, hydroxy, alkoxy en C₁ à C₆, alkylamino en C₁ à C₆, (alkoxy en C₁ à C₆)carbonyle et aminocarbonyle,
ou bien
deux substituants R^{A} forment, conjointement avec les atomes de carbone auxquels ils sont liés, un reste cycloalkyle pentagonal ou hexagonal ou un reste hétérocyclyle pentagonal
ou hexagonal, ce reste cycloalkyle ou hétérocyclyle pouvant être substitué avec 0 ou 1 substituant R^{A-1}, les substituants R^{A-1} étant choisis indépendamment l'un de l'autre dans le groupe comprenant les substituants halogéno, nitro, amino, trifluorométhyle, hydroxy et alkoxy en C₁ à C₆,
B est un reste phényle, naphtyle ou un reste hétéroaryle pentagonal, hexagonal, nonagonal ou décagonal,

3. Composé suivant l'une des revendications 1 ou 2, **caractérisé en ce qu'**il répond à la formule (Ia), 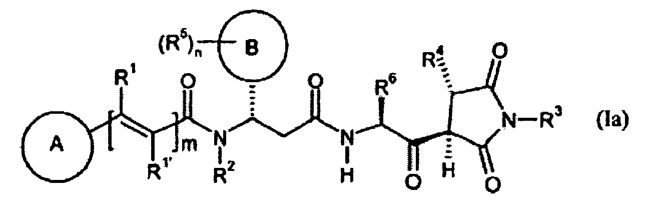 dans laquelle
R¹ représente l'hydrogène,
R^{1'} représente l'hydrogène, un reste méthyle ou le fluor,
R² représente l'hydrogène,
R³ représente l'hydrogène, un groupe amino, un reste méthyle, méthoxy, éthoxy, méthylamino ou diméthylamino,
R⁴ est un reste méthyle,
R⁵ représente le fluor, le chlore, un reste trifluorométhyle, alkoxy en C₁ à C₆, méthoxycarbonyle, alkyle en C₁ à C₄, phényle ou pyridyle,
ou bien
deux substituants R⁵ forment, conjointement avec les atomes de carbone auxquels ils sont liés, un reste hétérocyclyle pentagonal ou hexagonal,
R⁶ est un reste alkyle en C₃ à C₆, cycloalkyle en C₄ à C₆ ou cycloalcényle en C₅ ou C₆,
n représente le nombre 0, 1, ou 2,
les restes R⁵ pouvant être identiques ou différents lorsque n est égal à 2,
m représente le nombre 1,
A est un reste phényle, pyridyle, imidazolyle, thiényle, furannyle, oxadiazolyle, pyrazolyle, pyrazinyle, pyridazinyle, pyrimidinyle, quinolinyle ou isoquinolinyle,
A pouvant être substitué avec 0, 1 ou 2 substituants R^{A}, les substituants R^{A} étant choisis indépendamment les uns des autres dans le groupe comprenant les substituants halogéno, alkyle en C₁ à C₆, cyano, trifluorométhyle, phényle et alkoxy en C₁ à C₆,
ou bien
deux substituants R^{A} forment, conjointement avec les atomes de carbone auxquels ils sont liés, un reste hétérocyclyle pentagonal ou hexagonal,
B est un reste phényle, naphtyle, pyridyle, thiényle, furannyle, quinolinyle ou isoquinolinyle,

4. Composé suivant la revendication 3, **caractérisé en ce que**
R¹ représente l'hydrogène,
R^{1'} représente l'hydrogène,
R² représente l'hydrogène,
R³ représente l'hydrogène, un groupe amino, un reste méthylamino ou diméthylamino,
R⁴ est un reste méthyle,
R⁵ représente le fluor, le chlore, un reste trifluorométhyle, un groupe méthoxy, un reste alkyle en C₁ à C₄, phényle ou pyridyle,
ou bien
deux substituants R⁵ forment, conjointement avec le noyau phényle auquel ils sont liés, un groupement 1,3-benzodioxole
ou un groupement 1,4-benzodioxanne,
R⁶ est un reste isopropyle, tertiobutyle, isobutyle, isopentyle, cyclobutyle ou cyclopentyle,
n représente un nombre 0, 1, ou 2,
les restes R⁵ pouvant être identiques ou différents lorsque n est égal à 2,
m représente le nombre 1,
A est un reste phényle, pyridyle, thiényle, quinolinyle ou isoquinolinyle,
A pouvant être substitué avec 0, 1 ou 2 substituants R^{A}, les substituants R^{A} étant choisis indépendamment l'un de l'autre dans le groupe comprenant les substituants fluoro, chloro, alkyle en C₁ à C₃, cyano, trifluorométhyle, phényle et alkoxy en C₁ à C₃,
ou bien
deux substituants R^{A} forment, conjointement avec le noyau phényle auquel ils sont liés, un groupement 1,3-benzodioxole ou 1,4-benzodioxanne,
B est un reste phényle, naphtyle, thiényle, quinolinyle ou isoquinolinyle,

5. Composé suivant l'une des revendications 1 ou 2, **caractérisé en ce que** R¹ représente l'hydrogène.

6. Composé suivant l'une des revendications 1, 2, 3 ou 5, **caractérisé en ce que** R^{1'} représente l'hydrogène.

7. Composé suivant l'une des revendications 1, 5 ou 6, **caractérisé en ce que** R² représente l'hydrogène.

8. Composé suivant l'une des revendications 1, 5, 6 ou 7, **caractérisé en ce que** R⁴ représente un groupe méthyle.

9. Composé suivant l'une des revendications 1, 2, 5, 6 ou 7, **caractérisé en ce que** m représente le nombre 1.

10. Composé suivant l'une des revendications précédentes, **caractérisé en ce que** R³ représente l'hydrogène ou un groupe amino.

11. Composé suivant l'une des revendications précédentes, **caractérisé en ce que** n représente le nombre 0.

12. Composé suivant l'une des revendications précédentes, **caractérisé en ce que** n représente le nombre 1, B est un reste phényle et R⁵ représente le fluor, le chlore, un reste trifluorométhyle, alkoxy en C₁ à C₆, alkyle en C₁ à C₄, phényle ou pyridyle, R⁵ étant en position méta ou para par rapport au site de liaison du noyau phényle.

13. Composé suivant l'une des revendications précédentes, **caractérisé en ce que** R⁶ est un reste isopropyle, tertiobutyle, isobutyle, isopentyle ou cyclopentyle.

14. Composé suivant l'une des revendications précédentes, **caractérisé en ce que** A est un reste phényle ou pyridyle, A pouvant être substitué avec 0, 1 ou 2 substituants R^{A}, les substituants R^{A} étant choisis indépendamment l'un de l'autre dans le groupe comprenant les substituants fluoro, chloro, cyano, trifluorométhyle, phényle et méthoxy.

15. Procédé de production de composés suivant la revendication 1, **caractérisé en ce que**
selon le procédé [A]
un composé de formule 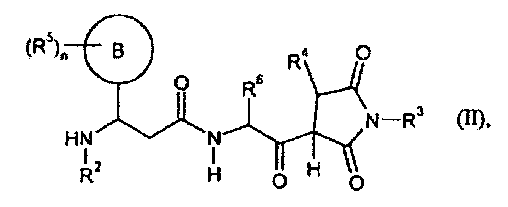 dans laquelle
R² à R⁶, B et n ont la définition indiquée dans la revendication 1,
est amené à réagir avec un composé de formule 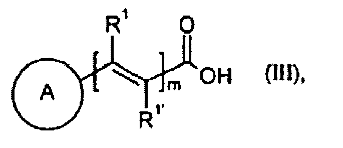 dans laquelle R¹ et R^{1'}, A et m ont la définition indiquée dans la revendication 1,
ces composés pouvant éventuellement exister sous la forme activée,
ou bien
selon le procédé [B]
un composé de formule 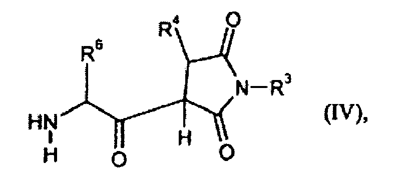 dans laquelle
R³, R⁴ et R⁶ ont la définition indiquée dans la revendication 1,
est amené à réagir avec un composé de formule 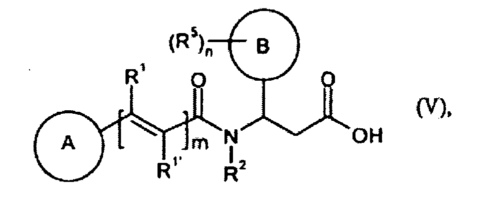 R¹, R^{1'}, R², R⁵, A, B, m et n ayant la définition indiquée dans la revendication 1,
ces composés pouvant éventuellement exister sous la forme activée.

16. Composé suivant l'une des revendications 1 à 14, destiné au traitement et/ou à la prophylaxie de maladies.

17. Médicament, contenant au moins un composé suivant l'une des revendications 1 à 14 en combinaison avec au moins un support pharmaceutiquement compatible et sans danger ou d'autres excipients.

18. Utilisation d'un composé suivant l'une des revendications 1 à 14 pour la préparation d'un médicament destiné au traitement et/ou à la prophylaxie d'infections bactériennes.

19. Médicament suivant la revendication 17, destiné au traitement et/ou à la prophylaxie d'infections bactériennes.
